# EUROPEAN PATENT SPECIFICATION

(11) **EP 2 145 877 B1**
(45) Date of publication and mention of the grant of the patent: **03.10.2012**
(21) Application number: 09173807.0
(22) Date of filing: 08.10.2007
(51) Int. Cl.: C07D 213/61, C07D 471/04, A61K 31/437

(54) **Aurora Kinase inhibitors**
Aurora Kinase-Inhibitoren
Inhibiteurs de kinases Aurora

(30) Priority: 09.10.2006 US 539857; 18.04.2007 US 912629 P; 10.10.2006 WO PCT/US2006/039667
(43) Date of publication of application: 20.01.2010
(62) Divisional of application: 07868390.1
(73) Proprietor: Takeda Pharmaceutical Company Limited, Osaka-shi, Osaka 541-0045 (JP)
(72) Inventor: Brown, Jason, W., San Diego, CA 92107 (US); Dong, Qing, San Diego, CA 92130 (US); Paraselli, Bheema, R., San Diego, CA 92130 (US); Stafford, Jeffrey, Alan, San Diego, CA 92130 (US); Wallace, Michael, B., San Diego, CA 92117 (US); Wijesekera, Hasanthi, QLD, Queensland 4078 (AU)
(74) Representative: Duncan, Garreth Andrew

(56) References cited:
- WO-A-2005/095400
- WO-A-2008/016184
- WO-A1-2007/044779

## Description

### FIELD OF THE INVENTION

The present invention relates to compounds that may be used to inhibit kinases as well as compositions of matter, kits and articles of manufacture comprising these compounds. The present invention also relates to compounds according to the present invention for use as medicaments. Methods of making the compounds of the present invention, as well as intermediates useful in such methods are described herein. In particular, the present invention relates to Aurora kinase inhibitors; compositions of matter, kits and articles of manufacture comprising these compounds; methods of making Aurora kinase inhibitors are described herein.

### BACKGROUND OF THE INVENTION

The present invention relates to inhibitors of enzymes that catalyze phosphoryl transfer and/or that bind ATP/GTP nucleotides, compositions comprising the inhibitors, kits and articles of manufacture comprising the inhibitors and compositions, and the inhibitors and inhibitor compositions for use as medicaments. The inhibitors and compositions comprising them are useful for treating or modulating disease in which phosphoryl transferases, including kinases, may be involved, symptoms of such disease, or the effect of other physiological events mediated by phosphoryl transferases, including kinases. Methods of making the inhibitor compounds are described herein.

Phosphoryl transferases are a large family of enzymes that transfer phosphorus-containing groups from one substrate to another. By the conventions set forth by the Nomenclature Committee of the International Union of Biochemistry and Molecular Biology (IUBMB) enzymes of this type have Enzyme Commission (EC) numbers starting with 2,7.-,- (See, Bairoch A., The ENZYME database in Nucleic Acids Res. 28:204-305 (2000)). Kinases are a class of enzymes that function in the catalysis of phosphoryl transfer, The protein kinases constitute the largest subfamily of structurally related phosphoryl transferases and are responsible for the control of a wide variety of signal transduction processes within the cell. (See, Hardie, G. and Hanks, S. (1995) The Protein Kinase Facts Book, I and II, Academic Press, San Diego, CA). Protein kinases are thought to have evolved from a common ancestral gene due to the conservation of their structure and catalytic function. Almost all kinases contain a similar 250-300 amino acid catalytic domain. The protein kinases may be categorized into families by the substrates they phosphorylate (e.g., protein-tyrosine, protein-serine/threonine, histidine, etc.). Protein kinase sequence motifs have been identified that generally correspond to each of these kinase families (See, for example, Hanks, S.K.; Hunter, T., FASEB J. 9:576-596 (1995); Kinghton et al., Science, 253:407-414 (1991); Hiles et al., Cell 70:419-429 (1992); Kunz et al., Cell, 73:585-596 (1993); Garcia-Bustos et al., EMBO J., 13:2352-2361 (1994)). Lipid kinases (e.g. PI3K) constitute a separate group of kinases with structural similarity to protein kinases.

Protein and lipid kinases regulate many different cell processes including, but not limited to, proliferation, growth, differentiation, metabolism, cell cycle events, apoptosis, motility, transcription, translation and other signaling processes, by adding phosphate groups to targets such as proteins or lipids. Phosphorylation events catalyzed by kinases act as molecular on/off switches that can modulate or regulate the biological function of the target protein. Phosphorylation of target proteins occurs in response to a variety of extracellular signals (hormones, neurotransmitters, growth and differentiation factors, etc.), cell cycle events, environmental or nutritional stresses, etc. Protein and lipid kinases can function in signaling pathways to activate or inactivate, or modulate the activity of (either directly or indirectly) the targets. These targets may include, for example, metabolic enzymes, regulatory proteins, receptors, cytoskeletal proteins, ion channels or pumps, or transcription factors. Uncontrolled signaling due to defective control of protein phosphorylation has been implicated in a number of diseases and disease conditions, including, for example, inflammation, cancer, allergy/asthma, diseases and conditions of the immune system, disease and conditions of the central nervous system (CNS), cardiovascular disease, dermatology, and angiogenesis.

Initial interest in protein kinases as pharmacological targets was stimulated by the findings that many viral oncogenes encode structurally modified cellular protein kinases with constitutive enzyme activity. These findings pointed to the potential involvement of oncogene related protein kinases in human proliferatives disorders. Subsequently, deregulated protein kinase activity, resulting from a variety of more subtle mechanisms, has been implicated in the pathophysiology of a number of important human disorders including, for example, cancer, CNS conditions, and immunologically related diseases. The development of selective protein kinase inhibitors that can block the disease pathologies and/or symptoms resulting from aberrant protein kinase activity has therefore generated much interest.

Cancer results from the deregulation of the normal processes that control cell division, differentiation and apoptotic cell death. Protein kinases play a critical role in this regulatory process. A partial non-limiting list of such kinases includes ab1, Aurora-A, Aurora-B, Aurora-C, ATK, bcr-abl, Blk, Brk, Btk, c-Kit, c-Met, c-Src, CDK1, CDK2, CDK4, CDK6, cRafl, CSF1R, CSK, EGFR, ErbB2, ErbB3, ErbB4, ERK, Fak, fes, FGFR1, FGFR2, FGFR3, FGFR4, FGFR5, Fgr, FLK-4, Flt-1, Fps, Frk, Fyn, Hck, IGF-1R, INS-R, Jak, KDR, Lck, Lyn, MEK, p38, PDGFR, PIK, PKC, PYK2, Ros, Tie1, Tie2, Trk, Yes and Zap70. In mammalian biology, such protein kinases comprise mitogen activated protein kinase (MAPK) signaling pathways. MAPK signaling pathways are inappropriately activated by a variety of common disease-associated mechanisms such as mutation of ras genes and deregulation of growth factor receptors (Magnuson et al., Seminars in Cancer Biology 5:247-252 (1994)). Therefore the inhibition of protein kinases is an object of the present invention.

Aurora kinases (Aurora-A, Aurora-B, Aurora-C) are serine/threonine protein kinases that have been implicated in human cancer, such as colon, breast and other solid tumors. Aurora-A (also sometimes referred to as AIK) is believed to be involved in protein phosphorylation events that regulate the cell cycle. Specifically, Aurora-A may play a role in controlling the accurate segregation of chromosomes during mitosis. Misregulation of the cell cycle can lead to cellular proliferation and other abnormalities. In human colon cancer tissue, Aurora-A, Aurora-B and Aurora-C have been found to be overexpressed (See, Bischoff et al., EMBO J., 17:3052-3065 (1998); Schumacher et al., J. Cell Biol. 143:1635-1646 (1998); Kimura et al., J. Biol. Chem., 272:13766-13771 (1997)).

There is a continued need to find new therapeutic agents to treat human diseases. The protein kinases, specifically but not limited to Aurora-A, Aurora-B and Aurora-C are especially attractive targets for the discovery of new therapeutics due to their important role in cancer, diabetes, Alzheimer's disease and other diseases.

### SUMMARY OF THE INVENTION

The present invention relates to compounds as specified herein that have activity for inhibiting kinases. The present invention also provides compositions, articles of manufacture and kits comprising these compounds.

In one embodiment, a pharmaceutical composition is provided that comprises a kinase inhibitor according to the present invention as an active ingredient. Pharmaceutical compositions according to the invention may optionally comprise 0.001%-100% of one or more kinase inhibitors of this invention. These pharmaceutical compositions may be administered or coadministered by a wide variety of routes, including for example, orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, or intrathecally. The compositions may also be administered or coadministered in slow release dosage forms.

The invention is also directed to kits and other articles of manufacture for treating disease states associated with kinases.

In one embodiment, a kit is provided that comprises a composition comprising at least one kinase inhibitor of the present invention in combination with instructions. The instructions may indicate the disease state for which the composition is to be administered, storage information, dosing information and/or instructions regarding how to administer the composition. The kit may also comprise packaging materials. The packaging material may comprise a container for housing the composition. The kit may also optionally comprise additional components, such as syringes for administration of the composition. The kit may comprise the composition in single or multiple dose forms.

In another embodiment, an article of manufacture is provided that comprises a composition comprising at least one kinase inhibitor of the present invention in combination with packaging materials. The packaging material may comprise a container for housing the composition. The container may optionally comprise a label indicating the disease state for which the composition is to be administered, storage information, dosing information and/or instructions regarding how to administer the composition. The kit may also optionally comprise additional components, such as syringes for administration of the composition. The kit may comprise the composition in single or multiple dose forms.

Methods for preparing compounds, compositions and kits according to the present invention are described herein. For example, several synthetic schemes are described herein for synthesizing compounds according to the present invention.

Also provided are compounds, compositions, kits and articles of manufacture according to the present invention for use as medicaments.

The compounds, compositions, kits and articles of manufacture are used to inhibit kinases. In particular, the compounds, compositions, kits and articles of manufacture are used to inhibit an Aurora kinase.

The compounds, compositions, kits and articles of manufacture are used to treat a disease state for which kinases possess activity that contributes to the pathology and/or symptomology of the disease state.

In another embodiment, there is provided a compound according to the present invention for use as a medicament.

In another embodiment, there is provided a compound according to the present invention for use in inhibiting cell proliferation.

It is noted in regard to all of the above embodiments that the present invention is intended to encompass all pharmaceutically acceptable ionized forms (e.g., salts) and solvates (e.g., hydrates) of the compounds, regardless of whether such ionized forms and solvates are specified since it is well know in the art to administer pharmaceutical agents in an ionized or solvated form. It is also noted that unless a particular stereochemistry is specified, recitation of a compound is intended to encompass all possible stereoisomers (e.g., enantiomers or diastereomers depending on the number of chiral centers), independent of whether the compound is present as an individual isomer or a mixture of isomers. Further, unless otherwise specified, recitation of a compound is intended to encompass all possible resonance forms and tautomers. With regard to the claims, the language "compound having the formula" is intended to encompass the compound and all pharmaceutically acceptable ionized forms and solvates, all possible stereoisomers, and all possible resonance forms and tautomers unless otherwise specifically specified in the particular claim.

### BRIEF DESCRIPTION OF THE FIGURES

Figure 1 is a characteristic XRPD spectrum of an amorphous form of Compound 88.

### DEFINITIONS

Unless otherwise stated, the following terms used in the specification and claims shall have the following meanings for the purposes of this Application.

"IC₅₀" means the molar concentration of an inhibitor that produces 50% inhibition of the target enzyme.

"Isomers" mean compounds having identical molecular formulae but differing in the nature or sequence of bonding of their atoms or in the arrangement of their atoms in space. Isomers that differ in the arrangement of their atoms in space are termed "stereoisomers" Stereoisomers that are not mirror images of one another are termed "diastereomers" and stereoisomers that are nonsuperimposable mirror images are termed "enantiomers" or sometimes "optical isomers." A carbon atom bonded to four nonidentical substituents is termed a "chiral center." A compound with one chiral center has two enantiomeric forms of opposite chirality. A mixture of the two enantiomeric forms is termed a "racemic mixture." A compound that has more than one chiral center has 2^{*n*-1} enantiomeric pairs, where n is the number of chiral centers. Compounds with more than one chiral center may exist as ether an individual diastereomer or as a mixture of diastereomers, termed a "diastereomeric mixture." When one chiral center is present a stereoisomer may be characterized by the absolute configuration of that chiral center. Absolute configuration refers to the arrangement in space of the substituents attached to the chiral center. Enantiomers are characterized by the absolute configuration of their chiral centers and described by the *R*- and *S*-sequencing rules of Cahn, Ingold and Prelog. Conventions for stereochemical nomenclature, methods for the determination of stereochemistry and the separation of Stereoisomers are well known in the art (*e.g*., see "Advanced Organic Chemistry", 4th edition, March, Jerry, John Wiley & Sons, New York, 1992).

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts of compounds of the present invention which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, *o*-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, 2-naphthalenesulfonic acid, *p*-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like.

Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, N-methylglucamine and the like.

"Subject" includes humans, non-human mammals (*e.g*., dogs, cats, rabbits, cattle, horses, sheep, goats, swine, deer, and the like) and non-mammals (*e.g*., birds, and the like).

"Therapeutically effective amount" means that amount which, when administered to an animal for treating a disease, is sufficient to effect such treatment for the disease.

"Treatment" or "treating" means any administration of a compound of the present invention and includes:
(1) preventing the disease from occurring in an animal which may be predisposed to the disease but does not yet experience or display the pathology or symptomatology of the disease,
(2) inhibiting the disease in an animal that is experiencing or displaying the pathology or symptomatology of the diseased (*i.e*., arresting further development of the pathology and/or symptomatology), or
(3) ameliorating the disease in an animal that is experiencing or displaying the pathology or symptomatology of the diseased (*i.e*., reversing the pathology and/or symptomatology).

"Crystalline", as the term is used herein, refers to a material that contains a specific compound, which may be hydrated and/or solvated, and has sufficient crystalline content to exhibit a discernable diffraction pattern by XRPD or other diffraction techniques. Often, a crystalline material that is obtained by direct crystallization of a compound dissolved in a solution or interconversion of crystals obtained under different crystallization conditions, will have crystals that contain the solvent used in the crystallization, termed a crystalline solvate. Also, the specific solvent system and physical embodiment in which the crystallization is performed, collectively termed crystallization conditions, may result in the crystalline material having physical and chemical properties that are unique to the crystallization conditions, generally due to the orientation of the chemical moieties of the compound with respect to each other within the crystal and/or the predominance of a specific polymorphic form of the compound in the crystalline material.

Depending upon the polymorphic form(s) of the compound that are present in a composition, various amounts of the compound in an amorphous solid state may also be present, either as a side product of the initial crystallization, and/or a product of degradation of the crystals comprising the crystalline material. Thus, crystalline, as the term is used herein, contemplates that the composition may include amorphous content; the presence of the crystalline material among the amorphous material being detectably among other methods by the composition having a discernable diffraction pattern.

The amorphous content of a crystalline material may be increased by grinding or pulverizing the material, which is evidenced by broadening of diffraction and other spectral lines relative to the crystalline material prior to grinding. Sufficient grinding and/or pulverizing may broaden the lines relative to the crystalline material prior to grinding to the extent that the XRPD or other crystal specific spectrum may become undiscernable, making the material substantially amorphous or quasi-amorphous.

Continued grinding would be expected to increase the amorphous content and further broaden the XRPD pattern with the limit of the XRPD pattern being so broadened that it can no longer be discerned above noise. When the XRPD pattern is broadened to the limit of being indiscernible, the material may be considered to no longer be a crystalline material, and instead be wholly amorphous. For material having increased amorphous content and wholly amorphous material, no peaks should be observed that would indicate grinding produces another form.

"Amorphous", as the term is used herein, refers to a composition comprising a compound that contains too little crystalline content of the compound to yield a discernable pattern by XRPD or other diffraction techniques. Glassy materials are a type of amorphous material. Amorphous materials do not have a true crystal lattice, and are consequently glassy rather than true solids, technically resembling very viscous non-crystalline liquids. Rather than being true solids, glasses may better be described as quasi-solid amorphous material. Thus, an amorphous material refers to a quasi-solid, glassy material.

"Broad" or "broadened", as the term is used herein to describe spectral lines, including XRPD, NMR and IR spectroscopy, and Raman spectroscopy lines, is a relative term that relates to the line width of a baseline spectrum. The baseline spectrum is often that of an unmanipulated crystalline form of a specific compound as obtained directly from a given set of physical and chemical conditions, including solvent composition and properties such as temperature and pressure. For example, broadened can be used to describe the spectral lines of a XRPD spectrum of ground or pulverized material comprising a crystalline compound relative to the material prior to grinding. In materials where the constituent molecules, ions or atoms, as solvated or hydrated, are not tumbling rapidly, line broadening is indicative of increased randomness in the orientation of the chemical moieties of the compound, thus indicative of an increased amorphous content. When comparisons are made between crystalline materials obtained via different crystallization conditions, broader spectral lines indicate that the material producing the relatively broader spectral lines has a higher level of amorphous material.

"About" as the term is used herein, refers to an estimate that the actual value falls within ±5% of the value cited.

### KINASE INHIBITORS

In one embodiment, kinase inhibitors of the present invention are of the formula: 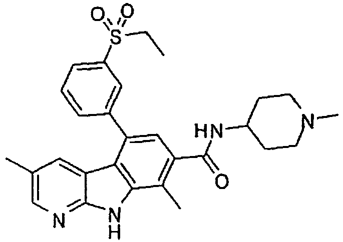 or a polymorph, solvate, ester, tautomer, enantiomer or pharmaceutically acceptable salt thereof.

In a further embodiment, kinase inhibitors of the present invention are of the formula: 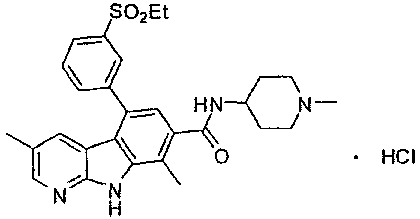 or a polymorph, solvate, ester, tautomer, enantiomer or pharmaceutically acceptable salt thereof, wherein at least a portion of the compound is present as Amorphous Form, characterized by physical properties which comprise one or more of the following:
(a) may be formed by lyophilizing a solution of Compound 88 in ACN and water;
(b) has an XRPD spectrum characterized by a diffuse halo with no discernable peaks; and/or
(c) shows 7.6 wt% Cl⁻ present using ion chromatography.
In one embodiment, the compounds of the present invention are selected from the group consisting of:
N-(2-(dimethylamino)ethyl)-5-(3-(ethylsulfonyl)phenyl)-3,8-dimethyl-9H-pyrido[2,3-b]indole-7-carboxamide;
N-(2-(methylamino)ethyl)-5-(3-(ethylsulfonyl)phenyl)-3,8-dimethyl-9H-pyrido[2,3-b]indole-7-carboxamide;
N-(2-(methoxy)ethyl)-5-(3-(ethylsulfonyl)phenyl)-3,8-dimethyl-9H-pyrido[2,3-b]indole-7-carboxamide;
N-(2-(dimethylamino)ethyl)-N-methyl-5-(3-(ethylsulfonyl)phenyl)-3,8-dimethyl-9H-pyrido[2,3-b]indole-7-carboxamide;
N,N-dimethyl-5-(3-(ethylsulfonyl)phenyl)-3,8-dimethyl-9H-pyrido[2,3-b]indole-7-methylcarboxamide;
5-(3-(ethylsulfonyl)phenyl)-3,8-dimethyl-9H-pyrido[2,3-b]indole-7-yl)(4-methylpiperazin-1-yl)methanone;
5-(3-(ethylsulfonyl)phenyl)-3,8-dimethyl-N-(2-piperazin-1-yl)ethyl)-9H-pyrido[2,3-b]indole-7-carboxamide;
5-(3-(ethylsulfonyl)phenyl)-3,8-dimethyl-N-(3-(4-methylpiperazin-1-yl)propyl)-9H-pyrido[2,3-b]indole-7-carboxamide;
5-(3-(ethylsulfonyl)phenyl)-3,8-dimethyl-9H-pyrido[2,3-b]indole-7-yl)(morpholino)methanone;
azetidin-1-yl(5-(3-(ethylsulfonyl)phenyl)-3,8-dimethyl-9H-pyrido[2,3-b]indol-7-yl)methanone;
(5-(3-(ethylsulfonyl)phenyl)-3,8-dimethyl-9H-pyrido[2,3-b]indol-7-yl)(thaiazolidin-3-yl)methanone;
(R)-5-(3-(ethylsulfonyl)phenyl)-N-(2-hydroxypropyl)-3,8-dimethyl- 9H-pyrido[2,3-b]indole-7-carboxamide;
(S)-5-(3-(ethylsulfonyl)phenyl)-N-(2-hydroxypropyl)-3,8-dimethyl- 9H-pyrido[2,3-b]indole-7-carboxamide;
5-(3-(ethylsulfonyl)phenyl)-N-(2-hydroxyethyl)-3,8-dimethyl- 9H-pyrido[2,3-b]indole-7-carboxamide;
N-(2,3-dihydroxypropyl)-5-(3-(ethylsulfonyl)phenyl)-3,8-dimethyl- 9H-pyrido[2,3-b]indole-7-carboxamide;
5-(3-(ethylsulfonyl)phenyl)-N-(2-hydroxy-2methylpropyl)-3,8-dimethyl- 9H-pyrido[2,3-b]indole-7-carboxamide;
5-(3-(ethylsulfonyl)phenyl)-N-(1-isopropylpiperidin-4-yl)-3,8-dimethyl- 9H-pyrido[2,3-b]indole-7-carboxamide;
N-(1-ethylpiperidin-4-yl)-5-(3-(ethylsulfonyl)phenyl)-3,8-dimethyl- 9H-pyrido[2,3-b]indole-7-carboxamide;
5-(3-(ethylsulfonyl)phenyl)-3,8-dimethyl- N-thiazol-2-yl)-9H-pyrido[2,3-b]indole-7-carboxamide;
5-(3-(ethylsulfonyl)phenyl)-3,8-dimethyl- N-(2-(2,2,2-trifluoroethoxy)ethyl-9H-pyrido[2,3-b]indole-7-carboxamide;
5-(3-(ethylsulfonyl)phenyl)-3,8-dimethyl- N-(piperidin-3-yl)-9H-pyrido[2,3-b]indole-7-carboxamide;
5-(3-(ethylsulfonyl)phenyl)-3,8-dimethyl- N-(piperidin-4-yl)-9H-pyrid o[2,3-b]indole-7-carboxamide;
5-(3-(ethylsulfonyl)phenyl)-3,8-dimethyl- N-(piperidin-3-yl)-9H-pyrido[2,3-b]indole-7-carboxamide;
5-(3-(ethylsulfonyl)phenyl)-N-(2-(2-hydroxyethoxy)ethyl-3,8-dimethyl-9H-pyrido[2,3-b]indole-7-carboxamide;
5-(3-(cyclopropanecarboxamido)phenyl)-N-(2-(dimethylamino)ethyl)-3,8-dimethyl-9H-pyrido[2,3-b]indole-7-carboxamide;
5-(3-(ethylsulfonyl)phenyl)-3,8-dimethyl-N-(1-methylpiperidin-4-yl)-9H-pyrido[2,3-b]indole-7-carboxamide;
5-(3-(ethylsulfonyl)phenyl)-3,8-dimethyl-N-((1-methylpiperidin-4-yl)methyl)-9H-pyrido[2,3-b]indole-7-carboxaniide;
N-(3-(dimethylamino)propyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-(2-(pyrrolidin-1-yl)ethyl)-9H-pyrido[2,3-b]indole-7-carboxamide;
(S)-5-(3-(ethylsulfonyl)phenyl)-3,8-dimethyl-N-(1-methylpiperidin-3-yl)-9H-pyrido[2,3-b]indole-7-carboxamide;
(R)-5-(3-(ethylsulfonyl)phenyl)-3,8-dimethyl-N-(1-methylpiperidin-3-yl)-9H-pyrido[2,3-b]indole-7-carboxamide;
5-(3-(cyclopropanecarboxamido)phenyl)-3,8-dimethyl-N-(1-methyl-piperidin-4-yl)-9H-pyrido[2,3-b]indole-7-carboxamide;
5-(3-(cyclopropylcarbamoyl)phenyl)-N-(2-(dimethylamino)ethyl)-3,8-dimethyl-9H-pyrido[2,3-b]indole-7-carboxamide;
[5-(3-Ethanesulfonyl-phenyl)-3-methyl-9H-pyrido[2,3-b]indol-7-yl]-(4-methyl-piperazin-1-yl)-methanone;
5-(3-Ethanesulfonyl-phenyl)-3-methyl-9H-pyrido[2,3-b]indole-7-carboxylic acid (2-dimethylamino-ethyl)-amide;
5-(3-Ethanesulfonyl-phenyl)-3-methyl-9H-pyrido[2,3-b]indole-7-carboxylic acid (3-dimethylamino-propyl)-amide;
5-(benzylthio)-N-(2-(dimethylamino)ethyl)-3-methyl-9H-pyrido[2,3-b]indole-7-carboxamide;
5-(3-(N-ethylsulfamoyl)phenyl)-8-methoxy-3-methyl-N-(1-methylpiperidin-4-yl)-9H-pyrido[2,3-b]indole-7-carboxamide;
5-(3-(cyclopropylsulfonyl)phenyl)-3,8-dimethyl-N-(1-methylpiperidin-4-yl)-9H-pyrido[2,3-b]indole-7-carboxamide;
N-(2-(diethylamino)ethyl)-5-(3-(ethylsulfonyl)phenyl)-3,8-dimethyl-9H-pyrido[2,3-b]indole-7-carboxamide; and
5-(3-(ethylsulfonyl)phenyl)-3,8-dimethyl-N-(3-morpholinopropyl)-9H-pyrido[2,3-b]indole-7-carboxamide.

Particular examples of compounds according to the present invention also include, but are not limited to:
5-(3-(ethylsulfonyl)phenyl)-3,8-dimethyl-N-(1-methylpiperidin-4-yl)-9H-pyrido[2,3-b]indole-7-carboxamide HCl salt.

In addition, particular examples of compounds according to the present invention include, but are not limited to:
N-(2-(methylamino)ethyl)-5-(3-(ethylsulfonyl)phenyl)-3,8-dimethyl-9H-pyrido[2,3-b]indole-7-carboxamide;
N-(2-(methoxy)ethyl)-5-(3-(ethylsulfonyl)phenyl)-3,8-dimethyl-9H-pyrido[2,3-b)indole-7-carboxamide;
N-(2-(dimethylamino)ethyl)-N-methyl-5-(3-(ethylsulfonyl)phenyl)-3,8-dimethyl-9H-pyrido[2,3-b]indole-7-carboxamide;
N,N-dimethyl-5-(3-(ethylsulfonyl)phenyl)-3,8-dimethyl-9H-pyrido[2,3-b]indole-7-methylcarboxamide;
5-(3-(ethylsulfonyl)phenyl)-3,8-dimethyl-9H-pyrido[2,3-b]indole-7-yl)(4-methylpiperazin-1-yl)methanone;
5-(3-(ethylsulfonyl)phenyl)-3,8-dimethyl-N-(2-piperazin-1-yl)ethyl)-9H-pyrido[2,3-b]indole-7-carboxamide;
5-(3-(ethylsulfonyl)phenyl)-3,8-dimethyl-N-(3-(4-methylpiperazin-1-yl)propyl)-9H-pyrido[2,3-b]indole-7-carboxamide;
5-(3-(ethylsulfonyl)phenyl)-3,8-dimethyl-9H-pyrido[2,3-b]indole-7-yl)(morpholino)methanone;
azetidin-1-yl(5-(3-(ethylsulfonyl)phenyl)-3,8-dimethyl-9H-pyrido[2,3-b]indol-7-yl)methanone;
(5-(3-(ethylsulfonyl)phenyl)-3,8-dimethyl-9H-pyrido[2,3-b]indol-7-yl)(thaiazolidin-3-yl)methanone;
(R)-5-(3-(ethylsulfonyl)phenyl)-N-(2-hydroxypropyl)-3,8-dimethyl- 9H-pyrido[2,3-b]indole-7-carboxamide;
(S)-5-(3-(ethylsulfonyl)phenyl)-N-(2-hydroxypropyl)-3,8-dimethyl- 9H-pyrido[2,3-b]indole-7-carboxamide;
5-(3-(ethylsulfonyl)phenyl)-N-(2-hydroxyethyl)-3,8-dimethyl- 9H-pyrido[2,3-b]indole-7-carboxamide;
N-(2,3-dihydroxypropyl)-5-(3-(ethylsulfonyl)phenyl)-3,8-dimethyl- 9H-pyrido[2,3-b]indole-7-carboxamide;
5-(3-(ethylsulfonyl)phenyl)-N-(2-hydroxy-2methylpropyl)-3,8-dimethyl- 9H-pyrido[2,3-b]indole-7-carboxamide;
5-(3-(ethylsulfonyl)phenyl)-N-(1-isopropylpiperidin-4-yl)-3,8-dimethyl- 9H-pyrido[2,3-b]indole-7-carboxamide;
N-(1-ethylpiperidin-4-yl)-5-(3-(ethylsulfonyl)phenyl)-3,8-dimethyl- 9H-pyrido[2,3-b]indole-7-carboxamide;
5-(3-(ethylsulfonyl)phenyl)-3,8-dimethyl- N-thiazol-2-yl)-9H-pyrido[2,3-b]indole-7-carboxamide;
5-(3-(ethylsulfonyl)phenyl)-3,8-dimethyl- N-(2-(2,2,2-trifluoroethoxy)ethyl-9H-pyrido[2,3-b]indole-7-carboxamide;
5-(3-(ethylsulfonyl)phenyl)-3,8-dimethyl- N-(piperidin-3-yl)-9H-pyrido[2,3-b]indole-7-carboxamide;
5-(3-(ethylsulfonyl)phenyl)-3,8-dimethyl- N-(piperidin-4-yl)-9H-pyrid o[2,3-b]indole-7-carboxamide;
5-(3-(ethylsulfonyl)phenyl)-3,8-dimethyl- N-(piperidin-3-yl)-9H-pyrido[2,3-b]indole-7-carboxamide;
5-(3-(ethylsulfonyl)phenyl)-N-(2-(2-hydroxyethoxy)ethyl-3,8-dimethyl-9H-pyrido[2,3-b]indole-7-carboxamide;
5-(3-(cyclopropanecarboxamido)phenyl)-N-(2-(dimethylamino)ethyl)-3,8-dimethyl-9H-pyrido[2,3-b]indole-7-carboxamide;
N-(2-(dimethylamino)ethyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-((1-methylpiperidin-4-yl)methyl)-9H-pyrido[2,3-b]indole-7-carboxamide;
N-(3-(dimethylamino)propyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-(2-(pyrrolidin-1-yl)ethyl)-9H-pyrido[2,3-b]indole-7-carboxamide;
(S)-5-(3-(ethylsulfonyl)phenyl)-3,8-dimethyl-N-(1-methylpiperidin-3-yl)-9H-pyrido[2,3-b]indole-7-carboxamide;
(R)-5-(3-(ethylsulfonyl)phenyl)-3,8-dimethyl-N-(1-methylpiperidin-3-yl)-9H-pyrido[2,3-b]indole-7-carboxamide;
5-(3-(cyclopropanecarboxamido)phenyl)-3,8-dimethyl-N-(1-methyl-piperidin-4-yl)-9H-pyrido[2,3-b] indole-7-carboxamide;
5-(3-(cyclopropylcarbamoyl)phenyl)-N-(2-(dimethylamino)ethyl)-3,8-dimethyl-9H-pyrido[2,3-b]indole-7-carboxamide;
[5-(3-Ethanesulfonyl-phenyl)-3-methyl-9H-pyrido[2,3-b]indol-7-yl]-(4-methyl-piperazin-1-yl)-methanone;
5-(3-Ethanesulfonyl-phenyl)-3-methyl-9H-pyrido[2,3-b]indole-7-carboxylic acid (2-dimethylamino-ethyl)-amide;
5-(3-Ethanesulfonyl-phenyl)-3-methyl-9H-pyrido[2,3-b]indole-7-carboxylic acid (3-dimethylamino-propyl)-amide;
5-(benzylthio)-N-(2-(dimethylamino)ethyl)-3-methyl-9H-pyrido[2,3-b]indole-7-carboxamide;
5-(3-(N-ethylsulfamoyl)phenyl)-8-methoxy-3-methyl-N-(1-methylpiperidin-4-yl)-9H-pyrido[2,3-b]indole-7-carboxamide;
5-(3-(cyclopropylsulfonyl)phenyl)-3,8-dimethyl-N-(1-methylpiperidin-4-yl)-9H-pyrido[2,3-b]indole-7-carboxamide;
5-(3-(cyclopropylcarbamoyl)phenyl)-3,8-dimethyl-N-(1-methylpiperidin-4-yl)-9H-pyrido[2,3-b]indole-7-carboxamide;
N-(2-(diethylamino)ethyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-(3-morpholinopropyl)-9H-pyrido[2,3-b]indole-7-carboxamide;
5-(3-(ethylsulfonyl)phenyl)-8-methyl-N-(1-methylpiperidin-4-yl)-9H-pyrido[2,3-b]indole-7-carboxamide;
5-(3-(cyclopropylsulfonyl)phenyl)-8-methyl-N-(1-methylpiperidin-4-yl)-9H-pyrido[2,3-b]indole-7-carboxamide;
5-(3-(cyclopropylsulfonyl)phenyl)-3-fluoro-8-methyl-N-(1-methylpiperidin-4-yl)-9H-pyrido[2,3-b]indole-7-carboxamide;
3-chloro-5-(3-(cyclopropylsulfonyl)phenyl)-8-methyl-N-(1-methylpiperidin-4-yl)-9H-pyrido[2,3-b]indole-7-carboxamide;
5-(3-(ethylsulfonyl)phenyl)-N-(1-(2-hydroxyethyl)piperidin-4-yl)-3,8-dimethyl-9H-pyrido[2,3-b]indole-7-carbaxamide;
3-chloro-5-(3-(ethylsulfonyl)phenyl)-N-(1-(2-hydroxyethyl)piperidin-4-yl)-8-methyl-9H-pyrido[2,3-b]indole-7-carboxamide;
5-(3-(cyclopropylsulfonyl)phenyl)-N-(1-(2-hydroxyethyl)piperidin-4-yl)-3,8-dimethyl-9H-pyrido[2,3-b]indole-7-carboxamide;
3-chloro-5-(3-(cyclopropylsulfonyl)phenyl)-N-(1-(2-hydroxyethyl)piperidin-4-yl)-8-methyl-9H-pyrido[2,3-b]indole-7-carboxamide;
5-(3-(ethylsulfonyl)phenyl)-N-(2-((2-hydroxyethyl)(methyl)amino)ethyl)-3,8-dimethyl-9H-pyrido[2,3-b]indole-7-carboxamide;
3-chloro-5-(3-(ethylsulfonyl)phenyl)-N-(2-((2-hydroxyethyl)(methyl)amino)ethyl)-8-methyl-9H-pyrido[2,3-b]indole-7-carboxamide;
5-(3-(cyclopropylsulfonyl)phenyl)-N-(2-((2-hydroxyethyl)(methyl)amino)ethyl)-3,8-dimethyl-9H-pyrido[2,3-b]indole-7-carboxamide;
3-chloro-5-(3-(cyclopropylsulfonyl)phenyl)-N-(2-((2-hydroxyethyl)(methyl)amino)ethyl)-8-methyl-9H-pyrido[2,3-b]indole-7-carboxamide;
N-(3-(ethyl(2-hydroxyethyl)amino)propyl)-5-(3-(ethylsulfonyl)phenyl)-3,8-dimethyl-9H-pyrido[2,3-b]indole-7-carboxamide;
5-(3-(ethylsulfonyl)phenyl)-N-((1R,4R)-4-hydroxycyclohexyl)-3,8-dimethyl-9H-pyrido[2,3-b]indole-7-carboxamide;
5-(3-(ethylsulfonyl)phenyl)-N-((1S,4S)-4-hydroxycyclohexyl)-3,8-dimethyl-9H-pyrido[2,3-b]indole-7-carboxamide;
5-(3-(ethylsulfonyl)phenyl)-N-(4-(hydroxymethyl)cyclohexyl)-3,8-dimethyl-9H-pyrido[2,3-b]indole-7-carboxamide;
5-(3-(ethylsulfonyl)phenyl)-N-(4-(2-hydroxyethyl)cyclohexyl)-3,8-dimethyl-9H-pyrido[2,3-b]indole-7-carboxamide;
(5-(3-(ethylsulfonyl)phenyl)-3,8-dimethyl-9H-pyrido[2,3-b]indol-7-yl)(4-(hydroxymethyl)piperidin-1-yl)methanone;
(5-(3-(ethylsulfonyl)phenyl)-3,8-dimethyl-9H-pyrido[2,3-b]indol-7-yl)(4-(2-hydroxyethyl)piperidin-1-yl)methanone;
(5-(3-(ethylsulfonyl)phenyl)-3,8-dimethyl-9H-pyrido[2,3-b]indol-7-yl)(4-hydroxypiperidin-1-yl)methanone;
(5-(3-(ethylsulfonyl)phenyl)-3,8-dimethyl-9H-pyrido[2,3-b]indol-7-yl)(2-(hydroxymethyl)pyrrolidin-1-yl)methanone;
(5-(3-(ethylsulfonyl)phenyl)-3,8-dimethyl-9H-pyrido[2,3-b]indol-7-yl)(2-(hydroxymethyl)morpholino)methanone;
5-(3-(ethylsulfonyl)phenyl)-N-(3-hydroxypropyl)-3,8-dimethyl-9H-pyrido[2,3-b]indole-7-carboxamide;
(R)-5-(3-(ethylsulfonyl)phenyl)-N-(3-hydroxybutyl)-3,8-dimethyl-9H-pyrido[2,3-b]indole-7-carboxamide;
(S)-5-(3-(ethylsulfonyl)phenyl)-N-(3-hydroxybutyl)-3,8-dimethyl-9H-pyrido[2,3-b]indole-7-carboxamide;
5-(3-(ethylsulfonyl)phenyl)-N-(3-hydroxy-3-methylbutyl)-3,8-dimethyl-9H-pyrido[2,3-b]indole-7-carboxamide;
(S)-N-(3-(dimethylamino)-2-hydroxypropyl)-5-(3-(ethylsulfonyl)phenyl)-3,8-dimethyl-9H-pyrido[2,3-b]indole-7-carboxamide;
(S)-N-(2-(dimethylamino)-3-hydroxypropyl)-5-(3-(ethylsulfonyl)phenyl)-3,8-dimethyl-9H-pyrido[2,3-b]indole-7-carboxamide;
N-(3-(ethyl(2-hydroxyethyl)amino)propyl)-5-(3-(ethylsulfonyl)phenyl)-3,8-dimethyl-9H-pyrido[2,3-b]indole-7-carboxamide;
5-(3-(ethylsulfonyl)phenyl)-N-(3-((2-hydroxyethyl)(methyl)amino)propyl)-3,8-dimethyl-9H-pyrido[2,3-b]indole-7-carboxamide;
N-(2-(ethyl(2-hydroxyethyl)amino)ethyl)-5-(3-(ethylsulfonyl)phenyl)-3,8-dimethyl-9H-pyrido[2,3-b]indole-7-carboxamide; and
5-(3-(ethylsulfonyl)phenyl)-N-(2-((2-hydroxyethyl)(methyl)amino)ethyl)-3,8-dimethyl-9H-pyrido[2,3-b]indole-7-carboxamide.

In addition, particular examples of compounds according to the present invention include, but are not limited to:
5-(3-(ethylsulfonyl)phenyl)-3,8-dimethyl-N-(1-methylpiperidin-4-yl)-9H-pyrido[2,3-b]indole-7-carboxamide;
N-(2-(methoxy)ethyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-(3-(4-methylpiperazin-1-yl)propyl)-9H-pyrido[2,3-b]indole-7-carboxamide;
(S)-5-(3-(ethylsulfonyl)phenyl)-N-(2-hydroxypropyl)-3,8-dimethyl- 9H-pyrido[2,3-b]indole-7-carboxamide;
5-(3-(ethylsulfonyl)phenyl)-N-(2-hydroxyethyl)-3,8-dimethyl- 9H-pyrido[2,3-b]indole-7-carboxamide;
N-(1-ethylpiperidin-4-yl)-5-(3-(ethylsulfonyl)phenyl)-3,8-dimethyl- 9H-pyrido[2,3-b]indole-7-carboxamide;
5-(3-(cyclopropanecarboxamido)phenyl)-N-(2-(dimethylamino)ethyl)-3,8-dimethyl-9H-pyrido[2,3-b]indole-7-carboxamide;
N-(2-(dimethylamino)ethyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-((1-methylpiperidin-4-yl)methyl)-9H-pyrido[2,3-b]indole-7-carboxamide;
(S)-5-(3-(ethylsulfonyl)phenyl)-3,8-dimethyl-N-(1-methylpiperidin-3-yl)-9H-pyrido[2,3-b]indole-7-carboxamide;
5-(3-(cyclopropanecarboxamido)phenyl)-3,8-dimethyl-N-(1-methyl-piperidin-4-yl)-9H-pyrido[2,3-b]indole-7-carboxamide;
5-(3-Ethanesulfonyl-phenyl)-3-methyl-9H-pyrido[2,3-b]indole-7-carboxylic acid amide;
5-(3-Ethanesulfonyl-phenyl)-3-methyl-9H-pyrido[2,3-b]indole-7-carboxylic acid (2-dimethylamino-ethyl)-amide;
5-(3-(N-ethylsulfamoyl)phenyl)-8-methoxy-3-methyl-N-(1-methylpiperidin-4-yl)-9H-pyrido[2,3-b]indole-7-carboxamide; and
5-(3-(cyclopropylsulfonyl)phenyl)-3,8-dimethyl-N-(1-methylpiperidin-4-yl)-9H-pyrido[2,3-b]indole-7-carboxamide.

It is noted that the compounds of the present invention may be in the form of a pharmaceutically acceptable salt, biohydrolyzable ester, biohydrolyzable amide, biohydrolyzable carbamate, solvate or hydrate thereof.

In one particular variation, the compound is in the form of a salt selected from the group consisting of a hydrochloric acid salt, a trifluoroacetic acid salt, a toluenesulfonic acid salt, a benzenesulfonic acid salt, a methanesulfonic acid salt, a succinic acid salt, a tartaric acid salt, a citric acid salt, a fumaric acid salt, a sulfuric acid salt, a phosphoric acid salt, a benzoic acid salt, a bis-hydrogen chloride salt, a *bis*-trifluoroacetic acid salt, a tosylate salt, a hemi-fumarate salt, a lactic acid salt, a malic acid salt, a hippuric acid salt and a hydrobromic acid salt.

In another particular variation, the compound is in the form of a salt selected from the group consisting of a hydrochloric acid salt, a toluenesulfonic acid salt, a hemifumarate salt, and a hippuric acid salt. In still another particular variation, the compound is in the form of a hydrochloric acid salt. In one particular variation, the hydrochloric acid salt is formed in acetonitrile. In yet another particular variation, the compound is in the form of a hemi-fumarate salt. In one particular variation, the hemi-fumarate salt is formed in methanol.

It is further noted that the compounds of the present invention may optionally be solely or predominantly in the enol tautomer in its active state. It is further noted that the compound may be present in a mixture of stereoisomers, or the compound may comprise a single stereoisomer.

The invention also provides pharmaceutical compositions comprising, as an active ingredient, a compound according to any one of the above embodiments and variations. In addition, the composition may be a solid or liquid formulation adapted for oral administration. In a further variation, the pharmaceutical composition may be a tablet. In yet another variation, the pharmaceutical composition may be a liquid formulation adapted for parenteral administration.

In one embodiment, there is provided the pharmaceutical composition comprising a compound according to any one of the above embodiments and variations wherein the composition is adapted for administration by a route selected from the group consisting of orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, and intrathecally.

In another embodiment, there is provided the pharmaceutical composition comprising:
a compound having the formula 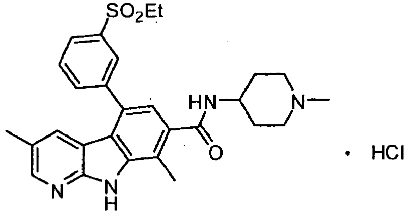
wherein at least a portion of the compound is present as Amorphous Form, characterized by physical properties which comprise one or more of the following:
   (a) may be formed by lyophilizing a solution of Compound 88 in ACN and water;
   (b) has an XRPD spectrum characterized by a diffuse halo with no discernable peaks; and/or
   (c) shows 7.6 wt% Cl⁻ present using ion chromatography.

In still another embodiment, there is provided the pharmaceutical composition comprising:
a compound having the formula 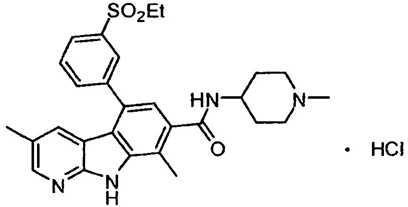 wherein at least a portion of the compound is present as Amorphous Form characterized by physical properties which compriseone or more of the following:
   (a) may be formed by lyophilizing a solution of Compound 88 in ACN and water;
   (b) has an XRPD spectrum characterized by a diffuse halo with no discernable peaks; and/or
   (c) shows 7.6 wt% Cl⁻ present using ion chromatography; and
one or more pharmaceutical carriers.

In one variation of the above embodiments, between 0.1% and 100% of the compound (by weight) is present in the composition as Amorphous Form. In a further variation of the above embodiments, between 0.1 % and 99% of the compound (by weight) is present in the composition as Amorphous Form. In still another variation of the above embodiments, greater than 0.1% of the compound (by weight) is present in the composition as Amorphous Form. In yet another variation of the above embodiments, greater than 1% of the compound (by weight) is present in the composition as Amorphous Form. In another variation of the above embodiments, greater than 5% of the compound (by weight) is present in the composition as Amorphous Form. In still another variation of the above embodiments, greater than 10% of the compound (by weight) is present in the composition as Amorphous Form. In yet another variation of the above embodiments, greater than 50% of the compound (by weight) is present in the composition as Amorphous Form. In a further variation of the above embodiments, greater than 75% of the compound (by weight) is present in the composition as Amorphous Form. In still a further variation of the above embodiments, greater than 90% of C the compound (by weight) is present in the composition as Amorphous Form. In yet a further variation of the above embodiments, greater than 99% of the compound (by weight) is present in the composition as Amorphous Form. In another variation of the above embodiments, greater than 99% of the compound (by weight) is present in the composition as Amorphous Form.

In a further variation of the above embodiments and variations, the composition is a pill or capsule adapted for oral administration. In still a further variation of the above embodiments and variations, the composition is in an oral dosage form selected from the group consisting of pills, tablets, capsules, emulsions, suspensions, microsuspensions, wafers, sprinkles, chewing gum, powders, lyophilized powders, granules, and troches. In yet a further variation of the above embodiments and variations, the composition is in a parenteral dosage form selected from the group consisting of suspensions, microsuspensions, emulsions, solid forms suitable for suspension or emulsification prior to injection, and implantable devices. In another variation of the above embodiments and variations, the composition is adapted for topical or transdermal administration. In still another variation of the above embodiments and variations, the composition is in a topical or transdermal dosage form selected from the group consisting of suspensions, microsuspensions, emulsions, creams, gels, ointments, lotions, tinctures, pastes, powders, foams, aerosols, irrigations, sprays, suppositories, bandages, and dermal patches. In still another variation of the above embodiments and variations, the composition is in a pulmonary dosage form selected from the group consisting of powders, aerosols, suspensions, microsuspensions, and emulsions.

In yet another variation of the above embodiments and variations, the polymorphic form of the compound is at least partially preserved for a period of time following administration.

The invention also provides a kit comprising a compound or composition according to any one of the above embodiments and variations, and instructions which comprise one or more forms of information selected from the group consisting of indicating a disease state for which the compound is to be administered, storage information for the compound, dosing information and instructions regarding how to administer the compound. In one variation, the kit comprises the compound or composition in a multiple dose form.

In another embodiment, the present invention provides an article of manufacture comprising a compound or composition according to any one of the above embodiments and variations, and packaging materials. In one variation, the packaging material comprises a container for housing the compound or composition. The container optionally comprises a label indicating a disease state for which the compound or composition is to be administered, storage information, dosing information and/or instructions regarding how to administer the compound or composition. In regard to the above embodiments and variations, the article of manufacture optionally comprises the compound or composition in a multiple dose form.

In another embodiment, the present invention provides a compound or composition according to any one of the above embodiments and variations for use a medicament.

In another embodiment, there is provided a compound or composition of the present invention for use in treating cancer. In one embodiment, the cancer is selected from the group consisting of squamous cell carcinoma, astrocytoma, Kaposi's sarcoma, glioblastoma, non small-cell lung cancer, bladder cancer, head and neck cancer, melanoma, ovarian cancer, prostate cancer, breast cancer, small-cell lung cancer, glioma, colorectal cancer, genitourinary cancer, gastrointestinal cancer, thyroid cancer, skin cancer, kidney cancer, rectal cancer, colonic cancer, cervical cancer, mesothelioma, pancreatic cancer, liver cancer, uterus cancer, cerebral tumor cancer, urinary bladder cancer and blood cancers including multiple myeloma. In particular embodiments, the compound is useful for inhibiting growth of cancer, for suppressing metastasis of cancer, for suppressing apoptosis and the like.

In another embodiment, there is provided a compound or composition of the present invention for use in treating inflammation, inflammatory bowel disease, psoriasis, or transplant rejection.

In another embodiment, there is provided a compound or composition of the present invention for use in preventing or treating amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss and contraceptive medication.

In yet another embodiment, there is provided a compound or composition of the present invention for use in preventing or treating mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment and androgenetic alopecia.

In a further embodiment, there is provided a compound or composition of the present invention for use in preventing or treating dementia related diseases, Alzheimer's Disease and conditions associated with kinases. In one particular variation, the dementia related diseases are selected from the group consisting of Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica.

In another embodiment, there is provided a compound or composition of the present invention for use in treating arthritis.

In still another embodiment, there is provided a compound according to any one of the above embodiments and variations for use as a medicament.

In still a further embodiment, there is provided a compound according to any one of the above embodiments and variations for use in the manufacture of a medicament for treating cancer, inflammation, inflammatory bowel disease, psoriasis, transplant rejection, amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss, contraception, mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment, cognitive impairment, androgenetic alopecia, dementia related diseases, and Alzheimer's Disease.

### Salts and Hydrates of Kinase Inhibitors

It should be recognized that the compounds of the present invention may be present and optionally administered in the form of salts and hydrates that are converted *in vivo* into the compounds of the present invention. For example, it is within the scope of the present invention to convert the compounds of the present invention into and use them in the form of their pharmaceutically acceptable salts derived from various organic and inorganic acids and bases in accordance with procedures well known in the art.

When the compounds of the present invention possess a free base form, the compounds can be prepared as a pharmaceutically acceptable acid addition salt by reacting the free base form of the compound with a pharmaceutically acceptable inorganic or organic acid, e.g., hydrohalides such as hydrochloride, hydrobromide, hydroiodide; other mineral acids and their corresponding salts such as sulfate, nitrate, phosphate, etc.; and alkyl and monoarylsulfonates such as ethanesulfonate, toluenesulfonate and benzenesulfonate; and other organic acids and their corresponding salts such as acetate, tartrate, maleate, succinate, citrate, benzoate, salicylate and ascorbate. Further acid addition salts of the present invention include, but are not limited to: adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate (from mucic acid), galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate. It should be recognized that the free base forms will typically differ from their respective salt forms somewhat in physical properties such as solubility in polar solvents, but otherwise the salts are equivalent to their respective free base forms for the purposes of the present invention.

When the compounds of the present invention possess a free acid form, a pharmaceutically acceptable base addition salt can be prepared by reacting the free acid form of the compound with a pharmaceutically acceptable inorganic or organic base. Examples of such bases are alkali metal hydroxides including potassium, sodium and lithium hydroxides; alkaline earth metal hydroxides such as barium and calcium hydroxides; alkali metal alkoxides, e.g. potassium ethanolate and sodium propanolate; and various organic bases such as ammonium hydroxide, piperidine, diethanolamine and N-methylglutamine. Also included are the aluminum salts of the compounds of the present invention. Further base salts of the present invention include, but are not limited to: copper, ferric, ferrous, lithium, magnesium, manganic, manganous, potassium, sodium and zinc salts. Organic base salts include, but are not limited to, salts of primary, secondary and tertiary amines, substituted amines including naturally occurring substituted amines, cyclic amines and basic ion exchange resins, e.g., arginine, betaine, caffeine, chloroprocaine, choline, N,N'-dibenzylethylenediamine (benzathine), dicyclohexylamine, diethanolamine, 2-diethylaminoethanol, 2-dimethylaminoethanol, ethanolamine, ethylenediamine, N-ethylmorpholine, N-ethylpiperidine, glucamine, glucosamine, histidine, hydrabamine, iso-propylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris-(hydroxymethyl)-methylamine (tromethamine). It should be recognized that the free acid forms will typically differ from their respective salt forms somewhat in physical properties such as solubility in polar solvents, but otherwise the salts are equivalent to their respective free acid forms for the purposes of the present invention.

Compounds of the present invention that comprise basic nitrogen-containing groups may be quaternized with such agents as (C₁₋₄) alkyl halides, e.g., methyl, ethyl, isopropyl and tert-butyl chlorides, bromides and iodides; di (C₁₋₄) alkyl sulfates, e.g., dimethyl, diethyl and diamyl sulfates; (C₁₀₋₁₈) alkyl halides, e.g., decyl, dodecyl, lauryl, myristyl and stearyl chlorides, bromides and iodides; and aryl (C₁₋₄) alkyl halides, e.g., benzyl chloride and phenethyl bromide. Such salts permit the preparation of both watersoluble and oil-soluble compounds of the present invention.

N-oxides of compounds according to the present invention can be prepared by methods known to those of ordinary skill in the art. For example, N-oxides can be prepared by treating an unoxidized form of the compound with an oxidizing agent (e.g., trifluoroperacetic acid, permaleic acid, perbenzoic acid, peracetic acid, meta-chloroperoxybenzoic acid, or the like) in a suitable inert organic solvent (e.g., a halogenated hydrocarbon such as dichloromethane) at approximately 0 °C. Alternatively, the N-oxides of the compounds can be prepared from the N-oxide of an appropriate starting material.

Protected derivatives of compounds of the present invention can also be made. Examples of techniques applicable to the creation of protecting groups and their removal can be found in T.W. Greene, Protecting Groups in Organic Synthesis, 3rd edition, John Wiley & Sons, Inc. 1999.

Compounds of the present invention may also be conveniently prepared, or formed during the process of the invention, as solvates (e.g. hydrates). Hydrates of compounds of the present invention may be conveniently prepared by recrystallization from an aqueous/organic solvent mixture, using organic solvents such as dioxin, tetrahydrofuran or methanol.

A "pharmaceutically acceptable salt", as used herein, is intended to encompass any compound according to the present invention that is utilized in the form of a salt thereof, especially where the salt confers on the compound improved pharmacokinetic properties as compared to the free form of compound or a different salt form of the compound. The pharmaceutically acceptable salt form may also initially confer desirable pharmacokinetic properties on the compound that it did not previously possess, and may even positively affect the pharmacodynamics of the compound with respect to its therapeutic activity in the body. An example of a pharmacokinetic property that may be favorably affected is the manner in which the compound is transported across cell membranes, which in turn may directly and positively affect the absorption, distribution, biotransformation and excretion of the compound. While the route of administration of the pharmaceutical composition is important, and various anatomical, physiological and pathological factors can critically affect bioavailability, the solubility of the compound is usually dependent upon the character of the particular salt form thereof, which it utilized. One of skill in the art will appreciate that an aqueous solution of the compound will provide the most rapid absorption of the compound into the body of a subject being treated, while lipid solutions and suspensions, as well as solid dosage forms, will result in less rapid absorption of the compound.

### PREPARATION OF KINASE INHIBITORS

Various methods may be developed for synthesizing compounds according to the present invention. Representative methods for synthesizing these compounds are provided in the Examples. It is noted, however, that the compounds of the present invention may also be synthesized by other synthetic routes that others may devise.

It will be readily recognized that certain compounds according to the present invention have atoms with linkages to other atoms that confer a particular stereochemistry to the compound (e.g., chiral centers). It is recognized that synthesis of compounds according to the present invention may result in the creation of mixtures of different stereoisomers (enantiomers, diastereomers). Unless a particular stereochemistry is specified, recitation of a compound is intended to encompass all of the different possible stereoisomers.

Various methods for separating mixtures of different stereoisomers are known in the art. For example, a racemic mixture of a compound may be reacted with an optically active resolving agent to form a pair of diastereoisomeric compounds. The diastereomers may then be separated in order to recover the optically pure enantiomers. Dissociable complexes may also be used to resolve enantiomers (e.g., crystalline diastereoisomeric salts). Diastereomers typically have sufficiently distinct physical properties (e.g., melting points, boiling points, solubilities, reactivity, etc.) that they can be readily separated by taking advantage of these dissimilarities. For example, diastereomers can typically be separated by chromatography or by separation/resolution techniques based upon differences in solubility. A more detailed description of techniques that can be used to resolve stereoisomers of compounds from their racemic mixture can be found in Jean Jacques Andre Collet, Samuel H. Wilen, Enantiomers, Racemates and Resolutions, John Wiley & Sons, Inc. (1981).

### COMPOSITION COMPRISING KINASE INHIBITORS

A wide variety of compositions and administration methods may be used in conjunction with the kinase inhibitors of the present invention. Such compositions may include, in addition to the kinase inhibitors of the present invention, conventional pharmaceutical excipients, and other conventional, pharmaceutically inactive agents. Additionally, the compositions may include active agents in addition to the kinase inhibitors of the present invention. These additional active agents may include additional compounds according to the invention, and/or one or more other pharmaceutically active agents.

The compositions may be in gaseous, liquid, semi-liquid or solid form, formulated in a manner suitable for the route of administration to be used. For oral administration, capsules and tablets are typically used. For parenteral administration, reconstitution of a lyophilized powder, prepared as described herein, is typically used.

Compositions comprising kinase inhibitors of the present invention may be administered or coadministered orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, or intrathecally. The compounds and/or compositions according to the invention may also be administered or coadministered in slow release dosage forms.

The kinase inhibitors and compositions comprising them may be administered or coadministered in any conventional dosage form. Co-administration in the context of this invention is intended to mean the administration of more than one therapeutic agent, one of which includes a kinase inhibitor, in the course of a coordinated treatment to achieve an improved clinical outcome. Such co-administration may also be coextensive, that is, occurring during overlapping periods of time.

Solutions or suspensions used for parenteral, intradermal, subcutaneous, or topical application may optionally include one or more of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; agents for the adjustment of tonicity such as sodium chloride or dextrose, and agents for adjusting the acidity or alkalinity of the composition, such as alkaline or acidifying agents or buffers like carbonates, bicarbonates, phosphates, hydrochloric acid, and organic acids like acetic and citric acid. Parenteral preparations may optionally be enclosed in ampules, disposable syringes or single or multiple dose vials made of glass, plastic or other suitable material.

When kinase inhibitors according to the present invention exhibit insufficient solubility, methods for solubilizing the compounds may be used. Such methods are known to those of skill in this art, and include, but are not limited to, using cosolvents, such as dimethylsulfoxide (DMSO), using surfactants, such as TWEEN, or dissolution in aqueous sodium bicarbonate. Derivatives of the compounds, such as prodrugs of the compounds may also be used in formulating effective pharmaceutical compositions.

Upon mixing or adding kinase inhibitors according to the present invention to a composition, a solution, suspension, emulsion or the like may be formed. The form of the resulting composition will depend upon a number of factors, including the intended mode of administration, and the solubility of the compound in the selected carrier or vehicle. The effective concentration needed to ameliorate the disease being treated may be empirically determined.

Compositions according to the present invention are optionally provided for administration to humans and animals in unit dosage forms, such as tablets, capsules, pills, powders, dry powders for inhalers, granules, sterile parenteral solutions or suspensions, and oral solutions or suspensions, and oil-water emulsions containing suitable quantities of the compounds, particularly the pharmaceutically acceptable salts, preferably the sodium salts, thereof. The pharmaceutically therapeutically active compounds and derivatives thereof are typically formulated and administered in unit-dosage forms or multiple-dosage forms. Unit-dose forms, as used herein, refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes individually packaged tablet or capsule. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pint or gallons. Hence, multiple dose form is a multiple of unit-doses that are not segregated in packaging.

In addition to one or more kinase inhibitors according to the present invention, the composition may comprise: a diluent such as lactose, sucrose, dicalcium phosphate, or carboxymethylcellulose; a lubricant, such as magnesium stearate, calcium stearate and talc; and a binder such as starch, natural gums, such as gum acaciagelatin, glucose, molasses, polvinylpyrrolidine, celluloses and derivatives thereof, povidone, crospovidones and other such binders known to those of skill in the art. Liquid pharmaceutically administrable compositions can, for example, be prepared by dissolving, dispersing, or otherwise mixing an active compound as defined above and optional pharmaceutical adjuvants in a carrier, such as, for example, water, saline, aqueous dextrose, glycerol, glycols, ethanol, and the like, to form a solution or suspension. If desired, the pharmaceutical composition to be administered may also contain minor amounts of auxiliary substances such as wetting agents, emulsifying agents, or solubilizing agents, pH buffering agents and the like, for example, acetate, sodium citrate, cyclodextrine derivatives, sorbitan monolaurate, triethanolamine sodium acetate, triethanolamine oleate, and other such agents. Actual methods of preparing such dosage forms are known in the art, or will be apparent, to those skilled in this art; for example, see Remington's Pharmaceutical Sciences, Mack Publishing Company, Easton, Pa., 15th Edition, 1975. The composition or formulation to be administered will, in any event, contain a sufficient quantity of a kinase inhibitor of the present invention to reduce kinases activity *in vivo*, thereby treating the disease state of the subject.

Dosage forms or compositions may optionally comprise one or more kinase inhibitors according to the present invention in the range of 0.005% to 100% (weight/weight) with the balance comprising additional substances such as those described herein. For oral administration, a pharmaceutically acceptable composition may optionally comprise any one or more commonly employed excipients, such as, for example pharmaceutical grades of mannitol, lactose, starch, magnesium stearate, talcum, cellulose derivatives, sodium crosscarmellose, glucose, sucrose, magnesium carbonate, sodium saccharin, talcum. Such compositions include solutions, suspensions, tablets, capsules, powders, dry powders for inhalers and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparing these formulations are known to those skilled in the art. The compositions may optionally contain 0.01%-100% (weight/weight) of one or more kinase inhibitors, optionally 0.1-95%, and optionally 1-95%.

In one variation, the composition comprises at least 0.1%, 0.25%, 0.5%, 1%, 5%, 10%, 25%, 50%, 75%, 80%, 85%, 90%, 95%, 97%, or 99% (by weight) of one or more kinase inhibitors according to the present invention. In particular variations, greater than 0.1%, 1%, 5%, 10%, 25%, 50%, 75%, 80%, 85%, 90%, 95%, 97% or 99% (by weight) of one or more kinase inhibitors according to the present invention is present in the composition as a single crystalline or amorphous form. The composition may optionally be a pharmaceutical composition. The pharmaceutical composition may optionally further include one or more pharmaceutical carriers.

Salts, preferably sodium salts, of the kinase inhibitors may be prepared with carriers that protect the compound against rapid elimination from the body, such as time release formulations or coatings. The formulations may further include other active compounds to obtain desired combinations of properties.

### Formulations for Oral Administration

Oral pharmaceutical dosage forms may be as a solid, gel or liquid. Examples of solid dosage forms include, but are not limited to tablets, capsules, granules, and bulk powders. More specific examples of oral tablets include compressed, chewable lozenges and tablets that may be enteric-coated, sugar-coated or film-coated. Examples of capsules include hard or soft gelatin capsules. Granules and powders may be provided in non-effervescent or effervescent forms. Each may be combined with other ingredients known to those skilled in the art.

In certain embodiments, kinase inhibitors according to the present invention are provided as solid dosage forms, preferably capsules or tablets. The tablets, pills, capsules, troches and the like may optionally contain one or more of the following ingredients, or compounds of a similar nature: a binder; a diluent; a disintegrating agent; a lubricant; a glidant; a sweetening agent; and a flavoring agent.

Examples of binders that may be used include, but are not limited to, microcrystalline cellulose, gum tragacanth, glucose solution, acacia mucilage, gelatin solution, sucrose and starch paste.

Examples of lubricants that may be used include, but are not limited to, talc, starch, magnesium or calcium stearate, lycopodium and stearic acid.

Examples of diluents that may be used include, but are not limited to, lactose, sucrose, starch, kaolin, salt, mannitol and dicalcium phosphate.

Examples of glidants that may be used include, but are not limited to, colloidal silicon dioxide.

Examples of disintegrating agents that may be used include, but are not limited to, crosscarmellose sodium, sodium starch glycolate, alginic acid, corn starch, potato starch, bentonite, methylcellulose, agar and carboxymethylcellulose.

Examples of coloring agents that may be used include, but are not limited to, any of the approved certified water soluble FD and C dyes, mixtures thereof; and water insoluble FD and C dyes suspended on alumina hydrate.

Examples of sweetening agents that may be used include, but are not limited to, sucrose, lactose, mannitol and artificial sweetening agents such as sodium cyclamate and saccharin, and any number of spray-dried flavors.

Examples of flavoring agents that may be used include, but are not limited to, natural flavors extracted from plants such as fruits and synthetic blends of compounds that produce a pleasant sensation, such as, but not limited to peppermint and methyl salicylate.

Examples of wetting agents that may be used include, but are not limited to, propylene glycol monostearate, sorbitan monooleate, diethylene glycol monolaurate and polyoxyethylene lauryl ether.

Examples of anti-emetic coatings that may be used include, but are not limited to, fatty acids, fats, waxes, shellac, ammoniated shellac and cellulose acetate phthalates.

Examples of film coatings that may be used include, but are not limited to, hydroxyethylcellulose, sodium carboxymethylcellulose, polyethylene glycol 4000 and cellulose acetate phthalate.

If oral administration is desired, the salt of the compound may optionally be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it may optionally additionally comprise a liquid carrier such as a fatty oil. In addition, dosage unit forms may optionally additionally comprise various other materials that modify the physical form of the dosage unit, for example, coatings of sugar and other enteric agents.

Compounds according to the present invention may also be administered as a component of an elixir, suspension, syrup, wafer, sprinkle, chewing gum or the like. A syrup may optionally comprise, in addition to the active compounds, sucrose as a sweetening agent and certain preservatives, dyes and colorings and flavors.

The kinase inhibitors of the present invention may also be mixed with other active materials that do not impair the desired action, or with materials that supplement the desired action, such as antacids, H2 blockers, and diuretics. For example, if a compound is used for treating asthma or hypertension, it may be used with other bronchodilators and antihypertensive agents, respectively.

Examples of pharmaceutically acceptable carriers that may be included in tablets comprising kinase inhibitors of the present invention include, but are not limited to binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets may be compressed tablets to which different layers of pharmaceutically acceptable substances are applied. Film-coated tablets may be compressed tablets that have been coated with polymers or other suitable coating. Multiple compressed tablets may be compressed tablets made by more than one compression cycle utilizing the pharmaceutically acceptable substances previously mentioned. Coloring agents may also be used in tablets. Flavoring and sweetening agents may be used in tablets, and are especially useful in the formation of chewable tablets and lozenges.

Examples of liquid oral dosage forms that may be used include, but are not limited to, aqueous solutions, emulsions, suspensions, solutions and/or suspensions reconstituted from non-effervescent granules and effervescent preparations reconstituted from effervescent granules.

Examples of aqueous solutions that may be used include, but are not limited to, elixirs and syrups. As used herein, elixirs refer to clear, sweetened, hydroalcoholic preparations. Examples of pharmaceutically acceptable carriers that may be used in elixirs include, but are not limited to solvents. Particular examples of solvents that may be used include glycerin, sorbitol, ethyl alcohol and syrup. As used herein, syrups refer to concentrated aqueous solutions of a sugar, for example, sucrose. Syrups may optionally further comprise a preservative.

Emulsions refer to two-phase systems in which one liquid is dispersed in the form of small globules throughout another liquid. Emulsions may optionally be oil-in-water or water-in-oil emulsions. Examples of pharmaceutically acceptable carriers that may be used in emulsions include, but are not limited to non-aqueous liquids, emulsifying agents and preservatives.

Examples of pharmaceutically acceptable substances that may be used in non-effervescent granules, to be reconstituted into a liquid oral dosage form, include diluents, sweeteners and wetting agents.

Examples of pharmaceutically acceptable substances that may be used in effervescent granules, to be reconstituted into a liquid oral dosage form, include organic acids and a source of carbon dioxide.

Coloring and flavoring agents may optionally be used in all of the above dosage forms.

Particular examples of preservatives that may be used include glycerin, methyl and propylparaben, benzoic add, sodium benzoate and alcohol.

Particular examples of non-aqueous liquids that may be used in emulsions include mineral oil and cottonseed oil.

Particular examples of emulsifying agents that may be used include gelatin, acacia, tragacanth, bentonite, and surfactants such as polyoxyethylene sorbitan monooleate.

Particular examples of suspending agents that may be used include sodium carboxymethylcellulose, pectin, tragacanth, Veegum and acacia. Diluents include lactose and sucrose. Sweetening agents include sucrose, syrups, glycerin and artificial sweetening agents such as sodium cyclamate and saccharin.

Particular examples of wetting agents that may be used include propylene glycol monostearate, sorbitan monooleate, diethylene glycol monolaurate and polyoxyethylene lauryl ether.

Particular examples of organic acids that may be used include citric and tartaric acid.

Sources of carbon dioxide that may be used in effervescent compositions include sodium bicarbonate and sodium carbonate. Coloring agents include any of the approved certified water soluble FD and C dyes, and mixtures thereof.

Particular examples of flavoring agents that may be used include natural flavors extracted from plants such fruits, and synthetic blends of compounds that produce a pleasant taste sensation.

For a solid dosage form, the solution or suspension, in for example propylene carbonate, vegetable oils or triglycerides, is preferably encapsulated in a gelatin capsule. Such solutions, and the preparation and encapsulation thereof, are disclosed in U.S. Pat. Nos. 4,328,245; 4,409,239; and 4,410,545. For a liquid dosage form, the solution, e.g., for example, in a polyethylene glycol, may be diluted with a sufficient quantity of a pharmaceutically acceptable liquid carrier, e.g. water, to be easily measured for administration.

Alternatively, liquid or semi-solid oral formulations may be prepared by dissolving or dispersing the active compound or salt in vegetable oils, glycols, triglycerides, propylene glycol esters (e.g. propylene carbonate) and other such carriers, and encapsulating these solutions or suspensions in hard or soft gelatin capsule shells. Other useful formulations include those set forth in U.S. Pat. Nos. Re 28,819 and 4,358,603.

### Injectables, Solutions, and Emulsions

The present invention is also directed to compositions designed to administer the kinase inhibitors of the present invention by parenteral administration, generally characterized by injection, either subcutaneously, intramuscularly or intravenously. Injectables may be prepared in any conventional form, for example as liquid solutions or suspensions, solid forms suitable for solution or suspension in liquid prior to injection, or as emulsions.

Examples of excipients that may be used in conjunction with injectables according to the present invention include, but are not limited to water, saline, dextrose, glycerol or ethanol. The injectable compositions may also optionally comprise minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. Implantation of a slow-release or sustained-release system, such that a constant level of dosage is maintained (see, e.g., U.S. Pat. No. 3,710,795) is also contemplated herein. The percentage of active compound contained in such parenteral compositions is highly dependent on the specific nature thereof, as well as the activity of the compound and the needs of the subject.

Parenteral administration of the formulations includes intravenous, subcutaneous and intramuscular administrations. Preparations for parenteral administration include sterile solutions ready for injection, sterile dry soluble products, such as the lyophilized powders described herein, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

When administered intravenously, examples of suitable carriers include, but are not limited to physiological saline or phosphate buffered saline (PBS), and solutions containing thickening and solubilizing agents, such as glucose, polyethylene glycol, and polypropylene glycol and mixtures thereof.

Examples of pharmaceutically acceptable carriers that may optionally be used in parenteral preparations include, but are not limited to aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles that may optionally be used include Sodium Chloride Injection, Ringers Injection, Isotonic Dextrose Injection, Sterile Water Injection, Dextrose and Lactated Ringers Injection.

Examples of nonaqueous parenteral vehicles that may optionally be used include fixed oils of vegetable origin, cottonseed oil, corn oil, sesame oil and peanut oil.

Antimicrobial agents in bacteriostatic or fungistatic concentrations may be added to parenteral preparations, particularly when the preparations are packaged in multiple-dose containers and thus designed to be stored and multiple aliquots to be removed. Examples of antimicrobial agents that may be used include phenols or cresols, mercurials, benzyl alcohol, chlorobutanol, methyl and propyl p-hydroxybenzoic acid esters, thimerosal, benzalkonium chloride and benzethonium chloride.

Examples of isotonic agents that may be used include sodium chloride and dextrose. Examples of buffers that may be used include phosphate and citrate. Examples of antioxidants that may be used include sodium bisulfate. Examples of local anesthetics that may be used include procaine hydrochloride. Examples of suspending and dispersing agents that may be used include sodium carboxymethylcellulose, hydroxypropyl methylcellulose and polyvinylpyrrolidone. Examples of emulsifying agents that may be used include Polysorbate 80 (TWEEN 80). A sequestering or chelating agent of metal ions include EDTA.

Pharmaceutical carriers may also optionally include ethyl alcohol, polyethylene glycol and propylene glycol for water miscible vehicles and sodium hydroxide, hydrochloric acid, citric acid or lactic acid for pH adjustment.

The concentration of a kinase inhibitor in the parenteral formulation may be adjusted so that an injection administers a pharmaceutically effective amount sufficient to produce the desired pharmacological effect. The exact concentration of a kinase inhibitor and/or dosage to be used will ultimately depend on the age, weight and condition of the patient or animal as is known in the art.

Unit-dose parenteral preparations may be packaged in an ampoule, a vial or a syringe with a needle. All preparations for parenteral administration should be sterile, as is know and practiced in the art.

Injectables may be designed for local and systemic administration. Typically a therapeutically effective dosage is formulated to contain a concentration of at least about 0.1 % w/w up to about 90% w/w or more, preferably more than 1% w/w of the kinase inhibitor to the treated tissue(s). The kinase inhibitor may be administered at once, or may be divided into a number of smaller doses to be administered at intervals of time. It is understood that the precise dosage and duration of treatment will be a function of the location of where the composition is parenterally administered, the carrier and other variables that may be determined empirically using known testing protocols or by extrapolation from *in vivo* or *in vitro* test data. It is to be noted that concentrations and dosage values may also vary with the age of the individual treated. It is to be further understood that for any particular subject, specific dosage regimens may need to be adjusted over time according to the individual need and the professional judgment of the person administering or supervising the administration of the formulations. Hence, the concentration ranges set forth herein are intended to be exemplary and are not intended to limit the scope or practice of the claimed formulations.

The kinase inhibitor may optionally be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the disease state and may be empirically determined.

### Lyophilized Powders

The kinase inhibitors of the present invention may also be prepared as lyophilized powders, which can be reconstituted for administration as solutions, emulsions and other mixtures. The lyophilized powders may also be formulated as solids or gels.

Sterile, lyophilized powder may be prepared by dissolving the compound in a sodium phosphate buffer solution containing dextrose or other suitable excipient. Subsequent sterile filtration of the solution followed by lyophilization under standard conditions known to those of skill in the art provides the desired formulation. Briefly, the lyophilized powder may optionally be prepared by dissolving dextrose, sorbitol, fructose, corn syrup, xylitol, glycerin, glucose, sucrose or other suitable agent, about 1-20%, preferably about 5 to 15%, in a suitable buffer, such as citrate, sodium or potassium phosphate or other such buffer known to those of skill in the art at, typically, about neutral pH. Then, a kinase inhibitor is added to the resulting mixture, preferably above room temperature, more preferably at about 30-35 °C, and stirred until it dissolves. The resulting mixture is diluted by adding more buffer to a desired concentration. The resulting mixture is sterile filtered or treated to remove particulates and to insure sterility, and apportioned into vials for lyophilization. Each vial may contain a single dosage or multiple dosages of the kinase inhibitor.

### Topical Administration

The kinase inhibitors of the present invention may also be administered as topical mixtures. Topical mixtures may be used for local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The kinase inhibitors may be formulated as aerosols for topical application, such as by inhalation (see, U.S. Pat. Nos. 4,044,126, 4,414,209, and 4,364,923, which describe aerosols for delivery of a steroid useful for treatment inflammatory diseases, particularly asthma). These formulations for administration to the respiratory tract can be in the form of an aerosol or solution for a nebulizer, or as a microfine powder for insufflation, alone or in combination with an inert carrier such as lactose. In such a case, the particles of the formulation will typically have diameters of less than 50 microns, preferably less than 10 microns.

The kinase inhibitors may also be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the kinase inhibitor alone or in combination with other pharmaceutically acceptable excipients can also be administered.

### Formulations for Other Routes of Administrations

Depending upon the disease state being treated, other routes of administration, such as topical application, transdermal patches, and rectal administration, may also be used. For example, pharmaceutical dosage forms for rectal administration are rectal suppositories, capsules and tablets for systemic effect. Rectal suppositories are used herein mean solid bodies for insertion into the rectum that melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax, (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. The typical weight of a rectal suppository is about 2 to 3 gm. Tablets and capsules for rectal administration may be manufactured using the same pharmaceutically acceptable substance and by the same methods as for formulations for oral administration.

### Examples of Formulations

The following are particular examples of oral, intravenous and tablet formulations that may optionally be used with compounds of the present invention. It is noted that these formulations may be varied depending on the particular compound being used and the indication for which the formulation is going to be used.

| ORAL FORMULATION | |
|---|---|
| Compound of the Present Invention | 10-100 mg |
| Citric Acid Monohydrate | 105 mg |
| Sodium Hydroxide | 18 mg |
| Flavoring | |
| Water | q.s. to 100 mL |

| INTRAVENOUS FORMULATION | |
|---|---|
| Compound of the Present Invention | 0.1-10 mg |
| Dextrose Monohydrate | q.s. to make isotonic |
| Citric Acid Monohydrate | 1.05 mg |
| Sodium Hydroxide | 0.18 mg |
| Water for Injection | q.s. to 1.0 mL |

| TABLET FORMULATION | |
|---|---|
| Compound of the Present Invention | 1% |
| Microcrystalline Cellulose | 73% |
| Stearic Acid | 25% |
| Colloidal Silica | 1%. |

### Kits Comprising Kinase Inhibitors

The invention is also directed to kits and other articles of manufacture for treating diseases associated with kinases. It is noted that diseases are intended to cover all conditions for which the kinases possesses activity that contributes to the pathology and/or symptomology of the condition.

In one embodiment, a kit is provided that comprises a composition comprising at least one kinase inhibitor of the present invention in combination with instructions. The instructions may indicate the disease state for which the composition is to be administered, storage information, dosing information and/or instructions regarding how to administer the composition. The kit may also comprise packaging materials. The packaging material may comprise a container for housing the composition. The kit may also optionally comprise additional components, such as syringes for administration of the composition. The kit may comprise the composition in single or multiple dose forms.

The invention also provided are kits and other articles of manufacture comprising a composition that comprises one or more compounds of the present invention, wherein the one or more compounds of the present invention are present as a single crystalline or amorphous form. In one variation, the composition comprises at least 0.1 %, 0.25%, 0.5%, 1%, 5%, 10%, 25%, 50%, 75%, 80%, 85%, 90%, 95%, 97%, or 99% of the one or more compounds of the present invention where greater than 0.1 %, 1%, 5%, 10%, 25%, 50%, 75%, 80%, 85%, 90%, 95%, 97% or 99% of the one or more compounds of the present invention (by weight) is present in the composition as a single crystalline or amorphous form. The composition in the kits and articles of manufacture may optionally be a pharmaceutical composition. The pharmaceutical composition may optionally further include one or more pharmaceutical carriers. In regard to each of the above embodiments including a pharmaceutical composition, the pharmaceutical composition may optionally be formulated such that a portion of the one or more compounds of the present invention is present as a single crystalline or amorphous form for a period of time subsequent to administration of the pharmaceutical formulation to a human.

In another embodiment, an article of manufacture is provided that comprises a composition comprising at least one kinase inhibitor of the present invention in combination with packaging materials. The packaging material may comprise a container for housing the composition. The container may optionally comprise a label indicating the disease state for which the composition is to be administered, storage information, dosing information and/or instructions regarding how to administer the composition. The kit may also optionally comprise additional components, such as syringes for administration of the composition. The kit may comprise the composition in single or multiple dose forms.

It is noted that the packaging material used in kits and articles of manufacture according to the present invention may form a plurality of divided containers such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container that is employed will depend on the exact dosage form involved, for example a conventional cardboard box would not generally be used to hold a liquid suspension. It is feasible that more than one container can be used together in a single package to market a single dosage form. For example, tablets may be contained in a bottle that is in turn contained within a box. Typically the kit includes directions for the administration of the separate components. The kit form is particularly advantageous when the separate components are preferably administered in different dosage forms (e.g., oral, topical, transdermal and parenteral), are administered at different dosage intervals, or when titration of the individual components of the combination is desired by the prescribing physician.

One particular example of a kit according to the present invention is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

Another specific embodiment of a kit is a dispenser designed to dispense the daily doses one at a time in the order of their intended use. Preferably, the dispenser is equipped with a memory-aid, so as to further facilitate compliance with the regimen. An example of such a memory-aid is a mechanical counter that indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

### Dosage, Host and Safety

The compounds of the present invention are stable and can be used safely. In particlar, the compounds of the present invention are useful as kinase inhibitors for a variety of subjects (*e.g.*, humans, non-human mammals and non-mammals). The optimal dose may vary depending upon such conditions as, for example, the type of subject, the body weight of the subject, the route of administration, and specific properties of the particular compound being used. In general, the daily dose for oral administration to an adult (body weight of about 60 kg) is about 1 to 1000 mg, about 3 to 300 mg, about 10 to 200 mg, about 100 to 500 mg, about 150 to 450 mg, abour 200 to 400 mg, or about 200 to 300 mg. It will be appreciated that the daily dose can be given in a single administration or in multiple (*e.g.*, 2 or 3) portions a day.

### Combination Therapies

A wide variety therapeutic agents may have a therapeutic additive or synergistic effect with kinase inhibitors according to the present invention. Combination therapies that comprise one or more compounds of the present invention with one or more other therapeutic agents can be used, for example, to: 1) enhance the therapeutic effect(s) of the one or more compounds of the present invention and/or the one or more other therapeutic agents; 2) reduce the side effects exhibited by the one or more compounds of the present invention and/or the one or more other therapeutic agents; and/or 3) reduce the effective dose of the one or more compounds of the present invention and/or the one or more other therapeutic agents. For example, such therapeutic agents may additively or synergistically combine with the kinase inhibitors to inhibit undesirable cell growth, such as inappropriate cell growth resulting in undesirable benign conditions or tumor growth.

In one embodiment, a method is provided for treating a cell proliferative disease state comprising treating cells with a compound according to the present invention in combination with an anti-proliferative agent, wherein the cells are treated with the compound according to the present invention before, at the same time, and/or after the cells are treated with the anti-proliferative agent, referred to herein as combination therapy. It is noted that treatment of one agent before another is referred to herein as sequential therapy, even if the agents are also administered together. It is noted that combination therapy is intended to cover when agents are administered before or after each other (sequential therapy) as well as when the agents are administered at the same time.

Examples of therapeutic agents that may be used in combination with kinase inhibitors include, but are not limited to, anticancer agents, alkylating agents, antibiotic agents, antimetabolic agents, hormonal agents, plant-derived agents, and biologic agents.

Alkylating agents are polyfunctional compounds that have the ability to substitute alkyl groups for hydrogen ions. Examples of alkylating agents include, but are not limited to, bischloroethylamines (nitrogen mustards, e.g. chlorambucil, cyclophosphamide, ifosfamide, mechlorethamine, melphalan, uracil mustard), aziridines (e.g. thiotepa), alkyl alkone sulfonates (e.g. busulfan), nitrosoureas (e.g. carmustine, lomustine, streptozocin), nonclassic alkylating agents (altretamine, dacarbazine, and procarbazine), platinum compounds (carboplastin and cisplatin). These compounds react with phosphate, amino, hydroxyl, sulfihydryl, carboxyl, and imidazole groups. Under physiological conditions, these drugs ionize and produce positively charged ion that attach to susceptible nucleic acids and proteins, leading to cell cycle arrest and/or cell death. Combination therapy including a kinase inhibitor and an alkylating agent may have therapeutic synergistic effects on cancer and reduce sides affects associated with these chemotherapeutic agents.

Antibiotic agents are a group of drugs that produced in a manner similar to antibiotics as a modification of natural products. Examples of antibiotic agents include, but are not limited to, anthracyclines (e.g. doxorubicin, daunorubicin, epirubicin, idarubicin and anthracenedione), mitomycin C, bleomycin, dactinomycin, plicatomycin. These antibiotic agents interfere with cell growth by targeting different cellular components. For example, anthracyclines are generally believed to interfere with the action of DNA topoisomerase II in the regions of transcriptionally active DNA, which leads to DNA strand scissions. Bleomycin is generally believed to chelate iron and forms an activated complex, which then binds to bases of DNA, causing strand scissions and cell death. Combination therapy including a kinase inhibitor and an antibiotic agent may have therapeutic synergistic effects on cancer and reduce sides affects associated with these chemotherapeutic agents.

Antimetabolic agents are a group of drugs that interfere with metabolic processes vital to the physiology and proliferation of cancer cells. Actively proliferating cancer cells require continuous synthesis of large quantities of nucleic acids, proteins, lipids, and other vital cellular constituents. Many of the antimetabolites inhibit the synthesis of purine or pyrimidine nucleosides or inhibit the enzymes of DNA replication. Some antimetabolites also interfere with the synthesis of ribonucleosides and RNA and/or amino acid metabolism and protein synthesis as well. By interfering with the synthesis of vital cellular constituents, antimetabolites can delay or arrest the growth of cancer cells. Examples of antimetabolic agents include, but are not limited to, fluorouracil (5-FU), floxuridine (5-FUdR), methotrexate, leucovorin, hydroxyurea, thioguanine (6-TG), mercaptopurine (6-MP), cytarabine, pentostatin, fludarabine phosphate, cladribine (2-CDA), asparaginase, and gemcitabine. Combination therapy including a kinase inhibitor and a antimetabolic agent may have therapeutic synergistic effects on cancer and reduce sides affects associated with these chemotherapeutic agents.

Hormonal agents are a group of drug that regulate the growth and development of their target organs. Most of the hormonal agents are sex steroids and their derivatives and analogs thereof, such as estrogens, androgens, and progestins. These hormonal agents may serve as antagonists of receptors for the sex steroids to down regulate receptor expression and transcription of vital genes. Examples of such hormonal agents are synthetic estrogens (e.g. diethylstibestrol), antiestrogens (e.g. tamoxifen, toremifene, fluoxymesterol and raloxifene), antiandrogens (bicalutamide, nilutamide, flutamide), aromatase inhibitors (e.g., aminoglutethimide, anastrozole and tetrazole), ketoconazole, goserelin acetate, leuprolide, megestrol acetate and mifepristone. Combination therapy including a kinase inhibitor and a hormonal agent may have therapeutic synergistic effects on cancer and reduce sides affects associated with these chemotherapeutic agents.

Plant-derived agents are a group of drugs that are derived from plants or modified based on the molecular structure of the agents. Examples of plant-derived agents include, but are not limited to, vinca alkaloids (e.g., vincristine, vinblastine, vindesine, vinzolidine and vinorelbine), podophyllotoxins (e.g., etoposide (VP-16) and teniposide (VM-26)), taxanes (e.g.; paclitaxel and docetaxel). These plant-derived agents generally act as antimitotic agents that bind to tubulin and inhibit mitosis. Podophyllotoxins such as etoposide are believed to interfere with DNA synthesis by interacting with topoisomerase II, leading to DNA strand scission. Combination therapy including a kinase inhibitor and a plant-derived agent may have therapeutic synergistic effects on cancer and reduce sides affects associated with these chemotherapeutic agents.

Biologic agents are a group of biomolecules that elicit cancer/tumor regression when used alone or in combination with chemotherapy and/or radiotherapy. Examples of biologic agents include, but are not limited to, immuno-modulating proteins such as cytokines, monoclonal antibodies against tumor antigens, tumor suppressor genes, and cancer vaccines. Combination therapy including a kinase inhibitor and a biologic agent may have therapeutic synergistic effects on cancer, enhance the patient's immune responses to tumorigenic signals, and reduce potential sides affects associated with this chemotherapeutic agent.

Cytokines possess profound immunomodulatory activity. Some cytokines such as interleukin-2 (IL-2, aldesleukin) and interferon have demonstrated antitumor activity and have been approved for the treatment of patients with metastatic renal cell carcinoma and metastatic malignant melanoma. IL-2 is a T-cell growth factor that is central to T-cell-mediated immune responses. The selective antitumor effects of IL-2 on some patients are believed to be the result of a cell-mediated immune response that discriminate between self and nonself. Examples of interleukins that may be used in conjunction with kinase inhibitor include, but are not limited to, interleukin 2 (IL-2), and interleukin 4 (IL-4), interleukin 12 (IL-12).

Interferon include more than 23 related subtypes with overlapping activities, all of the IFN subtypes within the scope of the present invention. IFN, has demonstrated activity against many solid and hematologic malignancies, the later appearing to be particularly sensitive.

Other cytokines that may be used in conjunction with a kinase inhibitor include those cytokines that exert profound effects on hematopoiesis and immune functions. Examples of such cytokines include, but are not limited to erythropoietin, granulocyte-CSF (filgrastin), and granulocyte, macrophage-CSF (sargramostim). These cytokines may be used in conjunction with a kinase inhibitor to reduce chemotherapy-induced myelopoietic toxicity.

Other immuno-modulating agents other than cytokines may also be used in conjunction with a kinase inhibitor to inhibit abnormal cell growth. Examples of such immuno-modulating agents include, but are not limited to bacillus Calmette-Guerin, levamisole, and octreotide, a long-acting octapeptide that mimics the effects of the naturally occurring hormone somatostatin.

Monoclonal antibodies against tumor antigens are antibodies elicited against antigens expressed by tumors, preferably tumor-specific antigens. For example, monoclonal antibody HERCEPTIN® (Trastruzumab) is raised against human epidermal growth factor receptor2 (HER2) that is overexpressed in some breast tumors including metastatic breast cancer. Overexpression of HER2 protein is associated with more aggressive disease and poorer prognosis in the clinic. HERCEPTIN® is used as a single agent for the treatment of patients with metastatic breast cancer whose tumors over express the HER2 protein. Combination therapy including kinase inhibitor and HERCEPTIN® may have therapeutic synergistic effects on tumors, especially on metastatic cancers.

Another example of monoclonal antibodies against tumor antigens is RITUXAN® (Rituximab) that is raised against CD20 on lymphoma cells and selectively deplete normal and malignant CD20⁺ pre-B and mature B cells. RITUXAN® is used as single agent for the treatment of patients with relapsed or refractory low-grade or follicular, CD20+, B cell non-Hodgkin's lymphoma. Combination therapy including kinase inhibitor and RITUXAN® may have therapeutic synergistic effects not only on lymphoma, but also on other forms or types of malignant tumors.

Tumor suppressor genes are genes that function to inhibit the cell growth and division cycles, thus preventing the development of neoplasia. Mutations in tumor suppressor genes cause the cell to ignore one or more of the components of the network of inhibitory signals, overcoming the cell cycle check points and resulting in a higher rate of controlled cell growth—cancer. Examples of the tumor suppressor genes include, but are not limited to, DPC-4, NF-1, NF-2, RB, p53, WT1, BRCA1 and BRCA2.

DPC-4 is involved in pancreatic cancer and participates in a cytoplasmic pathway that inhibits cell division. NF-1 codes for a protein that inhibits Ras, a cytoplasmic inhibitory protein. NF-1 is involved in neurofibroma and pheochromocytomas of the nervous system and myeloid leukemia. NF-2 encodes a nuclear protein that is involved in meningioma, schwanoma, and ependymoma of the nervous system. RB codes for the pRB protein, a nuclear protein that is a major inhibitor of cell cycle. RB is involved in retinoblastoma as well as bone, bladder, small cell lung and breast cancer. P53 codes for p53 protein that regulates cell division and can induce apoptosis. Mutation and/or inaction of p53 is found in a wide ranges of cancers. WT1 is involved in Wilms tumor of the kidneys. BRCA1 is involved in breast and ovarian cancer, and BRCA2 is involved in breast cancer. The tumor suppressor gene can be transferred into the tumor cells where it exerts its tumor suppressing functions. Combination therapy including a kinase inhibitor and a tumor suppressor may have therapeutic synergistic effects on patients suffering from various forms of cancers.

Cancer vaccines are a group of agents that induce the body's specific immune response to tumors. Most of cancer vaccines under research and development and clinical trials are tumor-associated antigens (TAAs). TAA are structures (i.e. proteins, enzymes or carbohydrates) which are present on tumor cells and relatively absent or diminished on normal cells. By virtue of being fairly unique to the tumor cell, TAAs provide targets for the immune system to recognize and cause their destruction. Example of TAAs include, but are not limited to gangliosides (GM2), prostate specific antigen (PSA), alpha-fetoprotein (AFP), carcinoembryonic antigen (CEA) (produced by colon cancers and other adenocarcinomas, e.g. breast, lung, gastric, and pancreas cancer s), melanoma associated antigens (MART-1, gp100, MAGE 1,3 tyrosinase), papillomavirus E6 and E7 fragments, whole cells or portions/lysates of antologous tumor cells and allogeneic tumor cells.

An adjuvant may be used to augment the immune response to TAAs. Examples of adjuvants include, but are not limited to, bacillus Calmette-Guerin (BCG), endotoxin lipopolysaccharides, keyhole limpet hemocyanin (GKLH), interleukin-2 (IL-2), granulocyte-macrophage colony-stimulating factor (GM-CSF) and cytoxan, a chemotherapeutic agent which is believe to reduce tumor-induced suppression when given in low doses.

### EXAMPLES:

### 1. Preparation of Kinase Inhibitors

Various methods may be developed for synthesizing compounds according to the present invention. Representative methods for synthesizing these compounds are provided in the Examples. It is noted, however, that the compounds of the present invention may also be synthesized by other synthetic routes that others may devise.

It will be readily recognized that certain compounds according to the present invention have atoms with linkages to other atoms that confer a particular stereochemistry to the compound (e.g., chiral centers). It is recognized that synthesis of compounds according to the present invention may result in the creation of mixtures of different stereoisomers (enantiomers, diastereomers). Unless a particular stereochemistry is specified, recitation of a compound is intended to encompass all of the different possible stereoisomers.

Various methods for separating mixtures of different stereoisomers are known in the art. For example, a racemic mixture of a compound may be reacted with an optically active resolving agent to form a pair of diastereoisomeric compounds. The diastereomers may then be separated in order to recover the optically pure enantiomers. Dissociable complexes may also be used to resolve enantiomers (e.g., crystalline diastereoisomeric salts). Diastereomers typically have sufficiently distinct physical properties (e.g., melting points, boiling points, solubilities, reactivity, etc.) that they can be readily separated by taking advantage of these dissimilarities. For example, diastereomers can typically be separated by chromatography or by separation/resolution techniques based upon differences in solubility. A more detailed description of techniques that can be used to resolve stereoisomers of compounds from their racemic mixture can be found in Jean Jacques Andre Collet, Samuel H. Wilen, Enantiomers, Racemates and Resolutions, John Wiley & Sons, Inc. (1981).

Compounds according to the present invention can also be prepared as a pharmaceutically acceptable acid addition salt by reacting the free base form of the compound with a pharmaceutically acceptable inorganic or organic acid. Alternatively, a pharmaceutically acceptable base addition salt of a compound can be prepared by reacting the free acid form of the compound with a pharmaceutically acceptable inorganic or organic base. Inorganic and organic acids and bases suitable for the preparation of the pharmaceutically acceptable salts of compounds are set forth in the definitions section of this Application. Alternatively, the salt forms of the compounds can be prepared using salts of the starting materials or intermediates.

The free acid or free base forms of the compounds can be prepared from the corresponding base addition salt or acid addition salt form. For example, a compound in an acid addition salt form can be converted to the corresponding free base by treating with a suitable base (e.g., ammonium hydroxide solution, sodium hydroxide, and the like). A compound in a base addition salt form can be converted to the corresponding free acid by treating with a suitable acid (e.g., hydrochloric acid, etc).

The *N*-oxides of compounds according to the present invention can be prepared by methods known to those of ordinary skill in the art. For example, *N*-oxides can be prepared by treating an unoxidized form of the compound with an oxidizing agent (e.g., trifluoroperacetic acid, permaleic acid, perbenzoic acid, peracetic acid, *meta*-chloroperoxybenzoic acid, or the like) in a suitable inert organic solvent (e.g., a halogenated hydrocarbon such as dichloromethane) at approximately 0 °C. Alternatively, the *N*-oxides of the compounds can be prepared from the *N*-oxide of an appropriate starting material.

Compounds in an unoxidized form can be prepared from *N*-oxides of compounds by treating with a reducing agent (e.g., sulfur, sulfur dioxide, triphenyl phosphine, lithium borohydride, sodium borohydride, phosphorus trichloride, tribromide, or the like) in an suitable inert organic solvent (e.g., acetonitrile, ethanol, aqueous dioxane, or the like) at 0 to 80 °C.

Protected derivatives of the compounds can be made by methods known to those of ordinary skill in the art. A detailed description of the techniques applicable to the creation of protecting groups and their removal can be found in T.W. Greene, Protecting Groups in Organic Synthesis, 3rd edition, John Wiley & Sons, Inc. 1999.

Compounds according to the present invention may be conveniently prepared, or formed during the process of the invention, as solvates (e.g. hydrates). Hydrates of compounds of the present invention may be conveniently prepared by recrystallization from an aqueous/organic solvent mixture, using organic solvents such as dioxin, tetrahydrofuran or methanol.

Compounds according to the present invention can also be prepared as their individual stereoisomers by reacting a racemic mixture of the compound with an optically active resolving agent to form a pair of diastereoisomeric compounds, separating the diastereomers and recovering the optically pure enantiomer. While resolution of enantiomers can be carried out using covalent diastereomeric derivatives of compounds, dissociable complexes are preferred (e.g., crystalline diastereoisomeric salts). Diastereomers have distinct physical properties (e.g., melting points, boiling points, solubilities, reactivity, etc.) and can be readily separated by taking advantage of these dissimilarities. The diastereomers can be separated by chromatography or, preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques Andre Collet, Samuel H. Wilen, Enantiomers, Racemates and Resolutions, John Wiley & Sons, Inc. (1981).

As used herein the symbols and conventions used in these processes, schemes and examples are consistent with those used in the contemporary scientific literature, for example, the Journal of the American Chemical Society or the Journal of Biological Chemistry. Standard single-letter or thee-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| i.v. (intravenous); | Hz (Hertz); |
| MHz (megahertz); | mol (moles); |
| mmol (millimoles); | RT (ambient temperature); |
| min (minutes); h (hours); | |
| mp (melting point); | TLC (thin layer chromatography); |
| Tr (retention time); | RP (reverse phase); |
| MeOH (methanol); | i-PrOH (isopropanol); |
| TEA (triethylamine); | TFA (trifluoroacetic acid); |
| TFAA (trifluoroacetic anhydride); | THF (tetrahydrofuran); |
| DMSO (dimethylsulfoxide); | EtOAc (ethyl acetate); |
| DME (1,2-dimethoxyethane); | DCM (dichloromethane); |
| DCE (dichloroethane); | DMF (N,N-dimethylformamide); |
| DMPU (N,N'-dimethylpropyleneurea); | CDI (1,1-carbonyldiimidazole); |
| IBCF (isobutyl chloroformate); | HOAc (acetic acid); |
| HOSu (N-hydroxysuccinimide); | HOBT (1-hydroxybenzotriazole); |
| Et₂O (diethyl ether); | EDCI (ethylcarbodiimide hydrochloride); |
| BOC (tert-butyloxycarbonyl);FMOC | (9-fluorenylmethoxycarbonyl); |
| DCC (dicyclohexylcarbodiimide); | CBZ (benzyloxycarbonyl); |
| Ac (acetyl); | atm (atmosphere); |
| TMSE (2-(trimethylsilyl)ethyl); | TMS (trimethylsilyl); |
| TIPS (triisopropylsilyl); | TBS (t-butyldimethylsilyl); |
| DMAP (4-dimethylaminopyridine); | Me (methyl); |
| OMe (methoxy); | Et (ethyl); |
| Et (ethyl); | tBu (tert-butyl); |
| HPLC (high pressure liquid | chromatography); |
| BOP (bis(2-oxo-3-oxazolidinyl)phosphinic chloride); | |
| TBAF (tetra-n-butylammonium fluoride); | |
| mCPBA (meta-chloroperbenzoic acid. | |

All references to ether or Et₂O are to diethyl ether; brine refers to a saturated aqueous solution of NaCl. Unless otherwise indicated, all temperatures are expressed in °C (degrees Centigrade). All reactions conducted under an inert atmosphere at RT unless otherwise noted.

¹H NMR spectra were recorded on a Bruker Avance 400. Chemical shifts are expressed in parts per million (ppm). Coupling constants are in units of Hertz (Hz). Splitting patterns describe apparent multiplicities and are designated as s (singlet), d (doublet), t (triplet), q (quartet), m (multiplet), br (broad).

Low-resolution mass spectra (MS) and compound purity data were acquired on a Waters ZQ LC/MS single quadrupole system equipped with electrospray ionization (ESI) source, UV detector (220 and 254 nm), and evaporative light scattering detector (ELSD). Thin-layer chromatography was performed on 0.25 mm E. Merck silica gel plates (60F-254), visualized with UV light, 5% ethanolic phosphomolybdic acid, Ninhydrin or p-anisaldehyde solution. Flash column chromatography was performed on silica gel (230-400 mesh, Merck).

The starting materials and reagents used in preparing these compounds are either available from commercial suppliers such as the Aldrich Chemical Company (Milwaukee, WI), Bachem (Torrance, CA), Sigma (St. Louis, MO), or may be prepared by methods well known to a person of ordinary skill in the art, following procedures described in such standard references as Fieser and Fieser's Reagents for Organic Synthesis, vols. 1-17, John Wiley and Sons, New York, NY, 1991; Rodd's Chemistry of Carbon Compounds, vols. 1-5 and supps., Elsevier Science Publishers, 1989; Organic Reactions, vols. 1-40, John Wiley and Sons, New York, NY, 1991; March J.: Advanced Organic Chemistry, 4th ed., John Wiley and Sons, New York, NY; and Larock: Comprehensive Organic Transformations, VCH Publishers, New York, 1989.

Descriptions of the syntheses of particular compounds according to the present invention are set forth herein.

### 3. Examples of Kinase Inhibitors

The present invention is further exemplified, but not limited by, the following examples that describe the synthesis of particular compounds according to the invention.

### Compound I (reference): N-(3-bromophenyl)-3-nitropyridin-2-amine

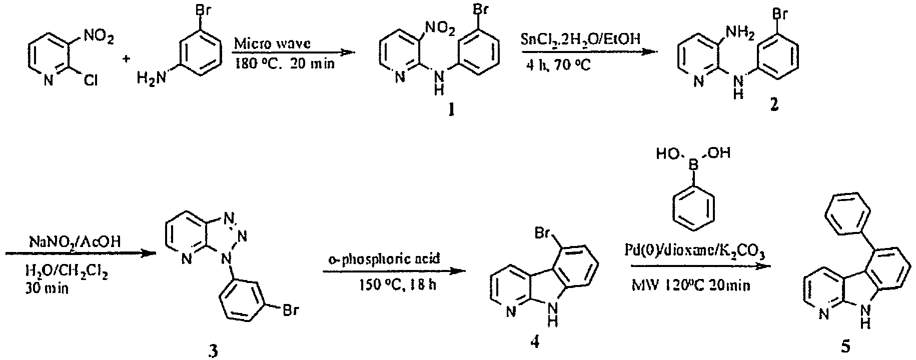

2-Chloro-3-nitropyridine (2-0 g, 12.6 mmol, 1 eq) was reacted with 5-bromoaniline (4.12 ml, 37.8 mmol, 3 eq) for 20 minutes at 180 °C in a microwave reactor. The product was isolated by column chromatography to provide the title compound as a red solid (4.9 g). [M+H] calc'd for C₁₁H₈BrN₃O₂, 293; found 293.

### Compound 2 (reference): N2-(3-bromophenyl)pyridine-2,3-diamine

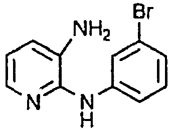

Compound 1 (4.9 g, 16.6 mmol) was dissolved in ethanol (20 ml). Tin (II) Chloride dihydrate (7.5 g, 33.3 mmol) was added and the solution stirred at 70 °C for 4 hours to provide the title compound. The product was confirmed by LC-MS. Addition of excess triethylamine caused a solid to form. The solid was filtered and the solution evaporated to leave an off white solid. The solid as recrystallized from ethanol to provide the title compound (3.8 g, 86%). [M+H] calc'd for C₁₁H₁₀BrN₃, 265; found 265.

### Compound 3 (reference): 3-(3-bromophenyl)-3H-[1,2,3]triazolo[4,5-b]pyridine

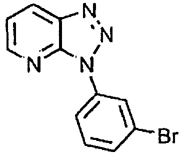

Compound 2 (3.8 g, 14.4 mmol) was dissolved in a mixture of acetic acid (4 mL), water (4 mL) and methylene chloride (4 mL). The mixture was cooled to 0 °C, then sodium nitrate (1.29 g, 18.7 mmol) was slowly added. Upon completion of the addition of sodium nitrate, the mixture was brought to room temperature and stirred for 20 minutes. The intended product was confirmed by LC-MS. The reaction mixture was diluted with methylene chloride (30 mL) and washed with water (3x30 mL). The organic layer was dried over magnesium sulfate and then evaporated to provide the title compound (2.9 g, 73%). [M+H] calc'd for C₁₁H₇BrN₄, 274; found, 274.

### Compound 4 (reference): 5-bromo-9H-pyrido[2,3-b]indole

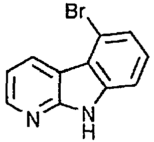

Compound 3 (2.8 g, 10.2 mmol) was dissolved in ortho-phosphoric acid (40 mL). The mixture was heated to 150°C for 18 hours, and the intended product confirmed by LC-MS. The mixture was cooled to 0 °C and neutralized with aqueous NaOH. The crude product was extracted with methylene chloride and purified by Preparative HPLC to provide Compound 4 (180 mg, 9%). ¹H NMR (400 MHz, CD₃OD) δ 9.16 (d, *J*=7.8 Hz I H) 8.48 (s, 1 H) 7.62 (d, *J*=7.8 Hz 1 H) 7-52 (d, *J*=6.8 Hz 1 H) 7.44 (m, 2 H). [M+H] calc'd for C₁₇H₁₂N2, 245; found 245.

### (Compounds 5 to 20 deleted)

### Compound 21 (reference): N-(4-(9H-pyrido[2,3-b]indol-5-ylthio)phenyl)acetamide

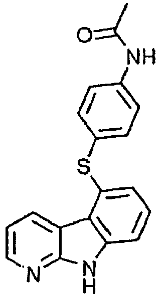

The title compound was synthesized by mixing Compound 4 (25 mg, 0.10 mmol, 4-mercapto-N-methylbenzamide (21 µl, 0.20 mmol), CS₂CO₃ (33 mg, 0.10 mmol) and [1,1'-Bis(diphenylphosphino)-ferrocene]dichloropalladium(II) (7 mg, 0.01 mmol) in DMF and heating at 170 °C for 20 minutes in a microwave reactor. The product was purified by HPLC (Yield= 42%). ¹H NMR (400 MHz, CD₃OD) δ 8.97 (d, *J*=7.84 1 H) 8.41 (d, *J*=5.56 Hz 1 H) 7.55 (m, 3 H) 7.50 (t, 1 H) 7.40 (q, 1 H) 7.36 (d, *J*=8.84 Hz 2 H) 7.12 (d, *J*=7.36 Hz 1 H) 2.11 (s, 3 H). [M+H] calc'd for C₁₉H₁₅N₃OS 334; found, 334.

### (Compounds 22 to 82 deleted)

### Compound 83 (reference): 3-(6-chloro-3-methyl-2-nitro-4-(trifluoromethyl)phenyl)-2-fluoro-5-methylpyridine

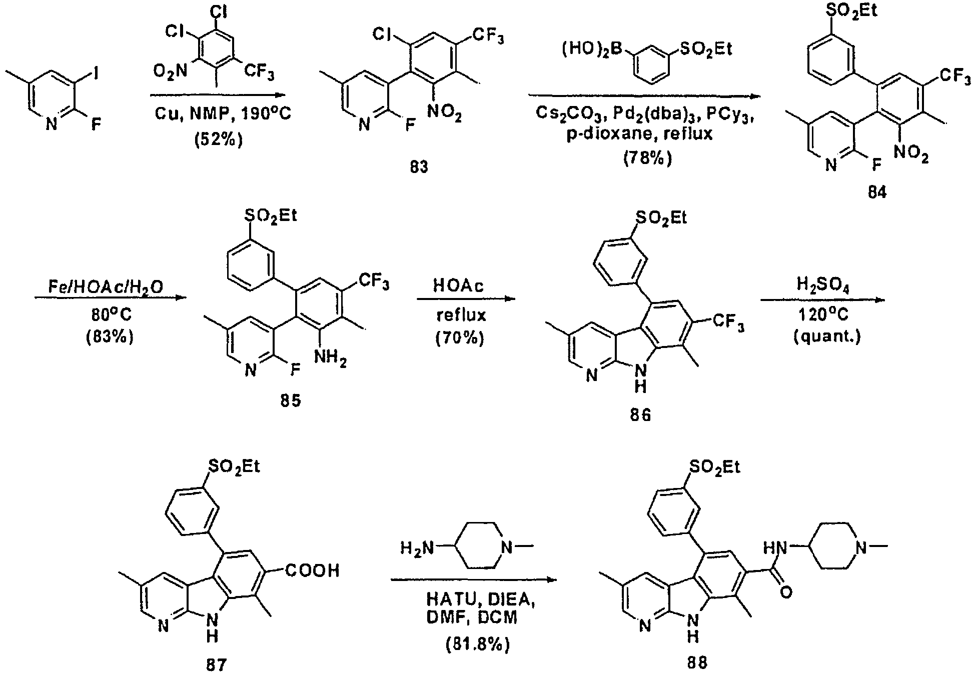

2-Fluoro-3-iodo-5-picoline (15.0 g, 63 mmol) was added drop wise during 2h as a solution in NMP (20 mL) to a stirred suspension of 3,4-dichloro-2-nitro-6-(trifluoromethyl)-toluene (52.1 g, 190 mmol) and copper (12.1 g, 190 mmol) in NMP (115 mL) at 190 °C. After completion of the reaction (2.5h), the mixture was cooled to room temperature, filtered, rinsed with NMP (3x5 mL) followed by EtOAc (1x100 mL). The filtrate was diluted with EtOAc (400 mL) affording a turbid solution. The organic layer was partitioned with sat. NaHCO₃ (150 mL) affording a suspension/emulsion. H₂O (50 mL) and MeOH (50 mL) were added to aid solubility. The aqueous layer was washed with EtOAc (5x150 mL). The organic layers were combined, dried (MgSO₄), and concentrated *in vacuo.* The crude product was purified by silica gel chromatography (98:2 Toluene:EtOAc) to provide the title compound as a tan solid (11.4 g, 52%).. ¹H NMR (400 MHz, DMSO-*d*₆): δ 8.34 (s, 1H), 8.26 (s, 1H), 7.86-7.89 (m, 1H), 2.4 (s, 3H), 2.34 (s, 3H). MS (ES) [m+H] calc'd for C₁₄H₉ClF₄N₂O₂, 349; found 349.2.

### Compound 84 (reference): 3-(3'-(ethylsulfonyl)-4-methyl-3-nitro-5-(trifluoromethyl)biphenyl-2-yl)-2-fluoro-5-methylpyridine

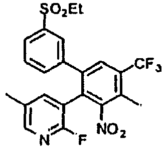

A mixture of Compound 83 (6.0 g, 17.2 mmol), 3-ethylsulfbnylphenylboronic acid (4.79 g, 22.4 mmol), bis(dibenzylidineacetone)Pd(0) (1.48 g, 2.6 mmol), tricyclohexylphosphine (1.45 g, 5.2 mmol), Cs₂CO₃ (14.0 g, 43 mmol), and dioxane (60 mL) was heated at reflux for 4.5 hr. After completion the reaction was cooled to room temperature, filtered, rinsed with dioxane, and concentrated *in vacuo.* The resulting oil was reconstituted in EtOAc (75 mL) washed with H₂O (1x30 mL) and brine (1x30 mL), dried (MSO₄), and concentrated *in vacuo.* The crude product was purified by silica gel chromatography (4:1 hexanes/EtOAc) to provide the title compound as a tan solid (6.5 g, 78%). ¹H NMR (400 MHz, DMSO-*d*₆): δ 8.15 (s, 1 H), 8.04 (s, 1 H), 7.90-7.93 (m, 1 H), 7.80-7.82 (m, 1H), 7.60-7.70 (m, 3H), 3.1-3.2 (m, 2H), 2.49 (s, 3H), 2.25 (s, 3H), 0.85 (t, 3H). MS (ES) [m+H] calc'd for C₂₂H₁₈F₄N₂O₄S, 483; found 483.3.

### Compound 85 (reference): 3'-(ethylsulfonyl)-2-(2-fluoro-5-methylpyridin-3-yl)-4-methyl-5-(trifluoromethyl)biphenyl-3-amine

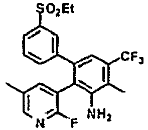

A mixture of Compound 84 (6.4 g, 13.3 mmol), iron (3.7 g, 66.3 mmol), HOAc, (32 mL), and H₂O (11 mL) was heated at 80 °C for 2 h. After completion the reaction was concentrated *in vacuo.* The residue was reconstituted in dichloromethane (100 mL), filtered, and rinsed with dichloromethane (3x30 mL). The organic phase was washed with sat. NaHCO₃ (1x100 mL) and brine (1x50 mL), dried (MSO₄), filtered, and concentrated *in vacuo.* The crude product was purified by silica gel chromatography (1:1 hexanes/EtOAc) to provide the title compound as a tan solid (5.0 g, 83%). ¹H NMR (400 MHz, DMSO-*d*₆): δ 7.93 (s, 1H), 7.67-7.7.71 (m, 2H), 7.53 (t, 1H), 7.46-7.48 (m, 1H), 7.42 (s, 1H), 6.93 (s, 1H), 5.09 (s, 2H), 3.11 (q, 2H), 2.27 (s, 3H), 2.21 (s, 3H), 0.85 (t, 3H). MS (ES) [m+H] calc'd for C₂₂H₂₀F₄N₂O₂S, 453; found 453.3.

### Compound 86 (reference): 5-(3-(ethylsulfonyl)phenyl)-3,8-dimethyl-7-(trifluoromethyl)-9H-pyrido[2,3-b]indole acetate

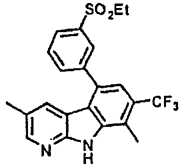

Compound 85 (4.9 g, 10.8 mmol) was dissolved in HOAc (35 mL) and heated at reflux for 3 h. The reaction mixture was cooled to room temperature affording a crystalline product. The resulting suspension was filtered, rinsed with HOAc (3x5 mL) followed by H₂O (3 x 10 mL) and the solids dried *in vacuo* to provide the title compound as a white solid (3.73 g, 70%). NMR analysis confirmed that the product was isolated as the mono-acetate salt. ¹H NMR (400 MHz, DMSO-*d*₆): δ 12.35 (s, 1H), 12.0 (s, 1H), 8.39 (s, 1H), 8.15 (s, 1H), 8.04-8.09 (m, 2H), 7.90 (t, 1H), 7.51 (s, 1H), 7.42 (s, 1H), 3.43 (q, 2H), 2.76 (s, 3H), 2.28 (s, 3H), 1.91 (s, 3H), 1.18 (t, 3H). MS (ES) [m+H] calc'd for C₂₂H₁₉F₃N₂O₂S, 433; found 433.3.

### Compound 87 (reference): 5-(3-(ethylsulfonyl)phenyl)-3,8-dimethyl-9H-pyrido[2,3-b]indole-7-carboxylic acid

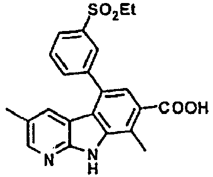

Compound 86 (3.6 g, 7.3 mmol) was dissolved in concentrated H₂SO₄ (30 mL) and heated at 120 °C for 30 min. The reaction was cooled to room temperature and poured over ice affording a white precipitate. The resulting suspension was filtered, rinsed with H₂O (3x30 mL) followed by IPA (3x10 mL) and dried *in vacuo* to provide the title compound as a white solid (3.2 g, quant.). ¹H NMR (400 MHz, DMSO-*d₆*): δ 12.20 (s, 1H), 8.36 (s, 1H), 8.12 (s, 1H), 8.02-8.07 (m, 2H), 7.89 (t, 1H), 7.61 (s, 1H), 7.54 (s, 1H), 3.43 (q, 2H), 2.85 (s, 3H), 2.28 (s, .3H), 1.18 (t, 3H). MS (ES) [m+H] calc'd for C₂₂H₂₀N₂O₄S, 409; found 409.3.

### Compound 88: 5-(3-(ethylsulfonyl)phenyl)-3,8-dimethyl-N-(1-methylpiperidin-4-yl)-9H-pyrido[2,3-b]indole-7-carboxamide

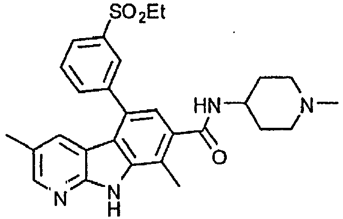

A mixture of Compound 87 (11.3 g, 27.6mmol), 1-methylpiperidin-4-amine (9.47 g, 82.9 mmol), HATU (1.3.66 g, 35.9 mmol), DIEA (17.88 g, 138 mmol), DMF (250 mL), and DCM (250 mL) was stirred at room temperature for 30 minutes. The resulting suspension was filtered, rinsed with DMF (10 mL x 4) and concentrated *in vacuo.* The residue was dissolved in DMSO (77 mL), filtered, and the filtrate was purified by preparative HPLC (ACN/H₂O with TFA). Following HPLC purification, the pure fractions were combined, basified with sodium bicarbonate and concentrated *in vacuo* to half volume. The resulting suspension was filtered, rinsed with H₂O (200 mL x 5) and dried *in vacuo* to provide Compound 88 as a white solid (11.41 g, 81.8%).

The hydrochloride salt of Compound 88 was prepared as follows. To a stirred suspension of Compound 88 (8.7 g) in ACN (175 mL) and H₂O (175 mL) was added 1N HCl (18.1 mL, 1.05 eq) affording a yellow solution. After 15 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide hydrochloride as a yellow solid (9.02 g, 96.7%). The above process provided 5-(3-(ethylsulfonyl)phenyl)-3,8-dimethyl-N-(1-methylpiperidin-4-yl)-9H-pyrido[2,3-b]indole-7-carboxamide hydrochloride as an amorphous material ("Amorphorus Form"), which may be characterized as having one or more of the following physical characteristics (it being noted that a composition need not necessarily exhibit all of these characteristics in order to indicate the presence of Amorphous Form):
(a) may be formed by lyophilizing a solution of Compound 88 in ACN, water, and HCl;
(b) has an XRPD spectrum characterized by a diffuse halo with no discernable peaks; and/or
(c) shows 7.6 wt% Cl⁻ present using ion chromatography.

The crystalline hydrochloride salt of Compound 88 was prepared as follows. To a stirred suspension of Compound 88 (0.55 g) in IPA (2.5 mL) and H₂O (2.5 mL) was added 12.1N HCl (1.05-1.10 eq) affording a yellow solution. After stirring for 45 minutes, crystallization occurred and additional IPA (15 mL) was added at room temperature. The resulting suspension was allowed to stir overnight. The solids were isolated by filtration and dried *in vacuo* at 60°C to provide 5-(3-(ethylsulfonyl)phenyl)-3,8-dimethyl-N-(1-methylpiperidin-4-yl)-9H-pyrido[2,3-b]indole-7-carboxamide hydrochloride as a tan to gold colored solid (0.51g, 87%).

The dihydrochloride salt of Compound 88 was prepared as follows. To a stirred suspension of Compound 88 (101 mg) in ACN (2.5 mL) and H₂O (2.5 mL) was added 12.1N HCl (0.42 mL, 2.1 eq) affording a yellow solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide dihydrochloride as a yellow solid (0.108 g).

The benzenesulfate salt of Compound 88 was prepared as follows. To a stirred suspension of Compound 88 (98 mg) in ACN (2.5 mL) and H₂O (2.5 mL) was added benzenesulfonic acid (32 mg, 1.05 eq) to give a slightly cloudy solution that was warmed to assist solubility. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide benzenesulfonate (118 mg).

The methanesulfonate salt of Compound 88 was prepared as follows. To a stirred suspension of Compound 88 (101 mg) in ACN (2.5 mL) and H₂O (2.5 mL) was added methanesulfonic acid (0.014 mL, 1.05 eq) affording a clear solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide methane sulfonate (116 mg).

The succinate salt of Compound 88 was prepared as follows. To a stirred suspension of Compound 88 (100 mg) in ACN (2.5 mL) and H₂O (2.5 mL) was added succinic acid (25 mg, 1.05 eq) to give a clear solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide succinate (119 mg).

The tartrate salt of Compound 88 was prepared as follows. To a stirred suspension of Compound 88 (108 mg) in ACN (2.5 mL) and H₂O (2.5 mL) was added L-tartaric acid (34 mg, 1.05 eq) affording a clear solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide tartrate (137 mg).

The citrate salt of Compound 88 was as follows. To a stirred suspension of Compound 88 (104 mg) in ACN (2.5 mL) and H₂O (2.5 mL) was added citric acid (42 mg, 1.05 eq) affording a clear solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide citrate (142 mg).

The fumarate salt of Compound 88 was prepared as follows. To a stirred suspension of Compound 88 (104 mg) in ACN (2.5 mL) and H₂O (2.5 mL) was added fumaric acid (25 mg, 1.05 eq) affording a very slightly cloudy solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide fumarate (123 mg).

The sulfate salt of Compound 88 was prepared as follows. To a stirred suspension of Compound 88 (107 mg) in ACN (2.5 mL) and H₂O (2.5 mL) was added sulfuric acid (0.012 mL, 1.05 eq) affording a yellow solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide sulfate (125 mg).

The phosphate salt of Compound 88 was prepared as follows. To a stirred suspension of Compound 88 (104 mg) in ACN (2.5 mL) and H₂O (2.5 mL) was added phosphoric acid (0.015 mL, 1.05 eq) affording a slightly cloudy solution that was warmed to assist solubility. After 5 minutes, the solution was frozen on dry ice/acetone and lyophilized to provide S-(3-(ethylsulfonyl)phenyl)-3,8-dimethyl-N-(1-methylpiperidin-4-yl)-9H-pyrido[2,3-b]indole-7-carboxamide phosphate (122 mg).

The benzoate salt of Compound 88 was prepared as follows. To a stirred suspension of Compound 88 (100 mg) in ACN (2.5 mL) and H₂O (2.5 mL) was added benzoic acid (25 mg, 1.05 eq) affording a clear solution containing a very small amount of suspended benzoic acid crystals. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide benzoate (118 mg).

The *bis*-trifluoroacetic acid salt of Compound 88 was prepared as follows. Following HPLC purification (ACN/H₂O with TFA) of crude Compound 88, the pure fractions were combined and lyophilized to provide 5-(3-(ethylsulfonyl)phenyl)-3,8-dimethyl-N-(1-methylpiperidin-4-yl)-9H-pyrido[2,3-b]indole-7-carboxamide bis(2,2,2-trifluoroacetate) as a yellow solid.

The tosylate salt of Compound 88 was prepared as follows. To a stirred suspension of Compound 88 (103 mg) in ACN (2.5 mL) and H₂O (2.5 mL) was added p-toluenesulfonic acid (39 mg, 1.05 eq) affording a clear solution. After 5 minutes, the solution was frozen on dry ice/acetone and lyophilized to provide 5-(3-(ethylsulfonyl)phenyl)-3,8-dimethyl-N-(1-methylpiperidin-4-yl)-9H-pyrido[2,3-b]indole-7-carboxamide tosylate (130 mg).

The hemi-fumarate salt of Compound 88 was prepared as follows. To a stirred solution of Compound 88 (360 mg) in MeOH at 58°C was added 0.5M fumaric acid (0.53 eq) in MeOH. After 15 minutes crystallization occurred and the resulting suspension was cooled to room temperature and allowed to stir for an additional 2 hours. The solids were isolated by filtration and dried *in vacuo* to provide 5-(3-(ethylsulfonyl)phenyl)-3,8-dimethyl-N-(1-methylpiperidin-4-yl)-9H-pyrido[2,3-b]indole-7-carboxamide hemifumarate as a white crystalline powder (219.24 mg, 50%).

### Compound 89: N-(2-(methylamino)ethyl)-5-(3-(ethylsulfonyl)phenyl)-3,8-dimethyl-9H-pyrido[2,3-b]indole-7-carboxamide

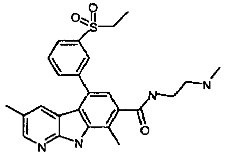

The title compound was synthesized using an analogous procedure to that described in the preparation of Compound 88. ¹H NMR (400 MHz, DMSO-*d*₆) δ ppm 1.17 (t, *J*=7.33 Hz, 3 H) 2.27 (s, 3 H) 2.63 (t, *J*=5.31 Hz, 3 H) 2.67 (s, 3 H) 3.12 (ddd, *J*=11.87, 6.32, 6.06 Hz, 2 H) 3.42 (q, *J*=7.41 Hz, 2 H) 3.56 (q, *J*=6.15 Hz, 2 H) 7.28 (s, 1 H) 7.51 (s, 1 H) 7.91 (t, *J*=7.83 Hz, 1 H) 8.04 (ddd, *J*=16.36, 7.77, 1.14 Hz, 2 H) 8.12 (s, 1 H) 8.33 (s, 1 H) 8.43 (br. s., 1 H) 8.57 (t, *J*=5.68 Hz, 1 H) 12.09 (s, 1 H) ESI-MS: m/z 465 (m + H)⁺

### Compound 90: N-(2-(methoxy)ethyl)-5-(3-(ethylsulfonyl)phenyl)-3,8-dimethyl-9H-pyrido[2,3-b]indole-7-carboxamide

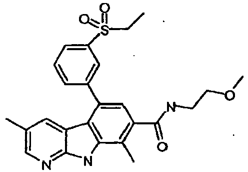

The title compound was synthesized using an analogous procedure to that described in the preparation of Compound 88. ¹H NMR (400 MHz, , DMSO-*d*₆) δ ppm 1.17 (t, *J*=7.33 Hz, 3 H) 2.27 (s, 3 H) 2.63 (s, 3 H) 3.29 (s, 3 H) 3.37 - 3.51 (m, 6 H) 7.12 (s, 1 H) 7.53 (d, *J*=1.26 Hz, 1 H) 7.89 (t, *J*=7.71 Hz, 1 H) 7.99 - 8.06 (m, 2 H) 8.12 (s, 1 H) 8.31 (s, 1 H) 8.43 (t, *J*=5.31 Hz, 1 H) 12.05 (s, 1 H) ESI-MS: m/z 466 (m + H)⁺

### Compound 91: N-(2-(dimethylamino)ethyl)-N-methyl-5-(3-(ethylsulfonyl)phenyl)-3,8-dimethyl-9H-pyrido[2,3-b]indole-7-carboxamide

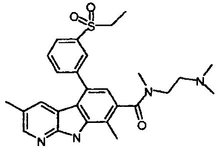

The title compound was synthesized using an analogous procedure to that described in the preparation of Compound 88. ¹H NMR (400 MHz, DMSO-*d₆*) δ ppm 1.17 (t, *J*=7.20 Hz, 3 H) 2.26 (s, 3 H) 2.64 (br. s., 3 H) 2.86 (s, 3 H) 2.91 (s, 3 H) 3.35 - 3.45 (m, 6 H) 7.06 (s, 1 H) 7.47 (s, 1 H) 7.89 (t, *J*=7.71 Hz, 1 H) 8.00 - 8.09 (m, 3 H) 8.31 (s, 1 H) 9.49 (br. s., 1 H) 12.11 (s, 1 H). ESI-MS: m/z 493 (m + H)⁺

### Compound 92: N,N-dimethyl-5-(3-(ethylsulfonyl)phenyl)-3,8-dimethyl-9H-pyrido[2,3-b]indole-7-methylcarboxamide

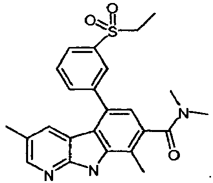

The title compound was synthesized using an analogous procedure to that described in the preparation of Compound 88. ¹H NMR (400 MHz, DMSO-*d*₆) δ ppm 1.17 (t, *J*=7.33 Hz, 3 H) 2.27 (s, 3 H) 2.46 (br. s., 3 H) 2.84 (s, 3 H) 3.05 (br. s., 3 H) 3.33 - 3.50 (m, 2 H) 6.97 (s, 1 H) 7.52 (d, *J*=1.52 Hz, 1 H) 7.87 (t, *J*=7.71 Hz, 1 H) 8.02 (t, *J*=7.33 Hz, 2 H) 8.10 (s, 1 H) 8.30 (d, *J*=1.52 Hz, 1 H) 12.08 (s, 1 H). ESI-MS: m/z 436 (m + H)⁺

### Compound 93: 5-(3-(ethylsulfonyl)phenyl)-3,8-dimethyl-9H-pyrido[2,3-b]indole-7-yl)(4-methylpiperazin-1-yl)methanone

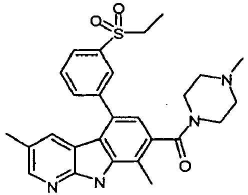

The title compound was synthesized using an analogous procedure to that described in the preparation of Compound 88. ¹H NMR (400 MHz, DMSO-*d*₆) δ ppm 1.18 (t, *J*=7.33 Hz, 3 H) 2.27 (s, 3 H) 2.57 (br. s., 3 H) 2.82 - 2.85 (br, 3 H) 3.10 -3.68 (m, 9 H) 4.77 (m, 1 H) 7.10 (br. d., 1 H) 7.51 (br. d, *J*=7.83 Hz, 1 H) 7.90 (t, *J*=7.33 Hz, 1 H) 7.99 - 8.13 (m, 3 H) 8.32 (s, 1 H) 9.96 (br. s., 1 H) 12.15 (s, 1 H). ESI-MS: m/z 491 (m + H)⁺

### Compound 94: 5-(3-(ethylsulfonyl)phenyl)-3,8-dimethyl-N-(2-piperazin-1-yl)ethyl)-9H-pyrido[2,3-b]indole-7-carboxamide

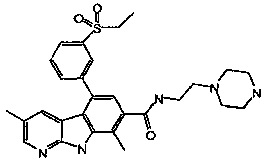

The title compound was synthesized using an analogous procedure to that described in the preparation of Compound 88. ¹H NMR (400 MHz, DMSO-*d*₆) δ ppm 1.17 (t, *J*=7.33 Hz, 3 H) 2.27 (s, 3 H) 2.66 (s, 3 H) 3.17 - 3.45 (m, 12 H) 3.59 (q, *J*=5.64 Hz, 2 H) 7.20 (s, 1 H) 7.52 (s, 1 H) 7.90 (t, *J*=7.71 Hz, 1 H) 8.04 (m, 2 H) 8.12 (s, 1 H) 8.33 (d, *J*=2.02 Hz, 1 H) 8.56 (t, *J*=5.68 Hz, 1 H) 8.99 (br. s., 1 H) 12.10 (s, 1 H). ESI-MS: m/z 520 (m + H)⁺

### Compound 95: 5-(3-(ethylsulfonyl)phenyl)-3,8-dimethyl-N-(3-(4-methylpiperazin-1-yl)propyl)-9H-pyrido[2,3-b]indole-7-carboxamide

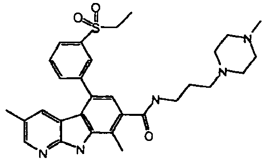

The title compound was synthesized using an analogous procedure to that described in the preparation of Compound 88. ¹H NMR (400 MHz, DMSO-*d*₆) δ ppm 1.17 (t, *J*=7.83 Hz, 3 H) 1.87 (br. s., 2 H) 2.27 (s, 3 H) 2.64 (s, 3 H) 2.82 (br. s., 3 H) 3.03 (br. s., 4 H) 3.31 - 3.49 (m, 8 H) 7.15 (s, 1 H) 7.52 (s, 1 H) 7.90 (t, *J*=7.71 Hz, 1 H) 8.00 - 8.07 (m, 2 H) 8.11 (s, 1 H) 8.32 (d, *J*=2.02 Hz, 1 H) 8.49 - 8.53 (m, 1 H) 12.08 (s, 1 H) ESI-MS: m/z 548 (m + H)⁺

### Compound 96: 5-(3-(ethylsulfonyl)phenyl)-3,8-dimethyl-9H-pyrido[2,3-b]indole-7-yl)(morpholino)methanone

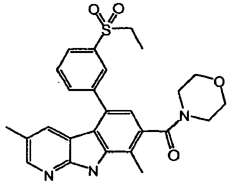

The title compound was synthesized using an analogous procedure to that described in the preparation of Compound 88. ¹H NMR (400 MHz, DMSO-*d*₆) δ ppm 1.38 (t,1 *J*=7.33 Hz, 3 H) 2.37 (s, 3 H) 2.71 (s, 3 H) 3.23 (q, *J*=7.33 Hz, 2 H) 3.39 (m, 2 H) 3.64 (d, *J*=13.14 Hz, 1 H) 3.64 (d, *J*=5.05 Hz, 1 H) 3.80 - 4.01 (m, 4 H) 7.04 (s, 1 H) 7.62 (s, 1 H) 7.78 (t, *J*=7.71 Hz, 1 H) 7.93 (dt, *J*=7.77, 1.42 Hz, 1 H) 8.07 (ddd, *J=*7.71, 1.64, 1.52 Hz, 1 H) 8.24 (t, *J*=1.64 Hz, 1 H) 8.34 (d, *J*=1.77 Hz, 1 H) 10.97 (br. s., 1 H) ESI-MS: m/z 478 (m + H)⁺

### Compound 97: azetidin-1-yl(5-(3-(ethylsulfonyl)phenyl)-3,8-dimethyl-9H-pyrido[2,3-b]indol-7-yl)methanone

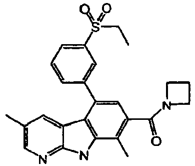

The title compound was synthesized using an analogous procedure to that described in the preparation of Compound 88. ¹H NMR (400 MHz, DMSO-*d*₆) δ ppm 1.37 (t, *J*=7.45 Hz, 3 H) 2.28 - 2.41 (m, 5 H) 2.75 (s, 3 H) 3.23 (q, *J*=7.58 Hz, 2 H) 4.03 (t, *J*=7.58 Hz, 2 H) 4.30 (t, *J*=7.96 Hz, 2 H) 7.12 (s, 1 H) 7.61 (s, 1 H) 7.77 (t, *J*=7.96 Hz, 1 H) 7.94 (ddd, *J*=7.89, 1.45, 1.26 Hz, 1 H) 8.06 (dd, *J*=8.21, 1.39 Hz, 1 H) 8.22 (t, *J*=1.52 Hz, 1 H) 8.33 (d, *J*=1.26 Hz, 1 H) 10.25 (br. s., 1 H). ESI-MS: m/z 448 (m + H)⁺

### Compound 98: (5-(3-(ethylsulfonyl)phenyl)-3,8-dimethyl-9H-pyrido[2,3-b]indol-7-yl)(thaiazolidin-3-yl)methanone

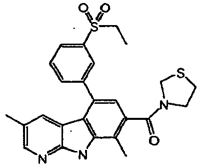

The title compound was synthesized using an analogous procedure to that described in the preparation of Compound 88. ¹H NMR (400 MHz, DMSO-*d*₆) δ ppm 1.18 (t, *J*=7.33 Hz, 3 H) 2.27 (s, 3 H) 2.56 (s, 3H) 2.99 (m, 1 H) 3.12 (m, 1 H) 3.43 - 3.51 m, 3H) 3.89 (m, 1 H) 4.32 (s, 1 H) 4.71 (s, 1 H) 7.06 (d, *J*=3.03 Hz, 1 H) 7.52 (br. s., 1 H) 7.88 (t, *J*=7.83 Hz, 1 H) 7.98 - 8.07 (m, 2 H) 8.12 (d, *J*=1.52 Hz, 1 H) 8.32 (d, *J*=1.77 Hz, 1 H) 12.11 (br. s., 1 H). ESI-MS: m/z 480 (m + H)⁺

### Compound 99: (R)-5-(3-(ethylsulfonyl)phenyl)-N-(2-hydroxypropyl)-3,8-dimethyl-9H-pyrido[2,3-b]indole-7-carboxamide

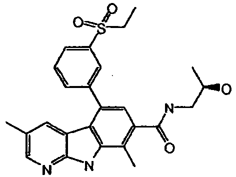

The title compound was synthesized using an analogous procedure to that described in the preparation of Compound 88. ¹H NMR (400 MHz, DMSO-*d*₆) δ ppm 1.13 (d, *J*=6.82 Hz, 3 H) 1.17 (t, *J*=7.33 Hz, 3 H) 2.26 (s, 3 H) 2.62 (s, 3 H) 3.30 -3.45 (m, 2 H) 3.41 (q, *J*=7.33 Hz, 2 H) 4.00 - 4.06 (m, 1 H) 7.12 (s, 1 H) 7.51 (d, *J*=1.26 Hz, 1 H) 7.89 (t, *J*=7.71 Hz, 1 H) 7.99 - 8.05 (m, 2 H) 8.11 (m, 2 H) 8.30 (s, 1 H) 12.04 (s, 1 H) ESI-MS: m/z 466 (m + H)⁺

### Compound 100: (S)-5-(3-(ethylsulfonyl)phenyl)-N-(2-hydmxypropyl)-3,8-dimethyl-9H-pyrido[2,3-b]indole-7-carboxamide

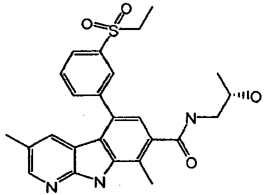

The title compound was synthesized using an analogous procedure to that described in the preparation of Compound 88. ¹H NMR (400 MHz, DMSO-*d*₆) δ ppm 1.10 (d, *J*=6.32 Hz, 3 H) 1.17 (t, *J*=7.33 Hz, 3 H) 2.27 (s, 3 H) 2.64 (s, 3 H) 3.22 (t, *J*=6.06 Hz, 2 H) 3.42 (q, *J*=7.33 Hz, 2 H) 3.72 - 3.88 (m, 1 H) 7.17 (s, 1 H) 7.55 (d, *J*=1.52 Hz, 1 H) 7.89 (t, *J*=7.71 Hz, 1 H) 8.03 (m, 2 H) 8.13 (t, *J*=1.64 Hz, 1 H) 8.31 (d, *J*=1.52 Hz, 1 H) 8.34 (t, *J*=5.94 Hz, 1 H) 12.09 (s, 1 H). ESI-MS: m/z 466 (m + H)⁺

### Compound 101: 5-(3-(ethylsulfonyl)phenyl)-N-(2-hydroxyethyl)-3,8-dimethyl- 9H-pyrido[2,3-b]indole-7-carboxamide

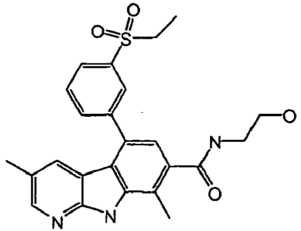

The title compound was synthesized using an analogous procedure to that described in the preparation of Compound 88. ¹H NMR (400 MHz, DMSO-*d*₆) δ ppm 1.17 (t, *J*=7.33 Hz, 3 H) 2.27 (s, 3 H) 2.63 (s, 3 H) 3.34 (q, *J*=6.23 Hz, 2 H) 3.42 (q, *J*=7.33 Hz, 2 H) 3.53 (t, *J*=6.19 Hz, 2 H) 7.17 (s, 1 H) 7.53 (d, *J*=1.77 Hz, 1H) 7.89 (t, *J*=7.71 Hz, 1 H) 8.03 (m, 2 H) 8.13 (t, *J*=1.64 Hz, 1 H) 8.34 (t, *J*=5.68 Hz, 1 H) 8.31 (d, *J*=1.52 Hz, 1 H) 12.05 (s, 1 H). ESI-MS: m/z 452 (m + H)⁺

### Compound 102: N-(2,3-dihydroxypropyl)-5-(3-(ethylsulfonyl)phenyl)-3,8-dimethyl- 9H-pyrido[2,3-b]indole-7-carboxamide

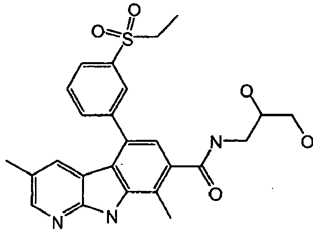

The title compound was synthesized using an analogous procedure to that described in the preparation of Compound 88. ¹H NMR (400 MHz, DMSO-*d*₆) δ ppm 1.17 (t, *J*=7.33 Hz, 3 H) 2.27 (s, 3 H) 2.64 (s, 3 H) 3.22 (ddd, *J*=13.14, 6.44, 6.19 Hz, 1 H) 3.35 - 3.45 (m, 5 H) 3.66 (qd, *J*=5.60, 5.43 Hz, 1 H) 7.18 (s, 1 H) 7.54 (s, 1 H) 7.89 (t, *J=7.83* Hz, 1 H) 8.03 (m, 2 H) 8.13 (s, 1 H) 8.29 - 8.35 (m, 2 H) 12.09 (s, 1 H). ESI-MS: m/z 482 (m + H)⁺

### Compound 103: 5-(3-(ethylsulfonyl)phenyl)-N-(2-hydroxy-2methylpropyl)-3,8-dimethyl-9H-pyrido[2,3-b]indole-7-carboxamide

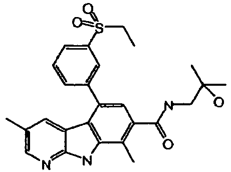

The title compound was synthesized using an analogous procedure to that described in the preparation of Compound 88. ¹H NMR (400 MHz, DMSO-*d*₆) δ ppm 1.17-1.14 (m, 9 H) 2.27 (s, 3 H) 2.64 (s, 3 H) 3.26 (d, *J*=6.32 Hz, 2 H) 3.41 (q, *J*=7.33 Hz, 2 H) 7.16 (s, 1 H) 7.54 (s, 1 H) 7.89 (t, *J*=7.71 Hz, 1 H) 8.04 (d, *J*=7.58 Hz, 2 H) 8.13 (t, *J*=1.64 Hz, 1 H) 8.25 (t, *J*=5.94 Hz, 1 H) 8.31 (d, *J*=1.26 Hz, 1 H) 12.07 (s, 1 H). ESI-MS: m/z 480 (m + H)⁺

### Compound 104: 5-(3-(ethylsulfonyl)phenyl)-N-(1-isopropylpiperidin-4-yl)-3,8-dimethyl-9H-pyrido[2,3-b]indole-7-carboxamide

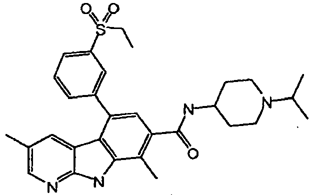

The title compound was synthesized using an analogous procedure to that described in the preparation of Compound 88. ¹H NMR (400 MHz, DMSO-*d*₆) δ ppm 1.13 - 1.27 (m, 9H) 1.72 - 1.84 (m, 2H) 2.05 - 2.17 (m, 2 H) 2.27 (s, 3 H) 2.63 (s, 3 H) 3.13 (m, 3 H) 3.42 (m, 4 H) 4.08 (m, 1H) 7.12 (s, 1 H) 7.53 (d, *J*=1.77 Hz, 1 H) 7.89 (t, *J*=7.71 Hz, 1 H) 8.04 (m, 2 H) 8.09 - 8.14 (s, 1 H) 8.32 (d, *J=*1.52 Hz, 1 H) 8.55 (d, *J*=7.58 Hz, 1 H) 9.11 (br. s., 1 H) 12.11 (s, 1 H). ESI-MS: m/z 533 (m + H)⁺

### Compound 105: N-(1-ethylpiperidin-4-yl)-5-(3-(ethylsulfonyl)phenyl)-3,8-dimethyl-9H-pyrido[2,3-b]indole-7-carboxamide

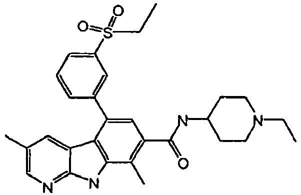

The title compound was synthesized using an analogous procedure to that described in the preparation of Compound 88. ¹H NMR (400 MHz, DMSO-*d*₆) δ ppm 1.14 - 1.25 (m, 6 H) 1.73 (m, 2 H) 2.00 -2.12 (m, 2 H) 2.27 (s, 3 H) 2.63 (s, 3 H) 3.00 - 3.17 (m, 4 H) 3.42 (q, *J*=7.33 Hz, 2 H) 3.53 (m, 2 H) 7.12 (s, 1 H) 7.52 (d, *J*=1.26 Hz, 1 H) 7.89 (t, *J*=7.71 Hz, 1 H) 7.99 - 8.07 (m, 2 H) 8.11 (s, 1 H) 8.31 (s, 1 H) 8.53 (d, *J*=7.58 Hz, 1 H) 9.17 (br. s., 1 H) 12.08 (s, 1 H). ESI-MS: m/z 519 (m + H)⁺

### Compound 106: 5-(3-(ethylsulfonyl)phenyl)-3,8-dimethyl-N-thiazol-2-yl)-9H-pyrido[2,3-b]indole-7-carboxamide

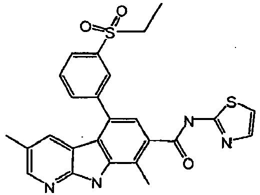

The title compound was synthesized using an analogous procedure to that described in the preparation of Compound 88. ¹H NMR (400 MHz, DMSO-*d*₆) δ ppm 1.18 (t, *J*=7.33 Hz, 3 H) 2.30 (s, 3 H) 2.72 (s, 3 H) 3.42 (q, *J*=7.41 Hz, 2 H) 7.30 (d, *J*=3.54 Hz, 1 H) 7.41 (s, 1 H) 7.56 (d, *J*=3.79 Hz, 1 H) 7.63 (s, 1 H) 7.90 (t, *J*=7.83 Hz, 1 H) 8.04 (d, *J*=7.59 Hz, 1 H) 8.12 (d, *J*=7.58 Hz, 1 H) 8.21 (s, 1 H) 8.36 (s, 1 H) 12.25 (s, 1 H) 12.66 (br. s., 1 H). ESI-MS: m/z 491 (m + H)⁺

### Compound 107: 5-(3-(ethylsulfonyl)phenyl)-3,8-dimethyl-N-(2-(2,2,2-trifluoroethoxy)ethyl-9H-pyrido[2,3-b]indole-7-carboxamide

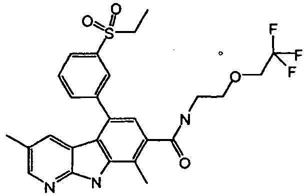

The title compound was synthesized using an analogous procedure to that described in the preparation of Compound 88. ¹H NMR (400 MHz, DMSO-*d*₆) δ ppm 1.17 (t, *J*=7.33 Hz, 3 H) 2.27 (s, 3 H) 2.63 (s, 3 H) 3.41 (q, *J*=7.33 Hz, 2 H) 3.47 (q, *J*=5.56 Hz, 2 H) 3.75 (t, *J*=5.68 Hz, 2 H) 4.11 (q, *J*=9.52 Hz, 2 H) 7.14 (s, 1 H) 7.56 (s, 1 H) 7.91 (t, *J*=7.83 Hz, 1 H) 7.99 - 8.06 (m, 2 H) 8.12 (s, 1 H) 8.32 (s, 1 H) 8.49 (t, *J*=5.68 Hz, 1 H) 12.10 (s, 1 H). ESI-MS: m/z 534 (m + H)⁺

### Compound 108: 5-(3-(ethylsulfonyl)phenyl)-3,8-dimethyl- N-(piperidin-3-yl)-9H-pyrido[2,3-b]indole-7-carboxamide

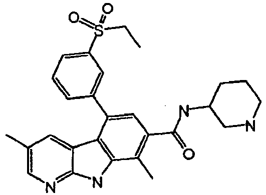

The title compound was synthesized using an analogous procedure to that described in the preparation of Compound 88. ¹H NMR (400 MHz, DMSO-*d*₆) δ ppm 1.17 (t, *J*=7.33 Hz, 3 H) 1.56 - 1.69 (m, 2 H) 1.96 - 1.88 (m, 2 H) 2.27 (s, 3 H) 2.63 (s, 3 H) 2.82 (m, 2 H) 3.22 (m, 1 H) 3.42 (m, 3 H) 4.16 (m, 1 H) 7.16 (s, 1 H) 7.51 (s, 1 H) 7.90 (t, *J*=7.71 Hz, 1 H) 7.99 - 8.08 (m, 2H) 8.11 (t, *J*=1.64 Hz, 1 H) 8.32 (d, *J*=2.02 Hz, 1 H) 8.51 (d, *J*=7.58 Hz, 1 H) 8.58 - 8.74 (m, 2 H) 12.09 (s, 1 H). ESI-MS: m/z 491 (m + H)⁺

### Compound 109: 5-(3-(ethylsulfonyl)phenyl)-3,8-dimethyl- N-(piperidin-4-yl)-9H-pyrid o[2,3-b]indole-7-carboxamide

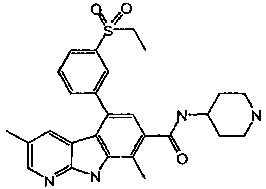

The title compound was synthesized using an analogous procedure to that described in the preparation of Compound 88. ¹H NMR (400 MHz, DMSO-*d*₆) δ ppm 1.18 (t, *J*=7.33 Hz, 3 H) 1.60 - 1.77 (m, 2 H) 2.05 - 2.03 (m, 2H) 2.27 (s, 3 H) 2.62 (s, 3 H) 3.04 (q, *J*=9.85 Hz, 2 H) 3.33 - 3.29 (m, 2H) 3.42 (q, *J*=7.49 Hz, 2 H) 4.10 (m, 1 H) 7.12 (s, 1 H) 7.51 (d, *J*=1.52 Hz, 1 H) 7.89 (t, *J*=7.71 Hz, 1 H) 7.99 - 8.08 (m, 2 H) 8.11 (s, 1 H) 8.32 (d, *J*=1.52 Hz, 1 H) 8.34-8.42 (m, 1H) 8.51 (d, *J*=7.58 Hz, 1 H) 8.60-8.66 (m,1H)12.08 (s, 1 H). ESI-MS: m/z 491 (m + H)⁺

### Compound 110: 5-(3-(ethylsulfonyl)phenyl)-3,8-dimethyl-N-(piperidin-3-yl)-9H-pyrido[2,3-b]indole-7-carboxamide

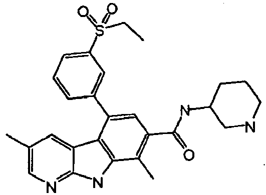

The title compound was synthesized using an analogous procedure to that described in the preparation of Compound 88. ¹H NMR (400 MHz, DMSO-*d*₆) δ ppm 1.17 (t, *J*=7.45 Hz, 3 H) 1.57 -1.69 (m, 2 H) 1.83 - 2.01 (m, 2 H) 2.27 (s, 3 H) 2.63 (s, 3 H) 2.74 - 2.90 (m, 2 H) 3.21 (m, 1 H) 3.42 ((m, 3 H)) 4.17 (m, 1 H) 7.16 (s, 1 H) 7.51 (s, 1 H) 7.90 (t, *J*=7.58 Hz, 1 H) 8.03 (m, 2 H) 8.11 (s, 1 H) 8.32 (s, 1 H) 8.50 (d, *J*=7.58 Hz, 1 H) 8.58 - 8.71 (m, 2 H) 12.09 (s, 1 H). ESI-MS: m/z 491 (m + H)⁺

### Compound 111: 5-(3-(ethylsulfonyl)phenyl)-N-(2-(2-hydroxyethoxy)ethyl-3,8-dimethyl-9H-pyrido[2,3-b]indole-7-carboxamide

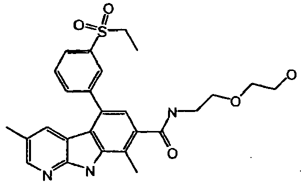

The title compound was synthesized using an analogous procedure to that described in the preparation of Compound 88. ¹H NMR (400 MHz, DMSO-*d*₆) δ ppm 1.17 (t, *J*=7.20 Hz, 3 H) 2.27 (s, 3 H) 2.63 (s, 3 H) 3.37 - 3.58 (m, 11 H) 7.14 (s, 1 H) 7.55 (s, 1 H) 7.88 (t, *J*=7.83 Hz, 1 H) 8.04 - 8.03 (m, 2H) 8.12 (s, 1 H) 8.31 (s, 1 H) 8.41 (t, *J*=5.68 Hz, 1 H) 12.08 (s, 1 H). ESI-MS: m/z 496 (m + H)⁺

### Compound 112: 5-(3-(cyclopropanecarboxamido)phenyl)-N-(2-(dimethylamino)ethyl)-3,8-dimethyl-9H-pyrido[2,3-b]indole-7-carboxamide

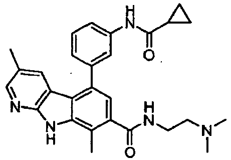

The title compound was synthesized using an analogous procedure to that described in the preparation of Compound 88. ¹H NMR (400 MHz, DMSO-*d*₆) δ ppm 0.75 - 0.84 (m, 4 H) 1.80 (t, *J*=4.93 Hz, 1 H) 2.27 (s, 3 H) 2.64 (s, 3 H) 2.87 (d, *J*=4.55 Hz, 6 H) 3.29 (q, *J*=5.56 Hz, 2 H) 3.61 (q, *J*=5.64 Hz, 2 H) 7.16 (s, 1 H) 7.27 (d, *J*=7.33 Hz, 1 H) 7.50 (t, *J*=7.71 Hz, 1 H) 7.63 (d, *J*=8.34 Hz, 1 H) 7.68 (s, 1 H) 7.99 (s, 1 H) 8.29 (s, 1 H) 8.58 (t, *J*=5.43 Hz, 1 H) 9.39 (br. s., 1 H) 10.38 (s, 1 H) 11.99 (s, 1 H); ESI-MS: *m*/*z* calc'd for C₂₈H₃₁N₅O₂ 469.25; found 470.4 (M+H)⁺

### Compound 113: N-(2-(dimethylamino)ethyl)-5-(3-(ethylsulfonyl)phenyl)-3,8-dimethyl-9H-pyrido[2,3-b]indole-7-carboxamide

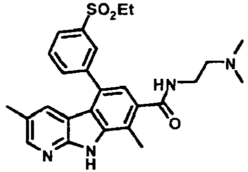

The title compound was synthesized using an analogous procedure to that described in the preparation of Compound 88. ¹H NMR (400 MHz, DMSO-*d₆*): δ 12.04 (s, 1H), 8.28-8.31 (m, 2H), 8.12 (s, 1H), 8.01-8.05 (m, 2H), 7.89 (t, 1H), 7.52 (s, 1H), 7.12 (s, 1H), 3.43 (q, 2H), 2.63 (s, 3H), 2.27 (s, 3H), 2.20 (s, 6H), 1.17 (t, 3H). MS (ES) [m+H] calc'd for C₂₆H₃₀N₄O₃S, 479; found 479.4.

### Compound 114: 5-(3-(ethylsulfonyl)phenyl)-3,8-dimethyl-N-((1-methylpiperidin-4-yl)methyl)-9H-pyrido[2,3-b]indole-7-carboxamide

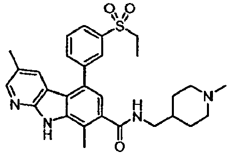

The title compound was synthesized using an analogous procedure to that described in the preparation of Compound 88. ¹H NMR (400 MHz, DMSO-*d*₆) δ ppm 1.18 (t, *J*=7.33 Hz, 3 H) 1.38 (d, *J*=10.86 Hz, 2 H) 1.78 (br. s., 1 H) 1.91 (d, *J*=13.39 Hz, 2 H) 2.27 (s, 3 H) 2.63 (s, 3 H) 2.75 (d, *J*=4.80 Hz, 3 H) 2.86 - 2.97 (m, 2 H) 3.20 (t, *J*=6.19 Hz, 2 H) 3.42 (q, *J*=7.33 Hz, 4 H) 7.14 (s, 1 H) 7.51 (d, *J*=1.26 Hz, 1 H) 7.89 (t, *J*=7.71 Hz, 1 H) 8.03 (dd, *J*=10.61, 8.59 Hz, 2 H) 8.12 (s, 1 H) 8.32 (d, *J*=1.52 Hz, 1 H) 8.50 (q, *J*=6.06 Hz, 1 H) 9.18 (br. s., 1 H) 12.08 (s, 1 H); ESI-MS: *m*/*z* calc'd for C₂₉H₃₄N₄O₃S 518.24; found 519.4 (M+H)⁺

### Compound 115: N-(3-(dimethylamino)propyl)-5-(3-(ethylsulfonyl)phenyl)-3,8-dimethyl-9H-pyrido[2,3-b]indole-7-carboxamide

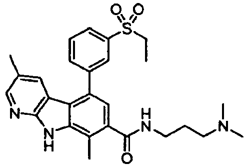

The title compound was synthesized using an analogous procedure to that described in the preparation of Compound 88. ¹H NMR (400 MHz, DMSO-*d*₆) δ ppm 1.18 (t, *J*=7.33 Hz, 3 H) 1.85 - 1.95 (m, 1 H) 1.91 (d, *J*=7.83 Hz, 1 H) 2.27 (s, 3 H) 2.65 (s, 3 H) 2.80 (d, *J*=4.80 Hz, 6 H) 3.13 (dt, *J*=10.36, 5.18 Hz, 2 H) 3.34 (q, *J*=6.32 Hz, 2 H) 3.42 (q, *J*=7.41 Hz, 2 H) 7.17 (s, 1 H) 7.52 (s, 1 H) 7.90 (t, *J*=7.71 Hz, 1 H) 8.04 (t, *J*=9.09 Hz, 2 H) 8.12 (s, 1 H) 8.32 (d, *J*=1.52 Hz, 1 H) 8.53 (t, *J*=5.81 Hz, 1 H) 9.35 (br. s., 1 H) 12.08 (s, 1 H); ESI-MS: *m*/*z* calc'd for C₂₇H₃₂N₄O₃S 492.22; found 493.4 (M+H)⁺

### Compound 116: 5-(3-(ethylsulfonyl)phenyl)-3,8-dimethyl-N-(2-(pyrrolidin-1-yl)ethyl)-9H-pyrido[2,3-b]indole-7-carboxamide

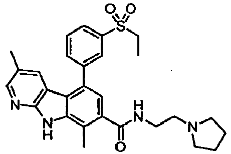

The title compound was synthesized using an analogous procedure to that described in the preparation of Compound 88. ¹H NMR (400 MHz, DMSO-*d*₆) δ ppm 1.17 (t, *J*=7.33 Hz, 3 H) 1.87 (dd, *J*=7.20, 4.93 Hz, 2 H) 2.03 (t, *J*=6.82 Hz, 2 H) 2.27 (s, 3 H) 3.08 (dd, *J*=10.48, 7.45 Hz, 2 H) 3.36 (q, *J*=5.89 Hz, 2 H) 3.42 (q, *J*=7.33 Hz, 2 H) 3.63 (td, *J*=12.88, 5.56 Hz, 4 H) 7.24 (s, 1 H) 7.52 (s, 1 H) 7.90 (t, *J*=7.71 Hz, 1 H) 8.04 (dd, *J*=14.27, 7.71 Hz, 2 H) 8.12 (s, 1 H) 8.33 (d, *J*=1.52 Hz, 1 H) 8.62 (t, *J*=5.68 Hz, 1 H) 9.53 (br. s., 1 H) 12.10 (s, 1 H); ESI-MS: *m*/*z* calc'd for C₂₈H₃₂N₄O₃S 504.22; found 505.4 (M+H)⁺

### Compound 117: (S)-5-(3-(ethylsulfonyl)phenyl)-3,8-dimethyl-N-(1-methylpiperidin-3-yl)-9H-pyrido[2,3-b]indole-7-carboxamide

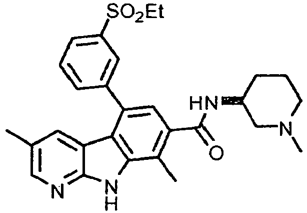

The title compound was synthesized using an analogous procedure to that described in the preparation of Compound 88. ¹H NMR (400 MHz, DMSO-*d*₆) δ ppm 1.18 (t, *J*=7.45 Hz, 3 H) 1.20-2.0 (m, 6 H) 2.27 (s, 3 H) 2.62 (s, 3 H) 2.70 - 4.4 (m, 8 H) 7.12 (s, 1 H) 7.52 (s, 1 H) 7.91 (d, *J*=7.58 Hz, 1 H) 8.01 (d, *J*=8.84 Hz, 1 H) 8.06 (d, *J*=8.84 Hz, 1 H) 8.11 (d, *J*=1.52 Hz, 1 H) 8.33 (s, 1 H) 8.62 (d, *J*=7.83 Hz, 1 H). [M+H] calc'd for C₂₈H₃₂N₂O₂S 505; found, 505.4.

### Compound 118: (R)-5-(3-(ethylsulfonyl)phenyl)-3,8-dimethyl-N-(1-methylpiperidin-3-yl)-9H-pyrido[2,3-b]indole-7-carboxamide

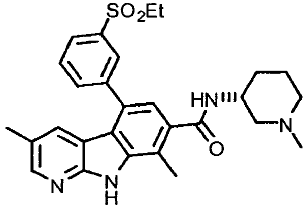

The title compound was synthesized using an analogous procedure to that described in the preparation of Compound 88. ¹H NMR (400 MHz, DMSO-*d*₆) δ ppm 1.18 (t, *J*=7.45 Hz, 3 H) 1.20 - 2.0 (m, 6 H) 2.27 (s, 3 H) 2.62 (s, 3 H) 2.70-4.40 (m, 8 H) 7.13 (s, 1 H) 7.53 (d, *J*=1.01 Hz, 1 H) 7.90 (t, *J*=7.83 Hz, 1 H) 8.04 (dd, *J*=17.43, 8.34 Hz, 2 H) 8.11 (d, *J*=1.52 Hz, 1 H) 8.33 (s, 1 H) 8.62 (d, *J*=7.83 Hz, 1 H) 12.11 (s, 1 H). [M+H] calc'd for C₂₈H₃₂N₂O₂S 505; found, 505.4.

### Compound 119 (reference): 5-chloro-3,8-dimethyl-N-(1-methylpiperidin-4-yl)-9H-pyrido[2,3-b]indole-7-carboxamide

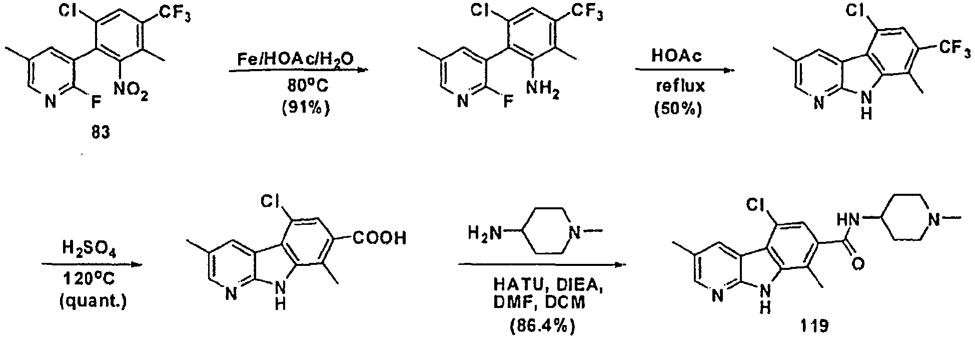

The title compound was synthesized from 5-chloro-3,8-dimethyl-9H-pyrido[2,3-b]indole-7-carboxylic acid and 1-methylpiperidin-4-amine using an analogous procedure to that described in the preparation of Compound 88. ¹H NMR (400 MHz, DMSO-*d*₆ with TFD) δ ppm 1.70-2.2 (m, 4 H) 2.53 (br. s., 3 H) 2.58 (s, 3 H) 2.74 - 2.82 (m, 3 H) 2.80-4.10 (m, 5 H) 7.29 (s, 1 H) 8.47 (s, 1 H) 8.70 (s, 1 H). [M+H] calc'd for C₂₀H₁₈N₂O₂S 371; found, 371.4.

### Compound 120: 5-(3-(cyclopropanecarboxamido)phenyl)-3,8-dimethyl-N-(1-methyl-piperidin-4-yl)-9H-pyrido[2,3-b]indole-7-carboxamide

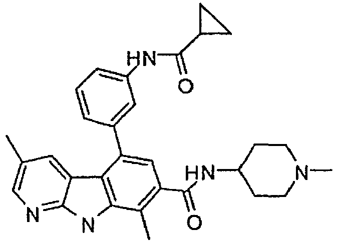

The title compound was synthesized from Compound 119 and 3-(cyclopropanecarboxamido) phenyl boronic acid using an analogous procedure to that described in the preparation of Compound 84. ¹H NMR (400 MHz, DMSO-*d*₆) δ ppm 0.77 - 0.82 (m, 4 H) 1.53 (qd, *J*=11.66, 3.41 Hz, 2 H) 1.79 -1.82 (m, 3 H) 1.95 (t, *J*=10.86 Hz, 2 H) 2.15 (s, 3 H) 2.27 (s, 3 H) 2.59 (s, 3 H) 2.74 (d, *J*=11.12 Hz, 2 H) 3.75 (m, I H) 6.98 (s, 1 H) 7.27 (d, *J*=7.58 Hz, 1 H) 7.49 (t, *J*=7.96 Hz, 1 H) 7.69 (d, *J*=2.02 Hz, 2 H) 7.91 (s, 1 H) 8.25 - 8.30 (m, 2 H) 10.37 (s, 1 H) 11.92 (br. s., 1 H); [M+H] calc'd for C₃₀H₃₄N₅O₂, 496.3.; found, 496.4.

### Compound 121 (reference): 5-chloro-N-(2-(dimethylamino)ethyl)-3,8-dimethyl-9H-pyrido[2,3-b]indole-7-carboxamide

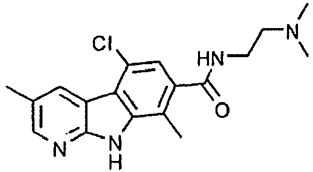

The title compound was synthesized from 5-chloro-3,8-dimethyl-9H-pyrido[2,3-b]indole-7-carboxylic acid and N,N-dimethylethane-1,2-diamine using an analogous procedure to that described in the preparation of Compound 88. ¹H NMR (400 MHz, DMSO-*d*₆) δ ppm 2.20 (s, 6 H) 2.42 (t, *J*=6.69 Hz, 2 H) 2.49 (br. s., 3 H) 2.55 (s, 3 H) 3.35 (d, *J*=6.57 Hz, 2 H) 7.18 (s, 1 H) 8.31 (t, *J*=5.56 Hz, 1 H) 8.40 (d, *J*=2.02 Hz, 1 H) 8.53 (s, 1 H) 12.14 (s, 1 H). [M+H] calc'd for C₁₈H₂₁ClN₄O 345; found, 345.4.

### Compound 122: 5-(3-(cyclopropylcarbamoyl)phenyl)-N-(2-(dimethylamino)ethyl)-3,8-dimethyl-9H-pyrido[2,3-b]indole-7-carboxamide

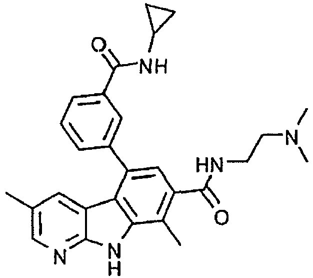

The title compound was synthesized from 5-chloro-N-(2-(dimethylamino)ethyl)-3,8-dimethyl-9H-pyrido[2,3-b]indole-7-carboxamide and 3-(cyclopropylcarbamoyl)phenyl boronic acid using an analogous procedure to that described in the preparation of Compound 84. ¹H NMR (400 MHz, DMSO-*d*₆) δ ppm 0.57 (dd, *J*=3.92, 2.40 Hz, 2 H) 0.71 (dd, *J*=6.95, 2.40 Hz, 2 H) 1.23 (s, 1 H) 2.26 (s, 3 H) 2.65 (s, 3 H) 2.87 (d, *J*=5.05 Hz, 6 H) 3.29 (q, *J*=5.98 Hz, 2 H) 3.61 (q, *J*=6.15 Hz, 2 H) 7.20 (s, 1 H) 7.50 (s, 1 H) 7.66 (t, *J*=7.83 Hz, 1 H) 7.77 (d, *J*=7.83 Hz, 1 H) 7.98 (d, *J*=7.83 Hz, 1 H) 8.08 (s, 1 H) 8.31 (d,*J*=1.77 Hz, 1 H) 8.57 - 8.61 (m, 1 H) 8.59 (d, *J*=4.55 Hz, 1 H) 12.05 (s, 1 H). [M+H] calc'd for C₂₈H₃₁N₅O₂ 470; found, 470.4.

### Compound 123 (reference): 4-(2-Fluoro-5-methyl-pyridin-3-yl)-3,5-dinitro-benzonitrile

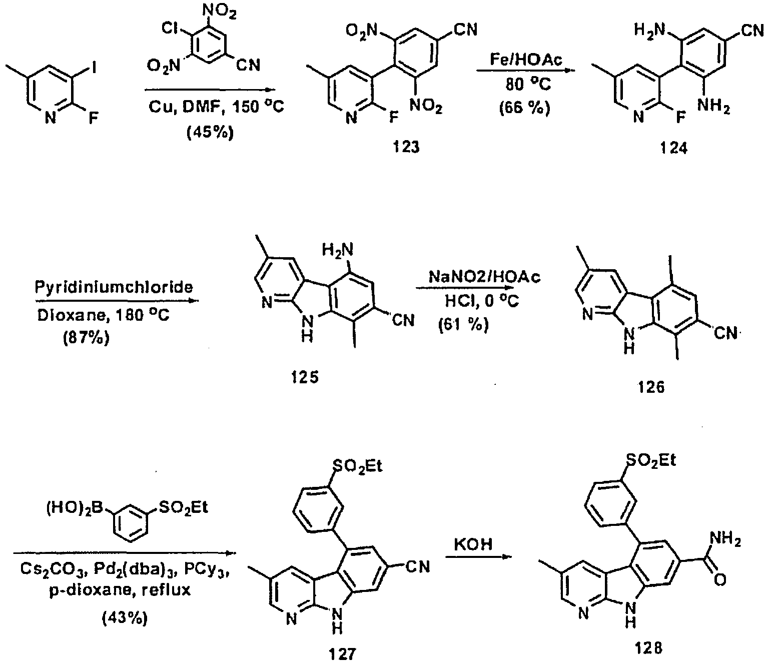

4-Chloro-3,5-dinitro-benzonitrile (200 mg, 0.88 mmol), 2-fluoro-3-iodo-5-picoline (208 mg, 0.88 mmol), and copper (45 µm powder, 168 mg, 2.6 mmol) were combined in DMF (2 mL) in a sealed tube purged with nitrogen. The reaction was heated at 150 °C for 30 min in the microwave. The reaction was diluted with acetone and the solids were removed by filtration. The solution was concentrated *in vacuo.* The crude product was purified by silica gel chromatography (80% CH₂Cl₂/hexanes) to provide the title compound as a faintly yellow solid (119 mg, 45%), which was slow to crystallize. ¹H NMR (400 MHz, CDCl₃): δ 8.50 (s, 2H), 8.16 (d, 1H, J = 1.2 Hz), 7.42 (dd, 1H, J = 8.8, 2.0 Hz), 2.38 (s, 3H). MS (ES) [m+H] calc'd for C₁₃H₇FN₄O₄, 303; found 303.

### Compound 124 (reference): 3,5-Diamino-4-(2-fluoro-5-methyl-pyridin-3-yl)-benzonitrile

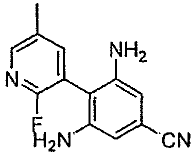

Compound 123 (119 mg, 0..39 mmol) was stirred in HOAc (3 mL) with H₂O (0.5 mL) and stirred at 76 °C. Iron powder (~325 mesh, 88 mg, 1.56 mmol) was added, and the reaction stirred for 4 h. The solution was concentrated *in vacuo,* diluted with EtOAc (30 mL), and made basic with sat. NaHCO₃. The material was then filtered through Celite, and the organics were separated, dried (MgSO₄), and concentrated *in vacuo* to provide the title compound as a brown oil (148 mg, 66%). MS (ES) [m+H] calc'd for C₁₃H₁₁FN₄, 243; found 243.

### Compound 125 (reference): 5-Amino-3-methyl-9H-pyrido[2,3-b]indole-7-carbonitrile

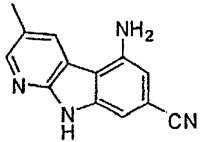

Compound 124 (148 mg, 0.61 mmol) was dissolved in dioxane (2 mL) and pyridinium chloride (80 mg), and the solution was heated at 180 °C in the microwave for 15 minutes. The solution was concentrated *in vacuo.* Purification by flash chromatography (20% acetone/CH₂Cl₂) to provide the title compound as an off-white solid (118 mg, 87%). MS (ES) [m+H] calc'd for C₁₃H₁₀N₄, 223; found 223.

### Compound 126 (reference): 5-Iodo-3-methyl-9H-pyrido[2,3-b]indole-7-carbonitrile

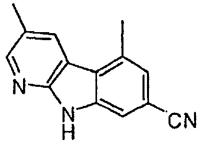

Compound 125 (118 mg, 0.53 mmol) was dissolved in HOAc (2 mL) and H₂O (1 mL), and the solution stirred at 0 °C. Concentrated HCl (120 µL) in H₂O (120 mL) was added, and the reaction stirred for 5 min. Sodium nitrite (54 mg, 0.78 mmol) in H₂O (120 µL) was added dropwise, and the red solution stirred for 10 min. A solution of iodine (10 mg) and potassium iodide (129 mg, 0.78 mmol) in H₂O (300 µL) was added dropwise, and the brown frothy solution stirred for 30 min at 0 °C and then 30 min while warming to r.t. The reaction was diluted with H₂O (5 mL) and extracted with CHCl₃. Organics were dried (MgSO₄ and concentrated *in vacuo.* Purification by silica gel chromatography provided the title compound as a faintly yellow solid (108 mg, 61%). MS (ES) [m+H] calc'd for C₁₃H₈IN₃, 334; found 334.

### Compound 127 (reference): 5-(3-Ethanesulfonyl-phenyl)-3-methyl-9H-pyrido[2,3-b]indole-7-carbonitrile

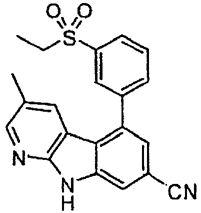

The title compound was prepared in 43% yield from Compound 126 according to the procedure outlined in the preparation of Compound 84. ¹H NMR (400 MHz, CD₃OD) δ 8.34 (s, 1H), 8.21 (s, 1H), 8.13 (d, 1H, J = 7.6 Hz), 8.00 (t, 1H, J = 7.6 Hz), 7.89-7.98 (m, 2H), 7.59 (s, 1H), 7.49 (s, 1H), 3.35 (q, 2H, J = 7.2 Hz), 2.33 (s, 3H), 1.29 (t, 3H, J = 7.2 Hz). MS (ES) [m+H] calc'd for C₂₁H₁₇N₃O₂S, 376; found 376.

### Compound 128 (preference): 5-(3-Ethanesulfonyl-phenyl)-3-methyl-9H-pyrido[2,3-b]indole-7-carboxylic acid amide

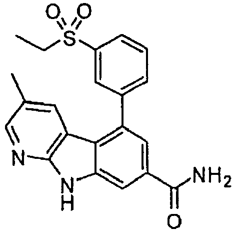

Compound 127 (30 mg, 0.08 mmol) was dissolved in dioxane (2 mL) and stirred at r.t. A solution of potassium hydroxide (25 mg, 0.44 mmol) in 30% H₂O₂ solution (1 mL) was added, and the reaction stirred for 18 h. The solution was neutralized with IN HCl and concentrated *in vacuo.* Purification by silica gel chromatography (5 to 8% MeOH/CH₂Cl₂ g provided the title compound as a white solid (14.8 mg, 47%). ¹H NMR (400 MHz, CD₃OD) δ 8.28 (s, 1H), 8.22 (s, 1H), 8.12 (s, 1H), 8.10 (d, 1H, J = 7.6 Hz), 8.02 (d, 1H, J = 7.6 Hz), 7.88 (t, 1H, J = 7.6 Hz), 7.68 (s, 1H), 7.62 (s, 1H), 3.34 (q, 2H, J = 7.2 Hz), 2.31 (s, 3H), 1.29 (t, 3H, J = 7.2 Hz). MS (ES) [m+H] calc'd for C₂₁H₁₉N₃O₃S, 394; found 394.

### Compound 129 (reference): 4-(2-Fluoro-5-methyl-pyridin-3-yl)-3,5-dinitro-benzoic acid methyl ester

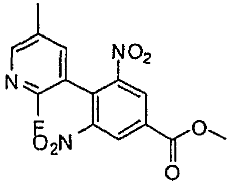

The title compound was prepared from 4-chloro-3,5-dinitro-benzoic acid methyl ester in 94% yield according to the procedure outline for the preparation of Compound 123. MS (ES) [m+H] calc'd for C₁₄H₁₀FN₃O₆, 336; found 336.

### Compound 130 (reference): 3,5-Diamino-4-(2-fluoro-5-methyl-pyridin-3-yl)-benzoic acid methyl ester

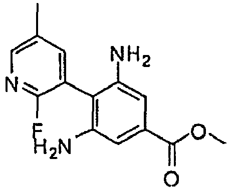

Compound 129 (2.02, 6.03 mmol) was stirred in MeOH (150 mL) with 10% Pd/C (200 mg) under a hydrogen atmosphere for 1.5 h. The reaction was filtered through Celite and concentrated to provide the title compound as a brown solid (1.64 g, 99%). MS (ES) [m+H] calc'd for C₁₄H₁₄FN₃O₂, 276; found 276.

### Compound 131 (reference): 5-Amino-3-methyl-9H-pyrido[2,3-b]indole-7-carboxylic acid methyl ester

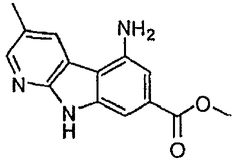

The title compound was prepared in 88% yield from example Compound 130 according to the procedure outlined for the preparation of Compound 125. MS (ES) [m+H] calc'd for C₁₄H₁₃N₃O₂, 256; found 256.

### Compound 132 (reference): 5-Iodo-3-methyl-9H-pyrido[2,3-b]indole-7-carboxylic acid methyl ester

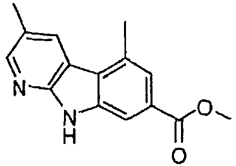

The title compound was prepared in 69% yield from Compound 131 according to the procedure outlined for the preparation of Compound 126. MS (ES) [m+H] calc'd for C₁₄H₁₁IN₂O₂, 367; found 367.

### Compound 133 (reference): 5-(3-Ethanesulfonyl-phenyl)-3-phenyl-9H-pyrido[2,3-b]indole-7-carboxylic acid methyl ester

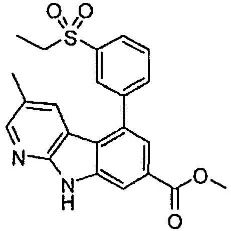

The title compound was prepared in 65% yield from Compound 132 according to the procedure outlined in the preparation of Compound 84. MS (ES) [m+H] calc'd for C₂₂H₂₀N₂O₄S, 409; found 409.

### (Compounds 134 to 139 deleted)

### Compound 140 (reference): 5-(3-Ethanesulfonyl-phenyl)-3-methyl-9H-pyrido[2,3-b]indole-7-carboxylic acid

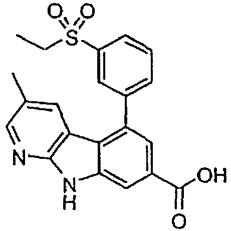

Compound 133 (260 mg, 0.64 mmol) was dissolved in 1N NaOH (1 mL) and MeOH (2 mL) at 60 °C for 2 h, The reaction was allowed to cool, and was acidified with 1 N HCl and extracted with CHCl₃. Organics were dried (MgSO₄) and concentrated to provide the title compound as a white solid (228 mg, 90%). ¹H NMR (400 MHz, CD₃OD) δ 8.34 (br s, 1H), 8.29 (s, 1H), 8.21 (s, 1H), 8.11 (d, 1H, J = 7.6 Hz), 8.01 (d, 1H, J = 7.6 Hz), 7.90 (t, 1H, J = 7.6 Hz), 7.87 (s, 1H), 7.79 (s, 1H), 3.31 (q, 2H, J = 7.2 MHz), 2.35 (s, 3H), 1.29 (t, 3H, J = 7.2 Hz). MS (ES) [m+H] calc'd for C₂₁H₁₈N₂O₄S, 395; found 395.

### Compound 141: [5-(3-Ethanesulfonyl-phenyl)-3-methyl-9H-pyrido[2,3-b]indol-7-yl]-(4-methyl-piperazin-1-yl)-methanone

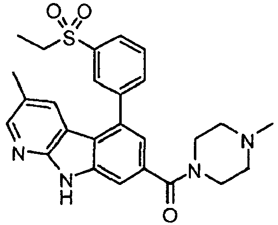

Compound 140 (40 mg, 0.1 mmol) and HOBT (17 mg, 0.11 mmol) were dissolved in CH₂Cl₂ (2 mL) at r.t. EDC (29 mg, 0.15 mmol) and 1-methylpiperazine (45 mL, 0.4 mmol) were added, and the reaction stirred for 3 h. Organics were washed with brine, dried (Na₂SO₄), and concentrated *in vacuo.* Purification by prep-HPLC provided the title compound as a pale yellow solid (32 mg, 67%). ¹H NMR (400 MHz, CD₃OD) δ 8.36 (s, 1H), 8.22 (s, 1H), 8.12 (d, 1H, J = 7.6 Hz), 8.03 (d, 1H, J = 7.6 Hz), 7.87-7.95 (m, 2H), 7.80 (s, 1H), 7.40 (s, 1H), 3.39-3.62 (m, 4H), 3.31 (q, 2H, J = 7.2 Hz), 3.16-3.30 (m, 4H), 2.95 (s, 3H), 2.38 (s, 3H), 1.29 (t, 3H, J = 7.2 Hz). MS (ES) [m+H] calc'd for C₂₆H₂₈N₄O₃S, 477; found 477.

### Compound 142: 5-(3-Ethanesulfonyl-phenyl)-3-methyl-9H-pyrido[2,3-b]indole-7-carboxylic acid (2-dimethylamino-ethyl)-amide

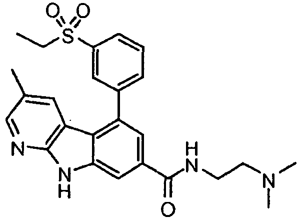

The title compound was prepared in 65% yield according to the procedure outlined for the preparation of Compound 141. ¹H NMR (400 MHz, CD₃OD δ 8.38 (br s, 1H), 8.23 (s, 1H), 8.20 (s, 1H), 8.12 (d, 1H, J = 7.6 Hz), 8.04 (d, 1H, J = 7.6 Hz), 7.91 (t, 1H, J = 7.6 Hz), 7.84 (s, 1H), 7.75 (s, 1H), 3.80-3.86 (m, 2H), 3.42 (t, 2H, J = 5.6 Hz), 3.34 (q, 2H, J = 7.2 Hz), 3.01 (s, 6H), 2.38 (s, 3H), 1.30 (t, 3H, J = 7.2 Hz). MS (ES) [m+H] calc'd for C₂₅H₂₈N₄O₃S, 465; found 465.

### Compound 143: 5-(3-Ethanesulfonyl-phenyl)-3-methyl-9H-pyrido[2,3-b]indole-7-carboxylic acid (3-dimethylamino-propyl)-amide

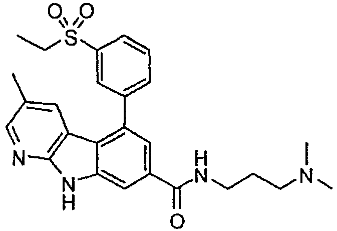

The title compound was prepared in 63% yield according to the procedure outlined for the preparation of Compound 141. ¹H NMR (400 MHz, CD₃OD) δ 8.39 (br s, 1H), 8,24 (s, 1H), 8.19 (s, 1H), 8.13 (d, 1H, J = 7.6 Hz), 8.04 (d, 1H, J = 7.6 Hz), 7.88-7.96 (m, 2H), 7.78 (s, 1H), 3.56 (t, 2H, J = 6.4 Hz), 3.20-3.35 (m, 4H), 2.93 (s, 6H), 2.39 (s, 3H), 2.02-2.11 (m, 2H), 1,30 (t, 3H, J = 7.2 Hz). MS (ES) [m+H] calc'd for C₂₆H₃₀N₄O₃S, 478; found 478.

### (Compounds 144 to 150 deleted)

### Compound 151 (reference): 5-(benzylthio)-3-methyl-9H-pyrido[2,3-b]indole-7-carboxylic acid

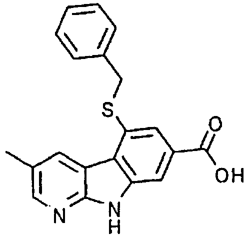

The title compound was synthesized using an analogous procedure to that described in the preparation of Compound 21. ¹H NMR (400 MHz, MeOD δ ppm 2.52 (s, 3 H) 4.39 (s, 2 H) 7.15 - 7.29 (m, 3 H) 7.34 (d, *J*=7.83 Hz, 2 H) 7.87 (s, 2 H) 7.92 (s, 1 H) 8.07 (s, I H) 8.30 (s, I H) 8.75 (br. s., 1 H) [M+H] calc'd for C₂₀H₁₆N₂O₂S, 349; found, 349.

### Compound 152: 5-(benzylthio)-N-(2-(dimethylamino)ethyl)-3-methyl-9H-pyrido[2,3-b]indole-7-carboxamide

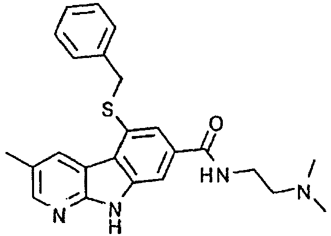

The title compound was synthesized from Compound 151 using an analogous procedure to that described in the preparation of Compound 141. ¹H NMR (400 MHz, MeOD) δ ppm 2.53 (s, 3 H) 3.02 (s, 6 H) 3.43 (t, *J*=5.81 Hz, 2 H) 3.82 (t, *J*=5.81 Hz, 2 H) 4.42 (s, 2 H) 7.16 - 7.26 (m, 3 H) 7.31 (d, *J*=7.83 MHz, 2 H) 7.82 (d, *J*=1.26 Hz, 1 H) 7.96 (s, 1 H) 8.30 (s, 1 H) 8.80 (s, 1 H) [M+H] calc'd for C₂₄H₂₆N₄OS, 419; found, 419.

### Compound 153: 5-(3-(N-ethylsulfamoyl)phenyl)-8-methoxy-3-methyl-N-(1-methylpiperidin-4-yl)-9H-pyrido[2,3-b]indole-7-carboxamide

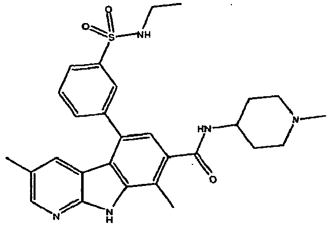

The title compound was synthesized from compound 119 and 3-(N-ethylsulfamoyl)phenylboronic acid using an analogous procedure to that described in the preparation of Compound 84. ¹H NMR (400 MHz, Methanol-d₄) δ 8.28 (s, 1 H) 8.13 (s, 1 H) 8.04 (m, 1 H) 7.88 (m, 1 H) 7.75 (m, 1 H) 7.81 (t, J= 7.84 Hz, 1 H) 7.24 (s, 1 H) 4.22 (m, 1 H) 3.62 (m, br, 2 H) 3.22 (m, 2 H) 3.01(q, J = 7.32 Hz, 2 H) 2.92 (s, 3 H) 2.72 (s, 3 H) 2.36 (m, 5 H) 1.93 (m, 2 H) 1.11 (t, J = 7.32 Hz, 3 H). [M+H] calc'd for C₂₈H₃₄N₅O₃S, 520; found, 520.

### Compound 154: 5-(3-(cyclopropylsulfonyl)phenyl)-3,8-dimethyl-N-(1-methylpiperidin-4-yl)-9H-pyrido[2,3-b]indole-7-carboxamide

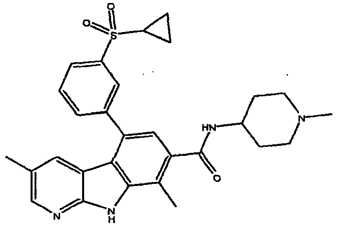

The title compound was synthesized from compound 119 and 3-(cyclopropylsulfonyl)phenylboronic acid using an analogous procedure to that described in the preparation of Compound 84. ¹H NMR (400 MHz, Methanol-d₄) δ 8.32 (s, 1 H) 8.19 (s, 1 H) 8.11 (m, 1 H) 7.99 (m, 1 H) 7.89 (m, 2 H) 7.3 (s, 1 H) 4.22 (m, 1 H) 3.74 (m, 1 H) 3.65 (m, 2 H) 3.22 (m, 2 H) 2.93 (s, 3 H) 2.72 (s, 3 H) 2.36 (m, 5 H) 1.93 (m, 2 H) 1.28 (m, 2 H) 1.14 (m, 2 H). [M+H] calc'd for C₂₉H₃₃N₄O₃S, 517; found, 517.

### (Compounds 155 to 227 deleted)

### Compound 228: 5-(3-(cyclopropylcarbamoyl)phenyl)-3,8-dimethyl-N-(1-methylpiperidin-4-yl)-9H-pyrido[2,3-b]indole-7-carboxamide

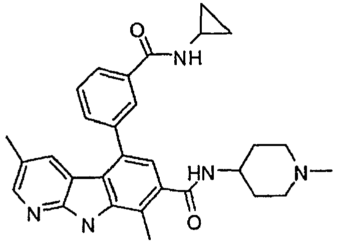

The title compound was synthesized from 5-chloro-3,8-dimethyl-N-(1-methylpiperidin-4-yl)-9H-pyrido[2,3-b] indole-7-carboxamide and .3-(cyclopropylcarbamoyl)phenylboronic acid using an analogous procedure to that described in the preparation of compound 88. ¹H NMR (400 MHz, DMSO-*d*₆) δ ppm 0.77 - 0.82 (m, 4 H) 1.53 (qd, *J*=11.66, 3.41 Hz, 2 H) 1.79 - 1.82 (m, 3 H) 1.95 (t, *J*=10.86 Hz, 2 H) 2.15 (s, 3 H) 2.27 (s, 3 H) 2.59 (s, 3 H) 2.74 (d, *J*=11.12 Hz, 2 H) 3.75 (m, 1 H) 6.98 (s, 1 H) 7.27 (d, *J*=7.58 Hz, 1 H) 7.49 (t, *J*=7.96 Hz, 1 H) 7.69 (d, *J*=2.02 Hz, 2 H) 7.91 (s, 1 H) 8.25 - 8.30 (m, 2 H) 10.37 (s, 1 H) 11.92 (br. s., 1 H); [M+H] calc'd for C₃₀H₃₃N₅O₂, 496.3; found, 496.4.

### (Compounds 229 to 261 deleted)

### Compound 262: N-(2-(diethylamino)ethyl)-5-(3-(ethylsulfonyl)phenyl)-3,8-dimethyl-9H-pyrido[2,3-b]indole-7-carboxamide

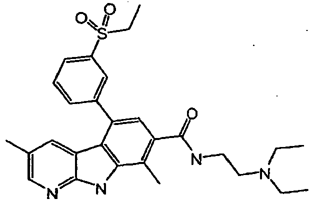

The title compound was synthesized using an analogous procedure to that described in the preparation of compound 88. ESI-MS: *m*/*z* calc'd for C₂₈H₃₄N₄O₃S 506.2; found 507.4 [M+H]⁺

### Compound 263: 5-(3-(ethylsulfonyl)phenyl)-3,8-dimethyl-N-(3-morpholinopropyl)-9H-pyrido[2,3-b]indole-7-carboxamide

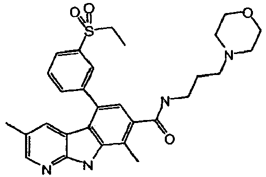

The title compound was synthesized using an analogous procedure to that described in the preparation of compound 88. ESI-MS: *m*/*z* calc'd for C₂₉H₃₄N₄O₄S 534.6; found 535.7 [M+H]⁺

### (Compounds 264 to 334 deleted)

### Compounds 335-353

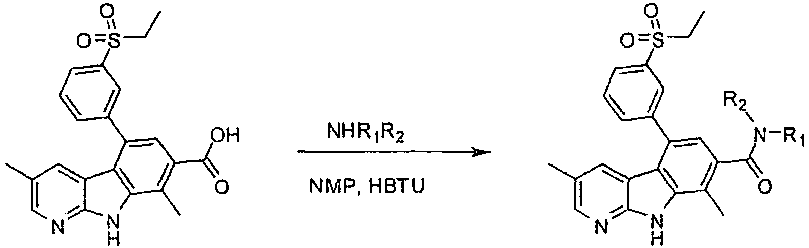

| **Compound** | **Name** | **Structure** |
|---|---|---|
| 335 | 5-(3-(ethylsulfonyl)phenyl)-N-((1r,4r)-4-hydroxycyclohexyl)-3,8-dimethyl-9H-pyrido[2,3-b]indole-7-carboxamide | 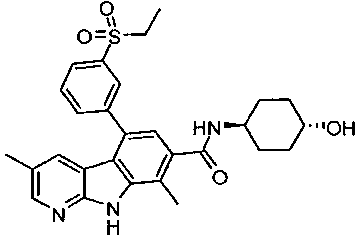 |
| 336 | 5-(3-(ethylsulfonyl)phenyl)-N-((1s,4s)-4-hydroxycyclohexyl)-3,8-dimethyl-9H-pyrido[2,3-b]indole-7-carboxamide | 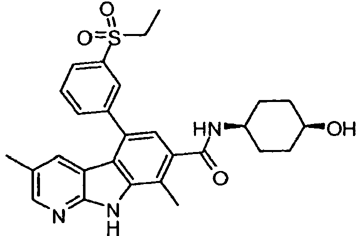 |
| 337 | 5-(3-(ethylsulfonyl)phenyl)-N-(4-(hydroxymethyl)cyclohexyl)-3,8-dimethyl-9H-pyrido[2,3-b]indole-7-carboxamide | 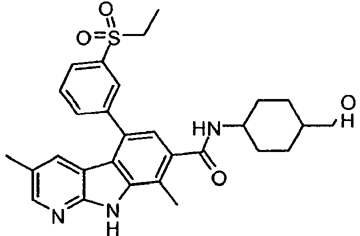 |
| 338 | 5-(3-(ethylsulfonyl)phenyl)-N-(4-(2-hydroxyethyl)cyclohexyl)-3,8-dimethyl-9H-pyrido[2,3-b]indole-7-carboxamide | 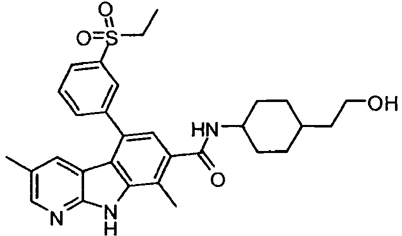 |
| 339 | (5-(3-(ethylsulfonyl)phenyl)-3,8-dimethyl-9H-pyrido[2,3-b]indol-7-yl)(4-(hydroxymethyl)piperidin-1-yl)methanone | 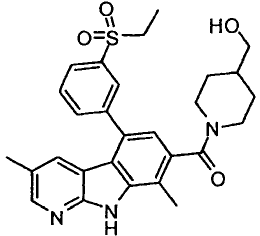 |
| 340 | (5-(3-(ethylsulfonyl)phenyl)-3,8-dimethyl-9H-pyrido[2,3-b]indol-7-yl)(4-(2-hydroxyethyl)piperidin-1-yl)methanone | 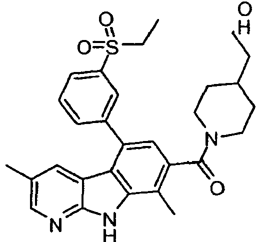 |
| 341 | (5-(3-(ethylsulfonyl)phenyl)-3,8-dimethyl-9H-pyrido[2,3-b]indol-7-yl)(4-hydroxypiperidin-1-yl)methanone | 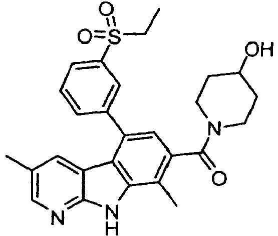 |
| 342 | (5-(3-(ethylsulfonyl)phenyl)-3,8-dimethyl-9H-pyrido[2,3-b]indol-7-yl)(2-(hydroxymethyl)pyrrolidin-1-yl)methanone | 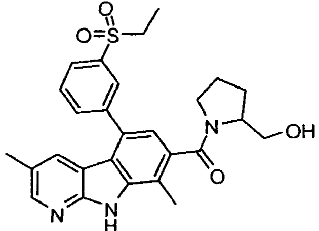 |
| 343 | (5-(3-(ethylsulfonyl)phenyl)-3,8-dimethyl-9H-pyrido[2,3-b]indol-7-yl)(2-(hydroxymethyl)morpholino)methanone | 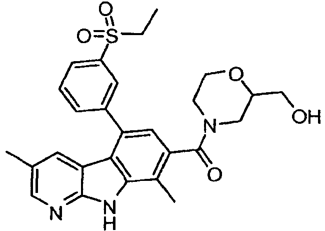 |
| 344 | 5-(3-(ethylsulfonyl)phenyl)-N-(3-hydroxypropyl)-3, 8-dimethyl-9H-pyrido[2,3-b]indole-7-carboxamide | 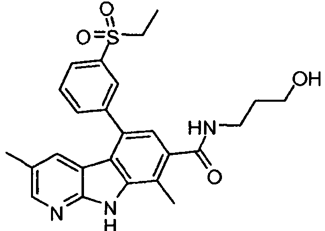 |
| 345 | (R)-5-(3-(ethylsulfonyl)phenyl)-N-(3-hydroxybutyl)-3,8-dimethyl-9H-pyrido[2,3-b]indole-7-carboxamide | 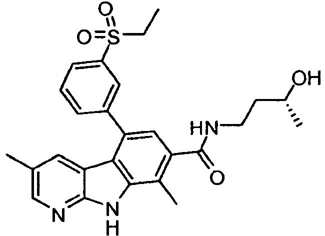 |
| 346 | (S)-5-(3-(ethylsulfonyl)phenyl)-N-(3-hydroxybutyl)-3,8-dimethyl-9H-pyrido[2,3-b]indole-7-carboxamide | 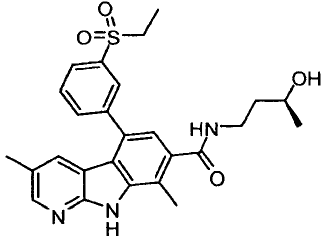 |
| 347 | 5-(3-(ethylsulfonyl)phenyl)-N-(3-hydroxy-3-methylbutyl)-3,8-dimethyl-9H-pyrido[2,3-b]indole-7-carboxamide | 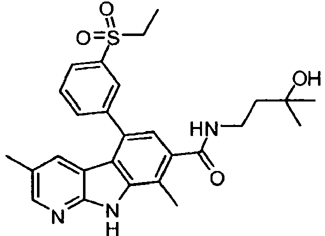 |
| 348 | (S)-N-(3-(dimethylamino)-2-hydroxypropyl)-5-(3-(ethylsulfonyl)phenyl)-3,8-dimethyl-9H-pyrido[2,3-b]indole-7-carboxamide | 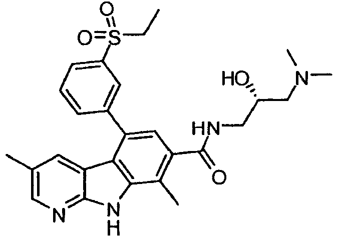 |
| 349 | (S)-N-(2-(dimethylamino)-3-hydroxypropyl)-5-(3-(ethylsulfonyl)phenyl)-3,8-dimethyl-9H-pyrido[2,3-b]indole-7-carboxamide | 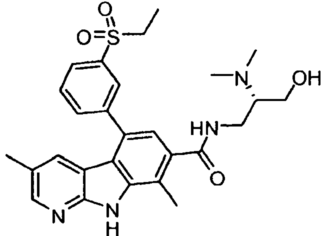 |
| 350 | N-(3-(ethyl(2-hydroxyethyl)amino)propyl)-5-(3-(ethylsulfonyl)phenyl)-3,8-dimethyl-9H-pyrido[2,3-b]indole-7-carboxamide | 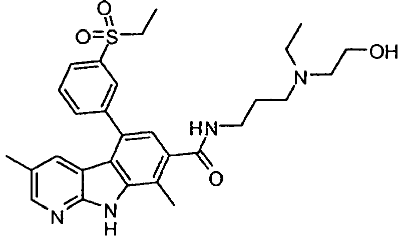 |
| 351 | 5-(3-(ethylsulfonyl)phenyl)-N-(3-((2-hydroxyethyl)(methyl)amino)propyl)-3,8-dimethyl-9H-pyrido[2,3-b]indole-7-carboxamide | 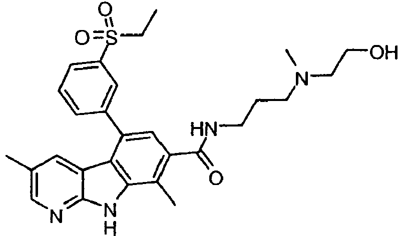 |
| 352 | N-(2-(ethyl(2-hydroxyethyl)amino)ethyl)-5-(3-(ethylsulfonyl)phenyl)-3,8-dimethyl-9H-pyrido[2,3-b]indole-7-carboxamide | 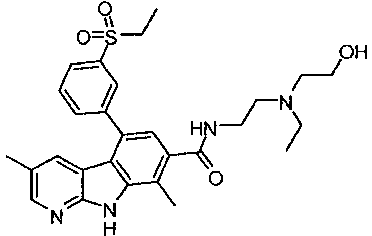 |
| 353 | 5-(3-(ethylsulfonyl)phenyl)-N-(2-((2-hydroxyethyl)(methyl)amino)ethyl)-3,8-dimethyl-9H-pyrido[2,3-b]indole-7-carboxamide | 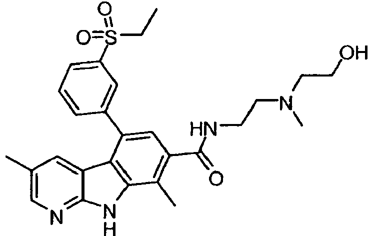 |

Compounds 335-353 can be prepared as follows. Crude 5-(3-(ethylsulfonyl)phenyl)-3,8-dimethyl-9H-pyrido[2,3-b]indole-7-carboxylic acid (Compound 87; *e.g*., 124.3 g, 0.244 mol) is heated with NMP to give a thin slurry (*e.g*., 1.0 L of NMP heated to 70 °C for 39 min). The heat is removed and the mixture cooled (*e.g*., to 16°C with the aid of a cold water bath). Appropriate amine (2.0 equiv) is added, followed by HBTU (92.6 g, 1.0 equiv) in portions over about 26 min. Over the next 2.5 h, additional HBTU (*e.g*., 4.7 and 4.65 g) can be added and the mixture stirred (*e.g*., overnight) until the reaction proceeds to completion (*e.g*., >99% conversion as indicated by HPLC analysis). The mixture is then be filtered (*e.g*., through a Celite impregnated pad, which is rinsed twice with NMP (27 and 39 g)). To the filtrate is added a solution of KOH (27.7 g, 1.7 equiv at 85% based on theoretical 5-(3-(ethylsulfonyl)phenyl)-3,8-dimethyl-9H-pyrido[2,3-b]indole-7-carboxylic acid) in water (250 mL) to give a solution with pH 11.7. After solids begin to form (*e.g*., about 15 min), water (500 mL) is added (*e.g*., over 30 min at 28-29 °C). After about 3 h at room temperature, the solids are removed by filtration, and the wet cake is rinsed with water (*e,g*., 1150 mL in several portions), and dried in a vacuum oven (45-55 °C) to give the desired product. HPLC analysis can be used to indicate purity (AUC at 215 nm).

Compounds according to the present invention can also be prepared as pharmaceutically acceptable salts. In addition, to the salts described above, salts of compounds of the present inventions can be formed using, for example, the following acids: benzoic acid, fumaric acid, HBr, HCl, hippuric acid, lactic acid, maleic acid, malic acid, MSA, phosphoric acid, p-TSA, succinic acid, sulfuric acid, tartaric acid, and the like. The salts of the above acids can be prepared by adding 0.5 to 2.0 equivalents of the appropriate acid in any of a variety of solvents (such as MeCN, EtOH, MeOH, DMA, THF, AcOH, and the like, or mixtures thereof) at a temperature of between about 10°C, and 75°C.

In addition to the foregoing, the above reaction schemes and variations thereof can be used to prepare the following:

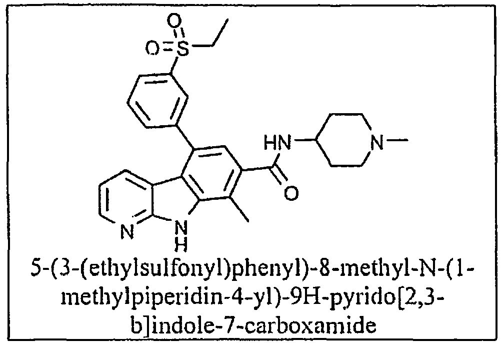

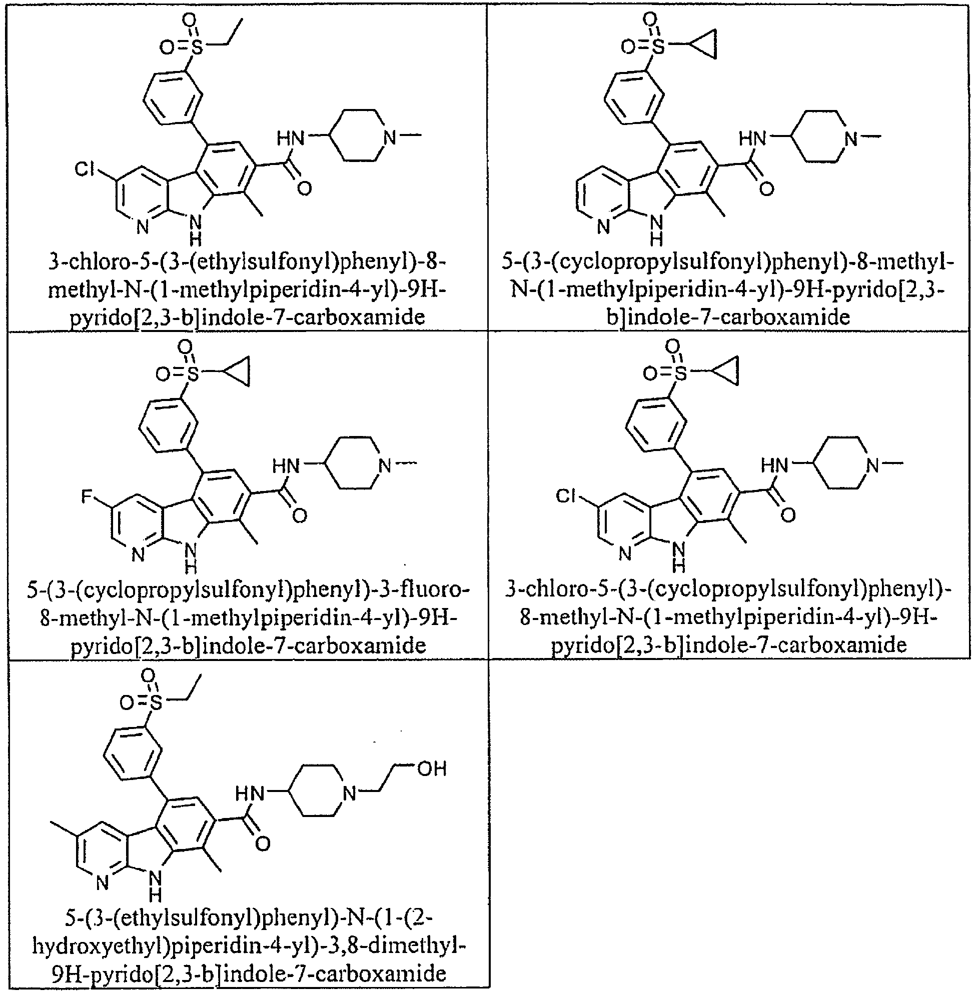

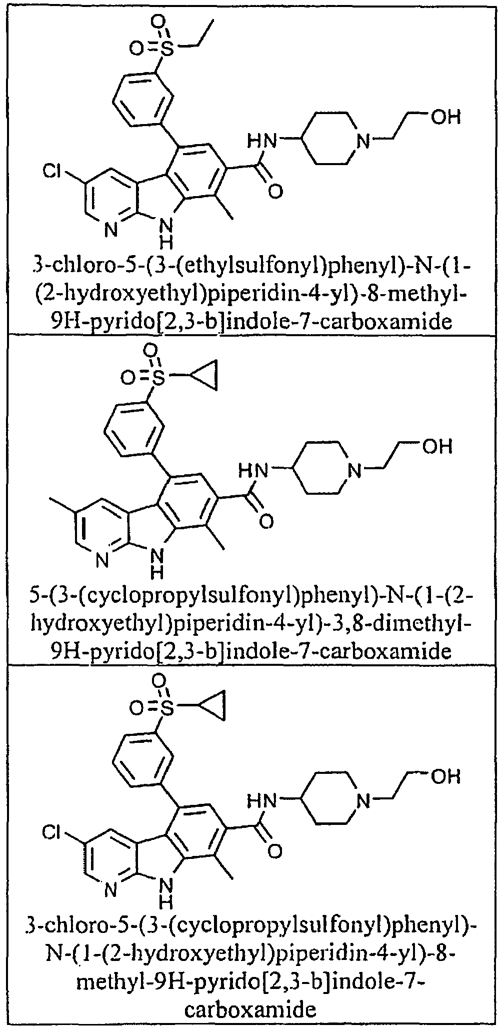

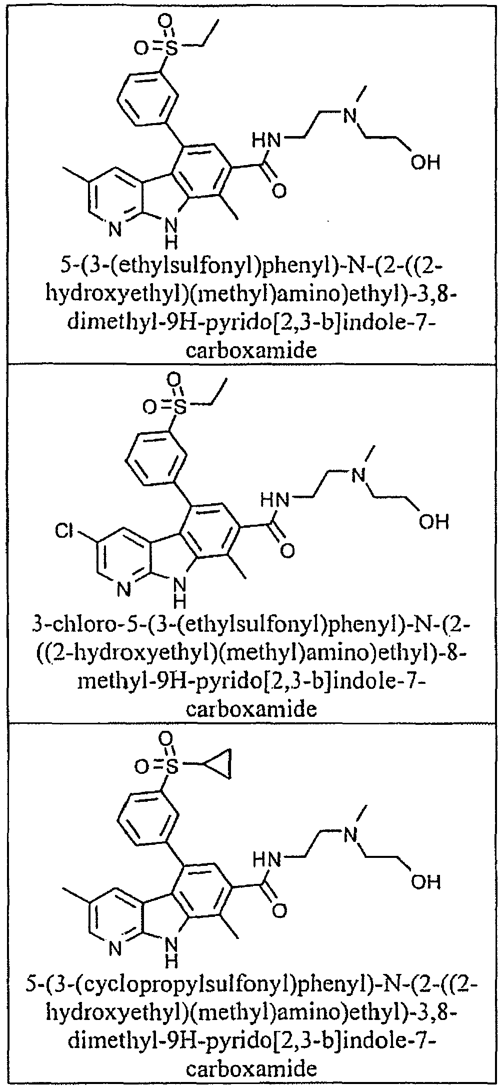

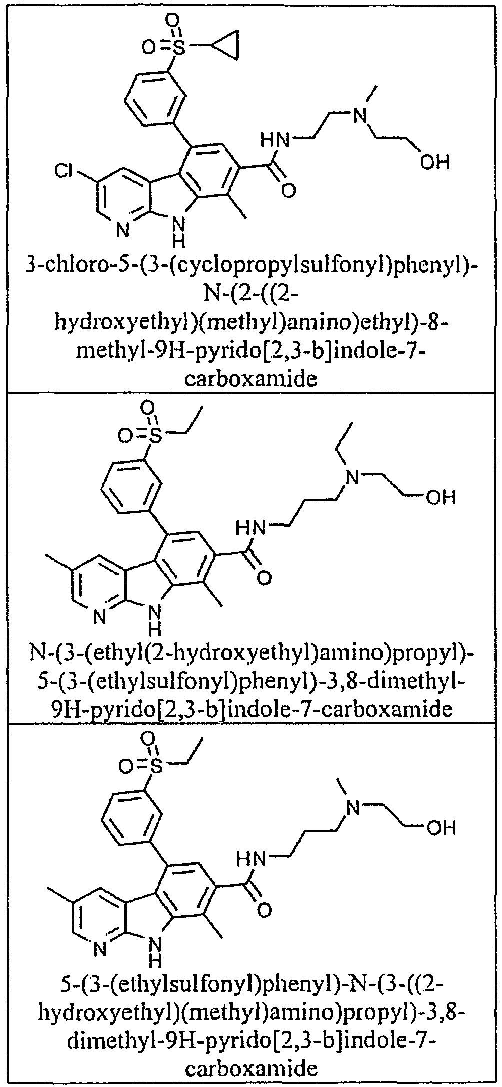

### Characterization of the Amorphous Form of Compound 88

The Amorphous Form of Compound 88 was characterized by XRPD and Ion Chromatograpgy.

### 1. X-Ray Powder Diffraction (XRPD)

X-ray powder diffraction (XRPD) analyses were performed using a Shimadzu XRD-6000 diffractometer. Real time data were collected using Cu-Kα radiation starting at approximately 3 °2θ at a scan rate of 2°/min with a step size of 0.04°. The tube voltage and amperage were set to 40 kV and 40 mA, respectively. The pattern is displayed from 2.5 to 45 °2θ. Samples were prepared for analysis by placing them on Si zero-return ultra-micro sample holders.

The resulting XRPD spectrum of the amorphous form of Compound 88 shows a diffuse halo with no discernable peaks, which confirms that the material is amorphous.

### 2. Ion Chromatography (IC)

Ion Chromatography (IC) was performed using a Dionex DX600 Ion Chromatograph using a Dionex IonPac AS 17, 250x4 mm column and a Dionex IonPac AS17, 50x4 mm guard column. The column temperature was 35±2 °C. The detector was operated in a suppressed conductivity mode with a Dionex ASRS Ultra 4 mm suppressor and a suppressor current of 220 mA. Mobile phase A was purified water and mobile phase B was potassium hydroxide (KOH), which was delivered using an eluent generator. A flow rate of 1.5 mL/min and an injection volume of 10 µL were used. The following gradient conditions were used:

| **Time (min)** | **Mobile Phase A** | **Concentration of KOH (mM)** |
|---|---|---|
| 0.0 | 100% | 5 |
| 3.0 | 100% | 5 |
| 10.0 | 100% | 15 |
| 20.0 | 100% | 60 |
| 20.1 | 100% | 5 |
| 30.0 | 100% | 5 |

IC analysis of the amorphous form of Compound 88 showed 7.6 wt% Cl⁻ present, which is 1.0 wt% higher than expected for a mono-HCl salt.

### Biological Testing

The activity of compounds as protein kinase inhibitors may be assayed in vitro, *in vivo* or in a cell line. In vitro assays include assays that determine inhibition of either the phosphorylation activity or ATPase activity of the activated protein kinase. Alternate in vitro assays quantitate the ability of the inhibitor to bind to the protein kinase. Inhibitor binding may be measured by radiolabelling the inhibitor prior to binding, isolating the inhibitor/protein kinase complex and determining the amount of radiolabel bound. Alternatively, inhibitor binding may be determined by running a competition experiment where new inhibitors are incubated with the protein kinase bound to known radioligands.

### A. Determination of Inhibition of AIK

The inhibitory properties of compounds relative to Aurora B/INCENP may be determined by the Direct Fluorescence Polarization detection method (FP) using a Greiner small volume black 384-well-plate format under the following reaction conditions: 50 mM Hepes pH 7.3, 10 mM MgCl₂, 10 mM NaCl, 1 mM DTT, 0.01% Brij35, 5FAM-GRTGRRNSI-NH2 (Provided by Anaspec), 5% DMSO, 10 µM ATP and 0.8 nM Aurora B/INCENP. Detection of the reaction product is performed by addition of Progressive IMAP binding reagent (Molecular Devices). Reaction product may be determined quantitatively by FP using an Analyst HT plate reader (Molecular Devices) with an excitation wavelength at 485 nm and emission at 530 nm and using a Fluorescein 505 dichroic mirror.

The assay reaction may be initiated as follows: 2 µl of (3x) 300 nM FI-Peptide/30 uM ATP was added to each well of the plate, followed by the addition of 2 µl of (3x) inhibitor (2 fold serial dilutions for 11 data points for each inhibitor) containing 15% DMSO. Two microliters of (3x) 2.4 nM AuroraB/INCENP solution may be added to initiate the reaction (final enzyme concentration was 0.8 nM for Aurora B/INCENP). The reaction mixture may then be incubated at room temperature for 45 min, and quenched and developed by addition of 20 µl of 1 to 400 diluted Progressive IMAP binding reagent in 1x proprietary Progressive IMAP binding buffer A. Fluorescence polarization readings of the resulting reaction mixtures may be measured after a 60-minute incubation at room temperature.

IC₅₀ values may be calculated by non-linear curve fitting of the compound concentrations and fluorescent polarization values to the standard IC₅₀ equation. As a reference point for this assay, Staurosporin showed an IC₅₀ of <10 nM. IC₅₀ values for select compounds of the invention against AIK B are given in Table 1.

**TABLE 1: IC₅₀ of Exemplified Compounds Against AIK B**

| **COMPOUND** | **IC₅₀ (nM)** |
|---|---|
| 88 | 6 - 10 |
| 90 | 6 - 10 |
| 95 | ≥11 |
| 100 | ≤5 |
| 101 | ≤5 |
| 105 | ≥11 |
| 112 | ≥11 |
| 113 | 6 - 10 |
| 114 | ≥11 |
| 117 | ≥11 |
| 120 | 6 - 10 |
| 142 | 6 - 10 |
| 153 | ≥11 |
| 154 | 6 - 10 |

### B. Determination of Inhibition of c-KIT

The inhibitory properties of compounds relative to c-Kit may be determined by the Time-Resolved Fluorescence Resonance Energy Transfer (TR-FRET) method using a small volume black 384-well-plate (Greiner) format under the following reaction conditions: 50 mM Hepes pH 7.3, 10 mM MgCl2, 10 mM NaCl, 1 mM DTT, 0.01% Brij35, 250 nM Biotin-EGPWLEEEEEAYGWMDF peptide (provided by SYNPEP), 5% DMSO, 100µM ATP. Detection of the reaction product may be performed by addition of Streptavidin-APC (Prozyme) and Eu-Anti-phosphotyrosine antibody (Perkin Elmer). Reaction product may be determined quantitatively by TR-FRET reading using an Analyst HT plate reader (Molecular Devices) with an excitation wavelength at 330 nm and emission at 615 nm (Europium) compared to 330 nm excitation (Europium) and emission 665 nm (APC) and using an Europium 400 dichroic mirror.

The assay reaction may be initiated as follows: 4 µl of (2.5x) 625 nM Biotin-Peptide / 250 µM ATP was added to each well of the plate, followed by the addition of 2 µl of (5x) inhibitor (2.5 fold serial dilutions for 11 data points for each inhibitor) containing 25% DMSO. 4 µl of (2.5x) c-Kit solution may be added to initiate the reaction (final enzyme concentration was 0.13 nM for c-Kit). The reaction mixture may then be incubated at room temperature for 30 min, and quenched and developed by addition of 10 µl of (2x) 3.2 nM Eu-Antibody and 25 nM Streptavidin-APC in 50mM Hepes pH 7.3, 30mM EDTA, 0.1% Triton X-100 buffer. TR-FRET readings of the resulting reaction mixtures may be measured after a 60-minute incubation at room temperature on the Analyst HT.
IC₅₀ values may be calculated by non-linear curve fitting of the compound concentrations and ratio metric Eu:APC values to the standard IC₅₀ equation. As a reference point for this assay, Staurosporin showed an IC₅₀ of <5 nM.

The following abbreviations have been used:
- ATP: Adenosine Triphophatase
- BSA: Bovine Serum Albumin
- EDTA: Ethylenediaminetetraacetic acid
- GSK3: Glycogen synthase kinase 3
- MOPS: Morpholinepropanesulfonic acid
- SPA: Scintillation Proximity Assay

It will be apparent to those skilled in the art that various modifications and variations can be made in the compounds, compositions, kits, and methods of the present invention without departing from the scope of the invention. Thus, it is intended that the present invention cover the modifications and variations of this invention provided they come within the scope of the appended claims.

## Claims

1. A compound selected from the group consisting of:
N-(2-(methylamino)ethyl)-5-(3-(ethylsulfonyl)phenyl)-3,8-dimethyl-9H-pyrido[2,3-b]indole-7-carboxamide;
N-(2-(methoxy)ethyl)-5-(3-(ethylsulfonyl)phenyl)-3,8-dimethyl-9H-pyrido[2,3-b]indole-7-carboxamide;
N-(2-(dimethylamino)ethyl)-N-methyl-5-(3-(ethylsulfonyl)phenyl)-3,8-dimethyl-9H-pyrido[2,3-b]indole-7-carboxamide;
N,N-dimethyl-5-(3-(ethylsulfonyl)phenyl)-3,8-dimethyl-9H-pyrido[2,3-b]indole-7-methylcarboxamide;
5-(3-(ethylsulfonyl)phenyl)-3,8-dimethyl-9H-pyrido[2,3-b]indole-7-yl)(4-methylpiperazin-1-yl)methanone;
5-(3-(ethylsulfonyl)phenyl)-3,8-dimethyl-N-(2-piperazin-1-yl)ethyl)-9H-pyrido[2,3-b]indole-7-carboxamide;
5-(3-(ethylsulfonyl)phenyl)-3,8-dimethyl-N-(3-(4-methylpiperazin-1-yl)propyl)-9H-pyrido[2,3-b]indole-7-carboxamide;
5-(3-(ethylsulfonyl)phenyl)-3,8-dimethyl-9H-pyrido[2,3-b]indole-7-yl)(morpholino)methanone;
azetidin-1-yl(5-(3-(ethylsulfonyl)phenyl)-3,8-dimethyl-9H-pyrido[2,3-b]indol-7-yl)methanone;
(5-(3-(ethylsulfonyl)phenyl)-3,8-dimethyl-9H-pyrido[2,3-b]indol-7-yl)(thaiazolidin-3-yl)methanone;
(R)-5-(3-(ethylsulfonyl)phenyl)-N-(2-hydroxypropyl)-3,8-dimethyl- 9H-pyrido[2,3-b]indole-7-carboxamide;
(S)-5-(3-(ethylsulfonyl)phenyl)-N-(2-hydroxypropyl)-3,8-dimethyl- 9H-pyrido[2,3-b]indole-7-carboxamide;
5-(3-(ethylsulfonyl)phenyl)-N-(2-hydroxyethyl)-3,8-dimethyl-9H-pyrido[2,3-b]indole-7-carboxamide;
N-(2,3-dihydroxypropyl)-5-(3-(ethylsulfonyl)phenyl)-3,8-dimethyl- 9H-pyrido[2,3-b]indole-7-carboxamide;
5-(3-(ethylsulfonyl)phenyl)-N-(2-hydroxy-2methylpropyl)-3,8-dimethyl-9H-pyrido[2,3-b]indole-7-carboxamide;
5-(3-(ethylsulfonyl)phenyl)-N-(1-isopropylpiperidin-4-yl)-3,8-dimethyl- 9H-pyrido[2,3-b]indole-7-carboxamide;
N-(1-ethylpiperidin-4-yl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-thiazol-2-yl)-9H-pyrido[2,3-b]indole-7-carboxamide;
5-(3-(ethylsulfonyl)phenyl)-3,8-dimethyl- N-(2-(2,2,2-trifluoroethoxy)ethyl-9H-pyrido[2,3-b] indole-7-carboxamide;
5-(3-(ethylsulfonyl)phenyl)-3,8-dimethyl- N-(piperidin-3-yl)-9H-pyrido[2,3-b]indole-7-carboxamide;
5-(3-(ethylsulfonyl)phenyl)-3,8-dimethyl-N-(piperidin-4-yl)-9H-pyrido[2,3-b]-b]indole-7-carboxamide;
5-(3-(ethylsulfonyl)phenyl)-3,8-dimethyl-N-(piperidin-3-yl)-9H-pyrido[2,3-b]indole-7-carboxamide;
5-(3-(ethylsulfonyl)phenyl)-N-(2-(2-hydroxyethoxy)ethyl-3,8-dimethyl-9H-pyrido[2,3-b]indole-7-carboxamide;
5-(3-(cyclopropanecarboxamido)phenyl)-N-(2-(dimethylamino)ethyl)-3,8-dimethyl-9H-pyrido[2,3-b]indole-7-carboxamide;
N-(2-(dimethylamino)ethyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-((1-methylpiperidin-4-yl)methyl)-9H-pyrido[2,3-b]indole-7-carboxamide;
N-(3-(dimethylamino)propyl)-5-(3-(ethylsulfonyl)phenyl)-3,8-dimethyl-9H-pyrido[2,3-b]indole-7-carboxamide;
5-(3-(ethylsulfonyl)phenyl)-3,8-dimethyl-N-(2-(pyirolidin-1-yl)ethyl)-9H-pyrido[2,3-b]indole-7-carboxamide;
(S)-5-(3-(ethylsulfonyl)phenyl)-3,8-dimethyl-N-(1-methylpiperidin-3 -yl)-9H-pyrido[2,3-b]indole-7-carboxamide;
(R)-5-(3-(ethylsulfonyl)phenyl)-3,8-dimethyl-N-(1-methylplperidin-3-yl)-9H-pyrido[2,3-b] indole-7-carboxamide;
5-(3-(cyclopropanecarboxamido)phenyl)-3,8-dimethyl-N-(1-methyl-piperidin-4-yl)-9H-pyrido[2,3-b]indole-7-carboxamide;
5-(3-(cyclopropylcarbamoyl)phenyl)-N-(2-(dimethylamino)ethyl)-3,8-dimethyl-9H-pyrido[2,3-b]indole-7-carboxamide;
[5-(3-Ethanesulfonyl-phenyl)-3-methyl-9*H*-pyrido[2,3-b]indol-7-yl]-(4-methyl-piperazin-1-yl)-methanone;
5-(3-Ethanesulfonyl-phenyl)-3-methyl-9*H*-pyrido[2,3-6]indole-7-carboxylic acid (2-dimethylamino-ethyl)-amide;
5-(3-Ethanesulfonyl-phenyl)-3-methyl-9*H*-pyrido[2,3-b]indole-7-carboxylic acid (3-dimethylamino-propyl)-amide;
5-(benzylthio)-N-(2-(dimethylamino)ethyl)-3-methyl-9H-pyrido[2,3-b]indole-7-carboxamide;
5-(3-(N-ethylsulfamoyl)phenyl)-8-methoxy-3-methyl-N-(1-methylpiperidin-4-yl)-9H-pyrido[2,3-b]indole-7-carboxamide;
5-(3-(cyclopropylsulfonyl)phenyl)-3,8-dimethyl-N-(1-methylpiperidin-4-yl)-9H-pyrido[2,3-b]indole-7-carboxamide;
N-(2-(diethylamino)ethyl)-5-(3-(ethylsulfonyl)phenyl)-3,8-dimethyl-9H-pyrido[2,3-b]indole-7-carboxamide; and
5-(3-(ethylsulfonyl)phenyl)-3,8-dimethyl-N-(3-morpholinopropyl)-9H-pyrido[2,3-b]indole-7-carboxamide; and
5-(3-(cyclopropylcarbamoyl)phenyl)-3,8-dimethyl-N-(1-methylpiperidin-4-yl)-9H-pyrido[2,3-b]indole-7-carboxamide;
or a pharmaceutically acceptable salt or solvate thereof.

2. A compound selected from the group consisting of:
5-(3-(ethylsulfonyl)phenyl)-N-((1*R*,4*R*)-4-hydroxycyclohexyl)-3,8-dimethyl-9H-pyrido[2,3-b]indole-7-carboxamide;
5-(3-(ethylsulfonyl)phenyl)-N-((1*S*,4*S*)-4-hydroxycyclohexyl)-3,8-dimethyl-9H-pyrido[2,3-b]indole-7-carboxamide;
5-(3-(ethylsulfonyl)phenyl)-N-(4-(hydroxymethyl)cyclohexyl)-3,8-dimethyl-9H-pyrido[2,3-b]indole-7-carboxamide;
5-(3-(ethylsulfonyl)phenyl)-N-(4-(2-hydroxyethyl)cyclohexyl)-3,8-dimethyl-9H-pyrido[2,3-b] indole-7-carboxamide;
(5-(3-(ethylsulfonyl)phenyl)-3,8-dimethyl-9H-pyrido[2,3-b]indol-7-yl)(4-(hydroxymethyl)piperidin-1-yl)methanone;
(5-(3-(ethylsulfonyl)phenyl)-3,8-dimethyl-9H-pyrido[2,3-b]indol-7-yl)(4-(2-hydroxyethyl)piperidin-1-yl)methanone;
(5-(3-(ethylsulfonyl)phenyl)-3,8-dimethyl-9H-pyrido[2,3-b]indol-7-yl)(4-hydroxypiperidin-1-yl)methanone;
(5-(3-(ethylsulfonyl)phenyl)-3,8-dimethyl-9H-pyrido[2,3-b]indol-7-yl)(2-(hydroxymethyl)pyrrolidin-1-yl)methanone;
(5-(3-(ethylsulfonyl)phenyl)-3,8-dimethyl-9H-pyrido[2,3-b]indol-7-yl)(2-(hydroxymethyl)morpholino)methanone;
5-(3-(ethylsulfonyl)phenyl)-N-(3-hydroxypropyl)-3,8-dimethyl-9H-pyrido[2,3-b]indole-7-carboxamide;
(R)-5-(3-(ethylsulfonyl)phenyl)-N-(3-hydroxybutyl)-3,8-dimethyl-9H-pyrido[2,3-b]indole-7-carboxamide;
(S)-5-(3-(ethylsulfonyl)phenyl)-N-(3-hydroxybutyl)-3,8-dimethyl-9H-pyrido[2,3-b]indole-7-carboxamide;
5-(3-(ethylsulfonyl)phenyl)-N-(3-hydroxy-3-methylbutyl)-3,8-dimethyl-9H-pyrido[2,3-b]indole-7-carboxamide;
(S)-N-(3-(dimethylamino)-2-hydroxypropyl)-5-(3-(ethylsulfonyl)phenyl)-3,8-dimethyl-9H-pyrido[2,3-b]indole-7-carboxamide;
(S)-N-(2-(dimethylamino)-3-hydroxypropyl)-5-(3-(ethylsulfonyl)phenyl)-3,8-dimethyl-9H-pyrido[2,3-b]indole-7-carboxamide;
N-(3-(ethyl(2-hydroxyethyl)amino)propyl)-5-(3-(ethylsulfonyl)phenyl)-3,8-dimethyl-9H-pyrido[2,3-b]indole-7-carboxamide;
5-(3-(ethylsulfonyl)phenyl)-N-(3 -((2-hydroxyethyl)(methyl)amino)-propyl)-3,8-dimethyl-9H-pyrido[2,3-b]indole-7-carboxamide;
N-(2-(ethyl(2-hydroxyethyl)amino)ethyl)-5-(3-(ethylsulfonyl)phenyl)-3,8-dimethyl-9H-pyrido[2,3-b]indole-7-carboxamide;
5-(3-(ethylsulfonyl)phenyl)-N-(2-((2-hydroxyethyl)(methyl)amino)ethyl)-3,8-dimethyl-9H-pyrido[2,3-b]indole-7-carboxamide;
5-(3-(ethylsulfonyl)phenyl)-8-methyl-N-(1-methylpiperidin-4-yl)-9H-pyrido[2,3-b]indole-7-carboxamide;
5-(3-(ethylsulfonyl)phenyl)-3-fluoro-8-methyl-N-(1-methylpiperidin-4-yl)-9H-pyrido[2,3-b]indole-7-carboxamide;
3-chloro-5-(3-(ethylsulfonyl)phenyl)-8-methyl-N-(1-methylpiperidin-4-yl)-9H-pyrido[2,3-b]indole-7-carboxamide;
5-(3-(cyclopropylsulfonyl)phenyl)-8-methyl-N-(1-methylpiperidin-4-yl)-9H-pyrido[2,3-b]indole-7-carboxamide;
5-(3-(cyclopropylsulfonyl)phenyl)-3-fluoro-8-methyl-N-(1-methylpiperidin-4-yl)-9H-pyrido[2,3-b]indole-7-carboxamide;
3-chloro-5-(3-(cyclopropylsulfonyl)phenyl)-8-methyl-N-(1-methylpiperidin-4-yl)-9H-pyrido[2,3-b]indole-7-carboxamide;
5-(3-(ethylsulfonyl)phenyl)-N-(1-(2-hydroxyethyl)piperidin-4-yl)-3,8-dimethyl-9H-pyrido[2,3-b]indole-7-carboxamide;
3-chloro-5-(3-(ethylsulfonyl)phenyl)-N-(1-(2-hydroxyethyl)piperidin-4-yl)-8-methyl-9H-pyrido[2,3-b]indole-7-carboxamide;
5-(3-(cyclopropylsulfonyl)phenyl)-N-(1-(2-hydroxyethyl)piperidin-4-yl)-3,8-dimethyl-9H-pyrido[2,3-b]indole-7-carboxamide;
3-chloro-5-(3-(cyclopropylsulfonyl)phenyl)-N-(1-(2-hydroxyethyl)piperidin-4-yl)-8-methyl-9H-pyrido[2,3-b]indole-7-carboxamide
5-(3-(ethylsulfonyl)phenyl)-N-(2-((2-hydroxyethyl)(methyl)amino)ethyl)-3,8-dimethyl-9H-pyrido[2,3-b]indole-7-carboxamide;
3-chloro-5-(3-(ethylsulfonyl)phenyl)-N-(2-((2-hydroxyethyl)(methyl)amino)ethyl)-8-methyl-9H-pyrido[2,3-b]indole-7-carboxamide;
5-(3-(cyclopropylsulfonyl)phenyl)-N-(2-((2-hydroxyethyl)(methyl)amino)ethyl)-3,8-dimethyl-9H-pyrido[2,3-b]indole-7-carboxamide;
3-chloro-5-(3-(cyclopropylsulfonyl)phenyl)-N-(2-((2-hydroxyethyl)(methyl)amino)ethyl)-8-methyl-9H-pyrido[2,3-b]indole-7-carboxamide;
N-(3-(ethyl(2-hydroxyethyl)amino)propyl)-5-(3-(ethylsulfonyl)phenyl)-3,8-dimethyl-9H-pyrido[2,3-b]indole-7-carboxamide; and
5-(3-(ethylsulfonyl)phenyl)-N-(3-((2-hydroxyethyl)(methyl)amino)-propyl)-3,8-dimethyl-9H-pyrido[2,3-b]indole-7-carboxamide;
or a pharmaceutically acceptable salt or solvate thereof.

3. The compound 5-(3-(ethylsulfonyl)phenyl)-3,8-dimethyl-N-(1-methylpiperidin-4-yl)-9H-pyrido[2,3-b]indole-7-carboxamide having the formula: 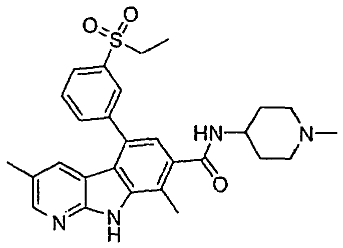 or a pharmaceutically acceptable salt or solvate thereof.

4. The compound according to claim 3 wherein the compound is in the form of a hydrochloric acid salt.

5. The compound according to claim 3 wherein the compound is in the form of a salt selected from the group consisting of a trifluoroacetic acid salt, a toluenesulfonic acid salt, a benzenesulfonic acid salt, a methanesulfonic acid salt, a succinic acid salt, a tartaric acid salt, a citric acid salt, a fumaric acid salt, a sulfuric acid salt, a phosphoric acid salt, a benzoic acid salt, a *bis*-hydrogen chloride salt, a *bis*-trifluoroacetic acid salt, a tosylate salt, a hemi-fumarate salt, a lactic acid salt, a malic acid salt, a hippuric acid salt and a hydrobromic acid salt.

6. A pharmaceutical composition comprising, as an active ingredient, a compound according to any one of claims 1-5 and a pharmaceutically acceptable excipient.

7. A compound according to any one of claims 1-5 for use as a medicament.

8. A compound according to any one of claims 1-5 for use in treating cancer.

9. The compound of claim 8, wherein the cancer is selected from the group consisting of squamous cell carcinoma, astrocytoma, Kaposi's sarcoma, glioblastoma, non small-cell lung cancer, bladder cancer, head and neck cancer, melanoma, ovarian cancer, prostate cancer, breast cancer, small-cell lung cancer, glioma, colorectal cancer, genitourinary cancer, gastrointestinal cancer, thyroid cancer and skin cancer.

10. A compound according to any one of claims 1-5 for use in treating inflammation, inflammatory bowel disease, psoriasis, or transplant rejection.

11. A compound according to any one of claims 1-5 for use in preventing or treating amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss and contraceptive medication.

12. A compound according to any one of claims 1-5 for use in preventing or treating mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment and androgenetic alopecia.

13. A compound according to any one of claims 1-5 for use in preventing or treating dementia related diseases, Alzheimer's Disease and conditions associated with kinases.

14. The compound of claim 13, wherein the dementia related diseases are selected from the group consisting of Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica.

15. A compound according to any one of claims 1-5 for use in treating arthritis.

## Patentansprüche

1. Verbindung, ausgewählt aus der Gruppe bestehend aus:
N-(2-(Methylamino)ethyl)-5-(3-(ethylsulfonyl)phenyl)-3, 8-dimethyl-9H-pyrido [2,3-b]indol-7-carboxamid;
N-(2-(Methoxy)ethyl)-5-(3-(ethylsulfonyl) phenyl)-3,8-dimethyl-9 H-pyrido [2,3-b]indol-7-carboxamid;
N-(2-(Dimethylamino)ethyl)-N-methyl-5-(3-(ethylsulfonyl)phenyl)-3,8-dimethyl-9H-pyrido[2,3-b]indol-7-carboxamid;
N,N-Dimethyl-5-(3-(ethylsulfonyl)phenyl)-3,8-dimethyl-9H-pyrido[2,3-b]indol-7-methylcarboxamid;
5-(3-(Ethylsulfonyl)phenyl)-3,8-dimethyl-9H-pyrido[2,3-b]indol-7-yl)(4-methylpiperazin-1-yl)methanon;
5-(3-(Ethylsulfonyl)phenyl)-3,8-dimethyl-N-(2-piperazin-1-yl)ethyl)-9H-pyrido[2,3-b]indol-7-carboxamid;
5-(3-(Ethylsulfonyl)phenyl)-3 ,8-dimethyl-N-(3-(4-methylpiperazin-1-yl)propyl)-9H-pyrido[2,3-b]indol-7-carboxamid;
5-(3-(Ethylsulfonyl)phenyl)-3 ,8-dimethyl-9H-pyrido [2,3-b]indol-7-yl)(morpholino)methanon;
Azetidin-1-yl(5-(3-(ethylsulfonyl)phenyl)-3,8-dimethyl-9H-pyrido[2,3-b]indol-7-yl)methanon;
(5-(3-(Ethylsulfonyl)phenyl)-3,8-dimethyl-9H-pyrido[2,3-b] indol-7-yl)(thaiazolidin-3-yl)methanon;
(R)-5-(3-(Ethylsulfonyl)phenyl)-N-(2-hydroxypropyl)-3,8-dimethyl-9H-pyrido[2,3-b]indol-7-carboxamid;
(S)-5-(3-(Ethylsulfonyl)phenyl)-N-(2-hydroxypropyl)-3,8-dimethyl-9H-pyrido[2,3-b] indol-7-carboxamid;
5-(3-(Ethylsulfonyl)phenyl)-N-(2-hydroxyethyl)-3,8-dimethyl-9H-pyrido[2,3-b]indol-7-carboxamid;
N-(2,3-Dihydroxypropyl)-5-(3-(ethylsulfonyl)phenyl)-3,8-dimethyl-9H-pyrido[2,3-b] indol-7-carboxamid;
5-(3-(Ethylsulfonyl)phenyl)-N-(2-hydroxy-2methylpropyl)-3,8-dimethyl-9H-pyrido[2,3-b]indol-7-carboxamid;
5-(3-(Ethylsulfonyl)phenyl)-N-(1-isopropylpiperidin-4-yl)-3,8-dimethyl-9H-pyrido[2,3-b]indol-7-carboxamid;
N-(1-Ethylpiperidin-4-yl)-5-(3-(ethylsulfonyl)phenyl)-3,8-dimethyl-9H-pyrido [2,3-b]indol-7-carboxamid;
5-(3-(Ethylsulfonyl)phenyl)-3,8-dimethyl-N-thiazol-2-yl)-9H-pyrido[2,3-b]indol-7-carboxamid;
5-(3-(Ethylsulfonyl)phenyl)-3,8-dimethyl-N-(2-(2,2,2-trifluorethoxy)ethyl-9H-pyrido[2,3-b]indol-7-carboxamid;
5-(3-(Ethylsulfonyl)phenyl)-3,8-dimethyl-N-(piperidin-3-yl)-9H-pyrido[2,3-b]indol-7-carboxamid;
5-(3-(Ethylsulfonyl)phenyl)-3,8-dimethyl-N-(piperidin-4-yl)-9H-pyrido[2,3-b]indol-7-carboxamid;
5-(3-(Ethylsulfonyl)phenyl)-3,8-dimethyl-N-(piperidin-3-yl)-9H-pyrido[2,3-b]indol-7-carboxamid;
5-(3-(Ethylsulfonyl)phenyl)-N-(2-(2-hydroxyethoxy)ethyl-3,8-dimethyl-9H-pyrido [2,3-b]indol-7-carboxamid;
5-(3-(Cyclopropancarboxamido)phenyl)-N-(2-(dimethylamino)ethyl)-3,8-dimethyl-9H-pyrido[2,3-b]indol-7-carboxamid;
N-(2-(Dimethylamino)ethyl)-5-(3-(ethylsulfonyl)phenyl)-3,8-dimethyl-9H-pyrido[2,3-b]indol-7-carboxamid;
5-(3-(Ethylsulfonyl)phenyl)-3,8-dimethyl-N-((1-methylpiperidin-4-yl)methyl)-9H-pyrido[2,3-b]indol-7-carboxamid;
N-(3-(Dimethylamino)propyl)-5-(3-(ethylsulfonyl)phenyl)-3,8-dimethyl-9H-pyrido[2,3-b]indol-7-carboxamid;
5-(3-(Ethylsulfonyl)phenyl)-3,8-dimethyl-N-(2-(pyrrolidin-1-yl)ethyl)-9H-pyrido[2,3-b]indol-7-carboxamid;
(S)-5-(3-(Ethylsulfonyl)phenyl)-3,8-dimethyl-N-(1-methylpiperidin-3-yl)-9H-pyrido [2,3-b]indol-7-carboxamid;
(R)-5-(3-(Ethylsulfonyl)phenyl)-3,8-dimethyl-N-(1-methylpiperidin-3-yl)-9H-pyrido[2,3-b)indol-7-carboxamid;
5-(3-(Cyclopropancarboxamido)phenyl)-3,8-dimethyl-N-(1-methyl-piperidin-4-yl)-9H-pyrido[2,3-b]indol-7-carboxamid;
5-(3-(Cyclopropylcarbamoyl)phenyl)-N-(2-(dimethylamino)ethyl)-3,8-dimethyl-9H-pyrido[2,3-b]indol-7-carboxamid;
[5-(3-Ethansulfonyl-phenyl)-3-methyl-9H-pyrido[2,3-b]indol-7-yl]-(4-methylpiperazin-1-yl)-methanon;
5-(3-Ethansulfonyl-phenyl)-3-methyl-9H-pyrido[2,3-b]indol-7-carbonsäure(2-dimethylamino-ethyl)-amid;
5-(3-Ethansulfonyl-phenyl)-3-methyl-9H-pyrido [2,3-b]indol-7-carbonsäure(3-dimethylamino-propyl)-amid;
5-(Benzylthio)-N-(2-(dimethylamino)ethyl)-3-methyl-9H-pyrido[2,3-b]indol-7-carboxamid;
5-(3-(N-Ethylsulfamoyl)phenyl)-8-methoxy-3-methyl-N-(1-methylpiperidin-4-yl)-9H-pyrido[2,3-b]indol-7-carboxamid;
5-(3-(Cyclopropylsulfonyl)phenyl)-3,8-dimethyl-N-(1-methylpiperidin-4-yl)-9H-pyrido[2,3-b]indol-7-carboxamid;
N-(2-(Diethylamino)ethyl)-5-(3-(ethylsulfonyl)phenyl)-3,8-dimethyl-9H-pyrido[2,3-b]indol-7-carboxamid und
5-(3-(Ethylsulfonyl)phenyl)-3,8-dimethyl-N-(3-morpholinopropyl)-9H-pyrido[2,3-b] indol-7-carboxamid und
5-(3-(Cyclopropylcarbamoyl)phenyl)-3,8-dimethyl-N-(1-methylpiperidin-4-yl)-9H-pyrido[2,3-b]indol-7-carboxamid
oder ein pharmazeutisch zulässiges Salz oder Solvat davon.

2. Verbindung, ausgewählt aus der Gruppe bestehend aus:
5-(3-(Ethylsulfonyl)phenyl)-N-((1R,4R)-4-hydroxycyclohexyl)-3,8-dimethyl-9H-pyrido[2,3-b]indol-7-carboxamid;
5-(3-(Ethylsulfonyl)phenyl)-N-((1S,4S)-4-hydroxycyclohexyl)-3,8-dimethyl-9H-pyrido [2,3-b]indol-7-carboxamid;
5-(3-(Ethylsulfonyl)phenyl)-N-(4-(hydroxymethyl)cyclohexyl)-3,8-dimethyl-9H-pyrido [2,3-b]indol-7-carboxamid;
5-(3-(Ethylsulfonyl)phenyl)-N-(4-(2-hydroxyethyl)cyclohexyl)-3,8-dimethyl-9H-pyrido[2,3-b]indol-7-carboxamid;
(5-(3-(Ethylsulfonyl)phenyl)-3,8-dimethyl-9H-pyrido[2,3-b]indol-7-yl)(4-(hydroxymethyl)piperidin-1-yl)methanon;
(5-(3-(Ethylsulfonyl)phenyl)-3,8-dimethyl-9H-pyrido[2,3-b]indol-7-yl)(4-(2-hydroxyethyl)piperidin-1-yl)methanon;
(5-(3-(Ethylsulfonyl)phenyl)-3,8-dimethyl-9H-pyrido[2,3-b]indol-7-yl)(4-hydroxypiperidin-1-yl)methanon;
(5-(3-(Ethylsulfonyl)phenyl)-3,8-dimethyl-9H-pyrido[2,3-b]indol-7-yl)(2-(hydroxymethyl)pyrrolidin-1-yl)methanon;
(5-(3-(Ethylsulfonyl)phenyl)-3,8-dimethyl-9H-pyrido[2,3-b]indol-7-yl)(2-(hydroxymethyl)morpholino)methanon;
5-(3-(Ethylsulfonyl)phenyl)-N-(3-hydroxypropyl)-3,8-dimethyl-9H-pyrido[2,3-b]indol-7-carboxamid;
(R)-5-(3-(Ethylsulfonyl)phenyl)-N-(3-hydroxybutyl)-3,8-dimethyl-9H-pyrido[2,3-b]indol-7-carboxamid;
(S)-5-(3-(Ethylsulfonyl)phenyl)-N-(3-hydroxybutyl)-3,8-dimethyl-9H-pyrido[2,3-b]indol-7-carboxamid;
5-(3-(Ethylsulfonyl)phenyl)-N-(3-hydroxy-3-methylbutyl)-3,8-dimethyl-9H-pyrido[2,3-b]indol-7-carboxamid;
(S)-N-(3-(Dimethylamino)-2-hydroxypropyl)-5-(3-(ethylsulfonyl)phenyl)-3,8-dimethyl-9H-pyrido[2,3-b]indol-7-carboxamid;
(S)-N-(2-(Dimethylamino)-3-hydroxypropyl)-5-(3-(ethylsulfonyl)phenyl)-3,8-dimethyl-9H-pyrido[2,3-b]indol-7-carboxamid;
N-(3-(Ethyl(2-hydroxyethyl)amino)propyl)-5-(3-(ethylsulfonyl)phenyl)-3,8-dimethyl-9H-pyrido[2,3-b]indol-7-carboxamid;
5-(3-(Ethylsulfonyl)phenyl)-N-(3-((2-hydroxyethyl)(methyl)amino)-propyl)-3,8-dimethyl-9H-pyrido[2,3-b]indol-7-carboxamid;
N-(2-(Ethyl(2-hydroxyethyl)amino)ethyl)-5-(3-(ethylsulfonyl)phenyl)-3,8-dimethyl-9H-pyrido[2,3-b]indol-7-carboxamid;
5-(3-(Ethylsulfonyl)phenyl)-N-(2-((2-hydroxyethyl)(methyl)amino)ethyl)-3,8-dimethyl-9H-pyrido[2,3-b]indol-7-carboxamid;
5-(3-(Ethylsulfonyl)phenyl)-8-methyl-N-(1-methylpiperidin-4-yl)-9H-pyrido [2,3-b] indol-7-carboxamid;
5-(3-(Ethylsulfonyl)phenyl)-3-fluor-8-methyl-N-(1-methylpiperidin-4-yl)-9H-pyrido [2,3-b]indol-7-carboxamid;
3-Chlor-5-(3-(ethylsulfonyl)phenyl)-8-methyl-N-(1-methylpiperidin-4-yl)-9H-pyrido [2,3-b]indol-7-carboxamid;
5-(3-(Cyclopropylsulfonyl)phenyl)-8-methyl-N-(1-methylpiperidin-4-yl)-9H-pyrido[2,3-b)indol-7-carboxamid;
5-(3-(Cyclopropylsulfonyl)phenyl)-3-fluor-8-methyl-N-(1-methylpiperidin-4-yl)-9H-pyrido[2,3-b]indol-7-carboxamid;
3-Chlor-5-(3-(cyclopropylsulfonyl)phenyl)-8-methyl-N-(1-methylpiperidin-4-yl)-9H-pylido[2,3-b]indol-7-carboxamid;
5-(3-(Ethylsulfonyl)phenyl)-N-(1-(2-hydroxyethyl)piperidin-4-yl)-3,8-dimethyl-9H-pyrido[2,3-b]indol-7-carboxamid;
3-Chlor-5-(3-(ethylsulfonyl)phenyl)-N-(1-(2-hydroxyethyl)piperidin-4-yl)-8-methyl-9H-pyrido[2,3-b]indol-7-carboxamid;
5-(3-(Cyclopropylsulfonyl)phenyl)-N-(1-(2-hydroxyethyl)piperidin-4-yl)-3,8-dimethyl-9H-pyrido[2,3-b]indol-7-carboxamid;
3-Chlor-5-(3-(cyclopropylsulfonyl)phenyl)-N-(1-(2-hydroxyethyl)piperidin-4-yl)-8-methyl-9H-pyrido[2,3-b]indol-7-carboxamid;
5-(3-(Ethylsulfonyl)phenyl)-N-(2-((2-hydroxyethyl)(methyl)amino)ethyl)-3,8-dimethyl-9H-pyrido[2,3-b]indol-7-carboxamid;
3-Chlor-5-(3-(ethylsulfonyl)phenyl)-N-(2-((2-hydroxyethyl)(methyl)amino)ethyl)-8-methyl-9H-pyrido[2,3-b]indol-7-carboxamid;
5-(3-(Cyclopropylsulfonyl)phenyl)-N-(2-((2-hydroxyethyl)(methyl)amino)ethyl)-3,8-dimethyl-9H-pyrido[2,3-b]indol-7-carboxamid;
3-Chlor-5-(3-(cyclopropylsulfonyl)phenyl)-N-(2-((2-hydroxyethyl)(methyl)amino)ethyl)-8-methyl-9H-pyrido[2,3-b]indol-7-carboxamid;
N-(3-(Ethyl(2-hydroxyethyl)amino)propyl)-5-(3-(ethylsulfonyl)phenyl)-3,8-dimethyl-9H-pyrido[2,3-b]indol-7-carboxamid und
5-(3-(Ethylsulfonyl)phenyl)-N-(3-((2-hydroxyethyl)(methyl)amino)-propyl-3,8-dimethyl-9H-pyrido[2,3-b]indol-7-carboxamid
oder ein pharmazeutisch zulässiges Salz oder Solvat davon.

3. Verbindung 5-(3-(Ethylsulfonyl)phenyl)-3,8-dimethyl-N-(1-methylpiperidin-4-yl)-9H-pyrido[2,3-b]indol-7-carboxamid mit der Formel: 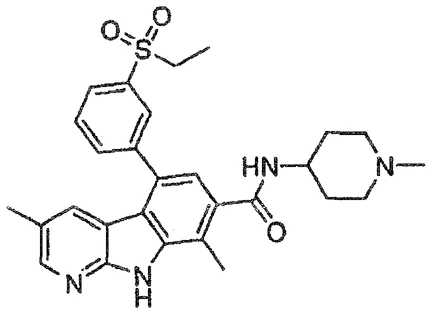 oder ein pharmazeutisch zulässiges Salz oder Solvat davon.

4. Verbindung nach Anspruch 3, wobei die Verbindung in der Form eines Chlorwasserstoffsäuresalzes vorliegt.

5. Verbindung nach Anspruch 3, wobei die Verbindung in der Form eines Salzes vorliegt, das aus der Gruppe ausgewählt ist, die aus einem Trifluoressigsäuresalz, einem Toluolsufonsäuresalz, einem Benzolsulfonsäuresalz, einem Methansulfonsäuresalz, einem Bernsteinsäuresalz, einem Weinsäuresalz, einem Zitronensäuresalz, einem Fumarsäuresalz, einem Schwefelsäuresalz, einem Phosphorsäuresalz, einem Benzoesäuresalz, einem Bis-Chlorwasserstoffsalz, einem Bis-Trifluoressigsäuresalz, einem Tosylatsalz, einem Hemifumaratsalz, einem Milchsäuresalz, einem Äpfelsäuresalz, einem Hippursäuresalz und einem Bromwasserstoffsäuresalz besteht.

6. Pharmazeutische Zusammensetzung, umfassend eine Verbindung nach einem der Ansprüche 1-5 als Wirkstoff und einen pharmazeutisch zulässigen Hilfsstoff.

7. Verbindung nach einem der Ansprüche 1-5 für die Verwendung als Medikament.

8. Verbindung nach einem der Ansprüche 1-5 für die Verwendung beim Behandeln von Krebs.

9. Verbindung nach Anspruch 8, wobei der Krebs aus der Gruppe ausgewählt ist, die aus Plattenepithelzellkarzinom, Astrozytom, Kaposisarkom, Glioblastom, nichtkleinzelligem Lungenkrebs, Blasenkrebs, Kopf- und Halskrebs, Melanom, Ovarialkrebs, Prostatakrebs, Brustkrebs, kleinzelligem Lungenkrebs, Gliom, Kolorektalkrebs, Urogenitalkrebs, Magendarmkrebs, Schilddrüsenkrebs und Hautkrebs besteht.

10. Verbindung nach einem der Ansprüche 1-5 für die Verwendung beim Behandeln von Entzündung, entzündlicher Darmerkrankung, Psoriasis oder Transplantatabstoßung.

11. Verbindung nach einem der Ansprüche 1-5 für die Verwendung beim Verhindern oder Behandeln von amyotropher Lateralsklerose, kortikobasaler Degeneration, Down-Syndrom, Huntington-Krankheit, Parkinson-Krankheit, postenzephelatischem Parkinson-Syndrom, progressiver supranukleärer Blickparese, Pick-Krankheit, Niemann-Pick-Krankheit, Schlaganfall, Kopftrauma und anderen chronischen neurodegenerativen Erkrankungen, Bipolar-Krankheit, affektiven Störungen, Depression, Schizophrenie, kognitiven Störungen, Haarausfall und empfängnisverhütende Medikation.

12. Verbindung nach einem der Ansprüche 1-5 für die Verwendung beim Verhindern oder Behandeln von leichtem kognitivem Defizit, altersbedingter Gedächtnisstörung, altersbedingtem kognitivem Abbau, kognitivem Defizit ohne Demenz, leichtem kognitivem Abbau, leichtem neurokognitivem Abbau, Altersvergesslichkeit, Gedächtnisstörung und kognitivem Defizit und androgener Alopezie.

13. Verbindung nach einem der Ansprüche 1-5 für die Verwendung beim Verhindern oder Behandeln von demenzbedingten Erkrankungen, Alzheimer-Krankheit und Zuständen in Zusammenhang mit Kinasen.

14. Verbindung nach Anspruch 13, wobei die demenzbedingten Erkrankungen aus der Gruppe ausgewählt sind, die aus frontotemporaler Demenz mit Parkinsonismus, Guam-Parkinson-Demenz-Komplex, HIV-Demenz, Krankheiten mit assoziierter Neurofibrillenpathologie, Prädemenzzuständen, vaskulärer Demenz, Demenz mit Lewy-Körperchen, frontotemporaler Demenz und Dementia pugilistica besteht.

15. Verbindung nach einem der Ansprüche 1-5 für die Verwendung beim Behandeln von Arthritis.

## Revendications

1. Composé choisi dans le groupe constitué par :
*N*-(2-(méthylamino)éthyl)-5-(3-(éthylsulfonyl)phényl)-3,8-diméthyl-9*H-*pyrido[2,3-b]indole-7-carboxamide ;
*N*-(2-(méthoxy)éthyl)-5-(3-(éthylsulfonyl)phényl)-3,8-diméthyl-9*H*-pyrido-[2,3-b]indole-7-carboxamide ;
*N*-(2-(diméthylamino)éthyl)-N-méthyl-5-(3-(éthylsulfonyl)phényl)-3,8-diméthyl-9*H*-pyrido[2,3-b]indole-7-carboxamide ;
*N*,*N*-diméthyl-5-(3-(éthylsulfonyl)phényl)-3,8-diméthyl-9*H*-pyrido[2,3-b]-indole-7-méthylcarboxamide ;
5-(3-(Éthylsulfonyl)phényl)-3,8-diméthyl-9*H*-pyrido[2,3-b]indol-7-yl)-(4-méthylpipérazin-1-yl)méthanone ;
5-(3-(Éthylsulfonyl)phényl)-3,8-diméthyl-*N*-(2-pipérazin-1-yl)éthyl)-9H-pyrido[2,3-b]indole-7-carboxamide ;
5-(3-(Éthylsulfonyl)phényl)-3,8-diméthyl-*N*-(3-(4-méthylpipérazin-1-yl)-propyl)-9*H*-pyrido[2,3-b)indole-7-carboxamide ;
5-(3-(Éthylsulfonyl)phényl)-3,8-diméthyl-9*H*-pyrido[2,3-b]indol-7-yl)-(morpholino)méthanone ;
Azétidin-1-yl(5-(3-(éthylsulfonyl)phényl)-3,8-diméthyl-9*H*-pyrido[2,3-b]-indol-7-yl)méthanone ;
(5-(3-(Éthylsulfonyl)phényl)-3,8-diméthyl-9*H*-pyrido[2,3-b]indol-7-yl)-(thiazolidin-3-yl)méthanone ;
(*R*)-5-(3-(éthylsulfonyl)phényl)-*N*-(2-hydroxypropyl)-3,8-diméthyl-9*H-*pyrido[2,3-b]indole-7-carboxamide ;
(*S*)-5-(3-(éthylsulfonyl)phényl)-*N*-(2-hydroxypropyl)-3,8-diméthyl-9*H-*pyrido[2,3-b]indole-7-carboxamide ;
5-(3-(Éthylsulfonyl)phényl)-*N*-(2-hydroxyéthyl)-3,8-diméthyl-9*H*-pyrido-[2,3-b]indole-7-carboxamide ;
*N*-(2,3-dihydroxypropyl)-5-(3-(éthylsulfonyl)phényl)-3,8-diméthyl-9*H-*pyrido[2,3-b]indole-7-carboxamide ;
5-(3-(Éthylsulfonyl)phényl)-*N*-(2-hydroxy-2-méthylpropyl)-3,8-diméthyl-9*H-*pyrido[2,3-b]indole-7-carboxamide ;
5-(3-(Éthylsulfonyl)phényl)-*N*-(1-isopropylpipéridin-4-yl)-3,8-diméthyl-9H-pyrido[2,3-b)indole-7-carboxamide ;
*N*-(1-éthylpipéridin-4-yl)-5-(3-(éthylsulfonyl)phényl)-3,8-diméthyl-9*H-*pyrido[2,3-b]indole-7-carboxamide ;
5-(3-(Éthylsulfonyl)phényl)-3,8-diméthyl-*N*-thiazol-2-yl)-9*H*-pyrido[2,3-b]-indole-7-carboxamide ;
5-(3-(Éthylsulfonyl)phényl)-3,8-diméthyl-*N*-(2-(2,2,2-triftuoroéthoxy)éthyl-9*H*-pyrido[2,3-b]indole-7-carboxamide ;
5-(3-(Éthylsulfonyl)phényl)-3,8-diméthyl-*N*-(pipéridin-3-yl)-9*H*-pyrido-[2,3-b]indole-7-carboxamide ;
5-(3-(Éthylsulfonyl)phényl)-3,8-diméthyl-*N*-(pipéridin-4-yl)-9H-pyrido-[2,3-b]indole-7-carboxamide ;
5-(3-(Éthylsulfonyl)phényl)-3,8-diméthyl-*N*-(pipéridin-3-yl)-9*H*-pyrido-[2,3-b]indole-7-carboxamide ;
5-(3-(Éthylsulfonyl)phényl)-*N*-(2-(2-hydroxyéthoxy)éthyl-3,8-diméthyl-9*H-*pyrido[2,3-b]indole-7-carboxamide ;
5-(3-(Cyclopropanecarboxamido)phényl)-*N*-(2-(diméthylamino)éthyl)-3,8-diméthyl-9*H*-pyrido[2,3-b]indole-7-carboxamide ;
*N*-(2-(diméthylamino)éthyl)-5-(3-(éthylsulfonyl)phényl)-3,8-diméthyl-9*H-*pyrido[2,3-b]indole-7-carboxamide ;
5-(3-(Éthylsulfonyl)phényl)-3,8-diméthyl-*N*-((1-méthylpipéridin-4-yl)-méthyl)-9*H*-pyrido[2,3-b]indole-7-carboxamide ;
*N*-(3-(diméthylamino)propyl)-5-(3-(éthylsulfonyl)phényl)-3,8-diméthyl-9*H-*pyrido[2,3-b]indole-7-carboxamide ;
5-(3-(Éthylsulfonyl)phényl)-3,8-diméthyl-*N*-(2-(pyrrolidin-1-yl)éthyl)-9*H-*pyrido[2,3-b]indole-7-carboxamide ;
(*S*)-5-(3-(éthylsulfonyl)phényl)-3,8-diméthyl-*N*-(1-méthylpipéridin-3-yl)-9*H-*pyrido[2,3-b]indole-7-carboxamide ;
(*R*)-5-(3-(éthylsulfonyl)phényl)-3,8-diméthyl-*N*-(1-méthylpipéridin-3-yl)-9*H-*pyrido[2,3-b]indole-7-carboxamide ;
5-(3-(Cyclopropanecarboxamido)phényl)-3,8-diméthyl-*N*-(1-méthyl-pipéridin-4-yl)-9*H*-pyrido[2,3-b]indole-7-carboxamide ;
5-(3-(Cyclopropylcarbamoyl)phényl)-*N*-(2-(diméthylamino)éthyl)-3,8-diméthyl-9*H*-pyrido[2,3-b]indole-7-carboxamide ;
[5-(3-Éthanesulfonylphényl)-3-méthyl-9*H*-pyrido[2,3-b]indol-7-yl]-(4-méthyl-pipérazin-1-yl)méthanone ;
(2-Diméthylaminoéthyl)amide d'acide 5-(3-éthanesulfonylphényl)-3-méthyl-9*H*-pyrido[2,3-b]indole-7-carboxylique ;
(3-Diméthylaminopropyl)amide d'acide 5-(3-éthanesulfonylphényl)-3-méthyl-9*H*-pyrido[2,3-b]indole-7-carboxylique ;
5-(Benzylthio)-*N*-(2-(diméthylamino)éthyl)-3-méthyl-9*H*-pyrido[2,3-b]-indole-7-carboxamide ;
5-(3-(*N*-éthylsulfamoyl)phényl)-8-méthoxy-3-méthyl-*N*-(1-méthylpipéridin-4-yl)-9*H*-pyrido[2,3-b]indole-7-carboxamide ;
5-(3-(Cydopropylsulfonyl)phényl)-3,8-diméthyl-*N*-(1-méthylpipéridin-4-yl)-9*H*-pyrido[2,3-b]indole-7-carboxamide ;
*N*-(2-(diéthylamino)éthyl)-5-(3-(éthylsulfonyl)phényl)-3,8-diméthyl-9*H-*pyrido[2,3-b]indole-7-carboxamido ;
5-(3-(Éthylsulfonyl)phényl)-3,8-diméthyl-*N*-(3-morpholinopropyl)-9H-pyrido[2,3-b]indole-7-carboxamide ; et
5-(3-(Cyclopropylcarbamoyl)phenyl)-3,8-diméthyl-*N*-(1-méthylpipéridin-4-yl)-9*H*-pyrido[2,3-b]indole-7-carboxamide ;
ou un sel ou solvate pharmaceutiquement acceptable de ce composé.

2. Composé choisi dans le groupe constitué par :
5-(3-(Éthylsulfonyl)phényl)-*N*-((1*R*,4*R*)-4-hydroxycyclohexyl)-3,8-diméthyl-9*H*-pyrido[2,3-b]indole-7-carboxamide ;
5-(3-(Éthylsulfonyl)phényl)-*N*-((1*S*,4*S*)-4-hydroxycyclohexyl)-3,8-diméthyl-9*H*-pyrido[2,3-b]indole-7-carboxamide ;
5-(3-(Éthylsulfonyl)phényl)-*N*-(4-(hydroxyméthyl)cyclohexyl)-3,8-diméthyl-9*H*-pyrido[2,3-b]indole-7-carboxamide ;
5-(3-(Éthylsulfonyl)phényl)-*N*-(4-(2-hydroxyéthyl)cyclohexyl)-3,8-diméthyl-9*H*-pyrido[2,3-b]indole-7-carboxamide ;
(5-(3-(Éthylsulfonyl)phényl)-3,8-diméthyl-9*H*-pyrido[2,3-b]indol-7-yl)(4-(hydroxyméthyl)pipéridin-1-yl)méthanone ;
(5-(3-(Éthylsulfonyl)phényl)-3,8-diméthyl-9*H*-pyrido[2,3-b]indol-7-yl)(4-(2-hydroxyéthyl)pipéridin-1-yl)méthanone ;
(5-(3-(Éthylsulfonyl)phényl)-3,8-diméthyl-9*H*-pyrido[2,3-b]indol-7-yl)(4-hydroxypipéridin-1-yl)méthanone ;
(5-(3-(Éthylsulfonyl)phényl)-3,8-diméthyl-9*H*-pyrido[2,3-b]indol-7-yl)(2-(hydroxyméthyl)pyrrolidin-1-yl)méthanone ;
(5-(3-(Éthylsulfonyl)phényl)-3,8-diméthyl-9*H*-pyrido[2,3-b]indol-7-yl)(2-(hydroxyméthyl)morpholino)méthanone ;
5-(3-(Éthylsulfonyl)phényl)-*N*-(3-hydroxypropyl)-3,8-diméthyl-9*H*-pyrido-[2,3-b]indole-7-carboxamide ;
(*R*)-5-(3-(éthylsulfonyl)phényl)-*N*-(3-hydroxybutyl)-3,8-diméthyl-9*H*-pyrido-[2,3-b]indole-7-carboxamide ;
(*S*)-5-(3-(éthylsulfonyl)phényl)-*N*-(3-hydroxybutyl)-3,8-diméthyl-9*H-*pyrido[2,3-b]indole-7-carboxamide ;
5-(3-(Éthylsulfonyl)phényl)-*N*-(3-hydroxy-3-méthylbutyl)-3,8-diméthyl-9*H-*pyrido[2,3-b]indole-7-carboxamide ;
(*S*)-*N*-(3-(diméthylamino)-2-hydroxypropyl)-5-(3-(éthylsulfonyl)phényl)-3,8-diméthyl-9*H*-pyrido[2,3-b]indole-7-carboxamide ;
(*S*)-*N*-(2-(diméthylamino)-3-hydroxypropyl)-5-(3-(éthylsulfonyl)phényl)-38-dimthyl-9*H*-pyrido[2,3-b]indole-7-carboxamide ;
*N*-(3-(éthyl(2-hydroxyéthyl)amino)propyl)-5-(3-(éthylsulfonyl)phényl)-3,8-diméthyl-9*H*-pyrido[2,3-b]indole-7-carboxamide ;
5-(3-(Éthylsulfonyl)phényl)-*N*-(3-((2-hydroxyéthyl)(méthyl)amino)propyl)-3,8-diméthyl-9*H*-pyrido[2,3-b]indole-7-carboxamide ;
*N*-(2-(éthyl(2-hydroxvéthyl)amino)éthyl)-5-(3-(éthylsulfonyl)phényl)-3,8-diméthyl-9*H*-pyrido[2,3-b]indole-7-carboxamide ;
5-(3-(Éthylsulfonyl)phényl)-*N*-(2-((2-hydroxyéthyl)(méthyl)amino)éthyl)-3,8-diméthyl-9*H*-pyrido[2,3-b]indole-7-carboxamide ;
5-(3-(Éthylsulfonyl)phényl)-8-méthyl-*N*-(1-méthylpipéridin-4-yl)-9*H*-pyrido-[2,3-b]indole-7-carboxamide ;
5-(3-(Éthylsulfonyl)phényl)-3-fluoro-8-méthyl-*N*-(1-méthylpipéridin-4-yl)-9*H*-pyrido[2,3-b]indole-7-carboxamide ;
3-Chloro-5-(3-(éthylsulfonyl)phényl)-8-méthyl-*N*-(1-méthylpipéridin-4-yl)-9*H*-pyrido[2,3-b]indole-7-carboxamide ;
5-(3-(Cyclopropylsulfonyl)phényl)-8-méthyl-*N*-(1-méthylpipéridin-4-yl)-9*H-*pyrido[2,3-b]indole-7-carboxamide ;
5-(3-(Cycopropylsulfonyl)phényl)-3-fluoro-8-méthyl-*N*-(1-méthylpipéridin-4-yl)-9*H*-pyrido[2,3-b]indole-7-carboxamide ;
3-Chloro-5-(3-(cyclopropylsulfonyl)phényl)-8-méthyl-*N*-(1-méthylpipéridin-4-yl)-9*H*-pyrido[2,3-b]indole-7-carboxamide ;
5-(3-(Éthylsulfonyl)phényl)-*N*-(1-(2-hydroxyéthyl)pipéridin-4-yl)-3,8-diméthyl-9*H*-pyrido[2,3-b]indole-7-carboxamide ;
3-Chloro-5-(3-(éthylsulfonyl)phényl)-*N*-(1-(2-hydroxyéthyl)pipéridin-4-yl)-8-méthyl-9*H*-pyrido[2,3-b]indole-7-carboxamide ;
5-(3-(Cyclopropylsulfonyl)phényl)-*N*-(1-(2-hydroxyéthyl)pipéridin-4-yl)-3,8-diméthyl-9*H*-pyrido[2,3-b]indole-7-carboxamide ;
3-Chloro-5-(3-(cyclopropylsulfonyl)phényl)-*N*-(1-(2-hydroxyéthyl)pipéridin-4-yl)-8-méthyl-9*H*-pyrido[2,3-b]indole-7-carboxamide ;
5-(3-(Éthylsulfonyl)phényl)-*N*-(2-((2-hydroxyéthyl)(méthyl)amino)éthyl)-3,8-diméthyl-9*H*-pyrido[2,3-b]indole-7-carboxamide ;
3-Chloro-5-(3-(éthylsulfonyl)phényl)-*N*-(2-((2-hydroxyéthyl)(méthyl)amino)-éthyl)-8-méthyl-9*H*-pyrido[2,3-b]indole-7-carboxamide ;
5-(3-(Cyclopropylsulfonyl)phényl)-*N*-(2-((2-hydroxyéthyl)(méthyl)amino)-éthyl)-3,8-diméthyl-9*H*-pyrido[2,3-b]indole-7-carboxamide ;
3-Chloro-5-(3-(cyclopropylsulfonyl)phényl)-*N*-(2-((2-hydroxyéthyl)(méthyl)-amino)éthyl)-8-méthyl-9*H*-pyrido[2,3-b]indole-7-carboxamide ;
*N*-(3-(Éthyl(2-hydroxyéthyl)amino)propyl)-5-(3-(éthylsulfonyl)phényl)-3,8-diméthyl-9*H*-pyrido[2,3-b]indole-7-carboxamide ;et
5-(3-(Éthylsulfonyl)phényl)-*N*-(3-((2-hydroxyéthyl)(méthyl)amino)propyl)-3,8-diméthyl-9*H*-pyrido[2,3-b]indole-7-carboxamide ;
ou un sel ou solvate pharmaceutiquement acceptable de ce composé.

3. Composé 5-(3-(éthylsulfonyl)phényl)-3,8-diméthyl-*N*-(1-méthylpipéridin-4-yl)-9*H*-pyrido[2,3-b]indole-7-carboxamide de formule : 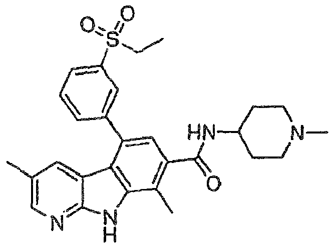 ou un sel ou solvate pharmaceutiquement acceptable de ce composé.

4. Composé selon la revendication 3, lequel composé se présente sous la forme d'un sel de l'acide chlorhydrique.

5. Composé selon la revendication 3, lequel composé se présente sous la forme d'un sel choisi dans le groupe constitué par un sel de l'acide trifluoroacétique, un sel de l'acide toluènesulfonique, un sel de l'acide benzènesulfonique, un sel de l'acide méthanesulfonique, un sel de l'acide succinique, un sel de l'acide tartrique, un sel de l'acide citrique, un sel de l'acide fumarique, un sel de l'acide sulfurique, un sel de l'acide phosphorique, un sel de l'acide benzoïque, un sel de l'acide bis-chlorhydrique, un sel de l'acide bis-trifluoroacétique, un sel tosylate, un sel hémifumarate, un sel de l'acide lactique, un sel de l'acide malique, un sel de l'acide hippurique et un sel de l'acide bromhydrique.

6. Composition pharmaceutique comprenant un composé selon l'une quelconque des revendications 1 à 5 comme principe actif et un excipient pharmaceutiquement acceptable.

7. Composé selon l'une quelconque des revendications 1 à 5, destiné à être utilisé comme médicament.

8. Composé selon l'une quelconque des revendications 1 à 5, destiné à être utilisé dans le traitement du cancer.

9. Composé selon la revendication 8, où le cancer est choisi dans le groupe constitué par le carcinome à cellules squameuses, l'astrocytome, le sarcome de Kaposi, le glioblastome, le cancer du poumon non à petites cellules, le cancer de la vessie, le cancer de la tête et du cou, le mélanome, le cancer de l'ovaire, le cancer de la prostate, le cancer du sein, le cancer du poumon à petites cellules, le gliome, le cancer colorectal, le cancer génito-urinaire, le cancer gastro-intestinal, le cancer de la thyroïde et le cancer de la peau.

10. Composé selon l'une quelconque des revendications 1 à 5, destiné à être utilisé dans le traitement de l'inflammation, de la maladie entérique inflammatoire, du psoriasis ou du rejet de greffe.

11. Composé selon l'une quelconque des revendications 1 à 5, destiné à être utilisé dans la prévention ou le traitement de la sclérose latérale amyotrophique, de la dégénérescence cortico-basale, du syndrome de Down, de la maladie de Huntington, de la maladie de Parkinson, du parkinsonisme post-encéphalitique, de la paralysie supranucléaire progressive, de la maladie de Pick, de la maladie de Niemann-Pick, de l'accident vasculaire cérébral, du traumatisme crânien et d'autres maladies neurodégénératives chroniques, de la maladie bipolaire, des troubles affectifs, de la dépression, de la schizophrénie, des troubles cognitifs, de la perte des cheveux, et comme médication contraceptive.

12. Composé selon l'une quelconque des revendications 1 à 5, destiné à être utilisé dans la prévention ou le traitement des troubles cognitifs légers, des troubles de la mémoire liés à l'âge, du déclin cognitif lié à l'âge, des troubles cognitifs sans démence, du déclin cognitif léger, du déclin neurocognitif léger, de la perte de mémoire liée à l'âge, des troubles mnésiques, des troubles cognitifs et de l'alopécie androgénétique.

13. Composé selon l'une quelconque des revendications 1 à 5, destiné à être utilisé dans la prévention ou le traitement de maladies associées à la démence, de la maladie d'Alzheimer et de conditions associées à des kinases.

14. Composé selon la revendication 13, où les maladies associées à la démence sont choisies dans le groupe constitué par la démence fronto-temporale avec syndrome parkinsonien, le complexe Parkinson-démence de Guam, la démence liée au HIV, des maladies s'accompagnant de pathologies à enchevêtrements neurofibrillaires, des états de pré-démence, de la démence vasculaire, de la démence à corps de Lewy, de la démence fronto-temporale et de la démence pugilistique.

15. Composé selon l'une quelconque des revendications 1 à 5, destiné à être utilisé dans le traitement de l'arthrite.
